# EUROPEAN PATENT APPLICATION

(11) **EP 4 644 557 A1**
(43) Date of publication of application: **05.11.2025**
(21) Application number: 24738449.8
(22) Date of filing: 02.01.2024
(51) Int. Cl.: C12N 15/90, C12N 9/22, C12N 15/113, C12N 15/55, A61K 38/46, A61P 31/12

(54) **RNA-GUIDED ENDONUCLEASE SYSTEM AND USE THEREOF IN GENE EDITING**

(30) Priority: 03.01.2023 WO PCT/CN2023/070108
(71) Applicant: Harbin Institute of Technology, Harbin, Heilongjiang 150001 (CN); Westlake University, Hangzhou, Zhejiang 310024 (CN)
(72) Inventor: HUANG, Zhiwei, Harbin, Heilongjiang 150001 (CN); ZHANG, Fan, Harbin, Heilongjiang 150001 (CN); REN, Kuan, Harbin, Heilongjiang 150001 (CN); ZHOU, Fengxia, Harbin, Heilongjiang 150001 (CN)
(74) Representative: Kraus & Lederer PartGmbB
(86) International application number: PCT/CN2024/070149
(87) International publication number: WO 2024/146516

(57) **Abstract**

Provided in the present application are an RNA-guided endonuclease system and the use thereof in gene editing. The endonuclease complex comprises an RNA-guided endonuclease and RNA, wherein the RNA-guided endonuclease comprises an amino acid sequence selected from SEQ ID NOs: 1-57 and SEQ ID NOs: 150-191, guide RNA comprises RNA associated with the RNA-guided endonuclease and targeting RNA, the RNA associated with the RNA-guided endonuclease comprises the sequence of any one of SEQ ID NOs: 58-114 and SEQ ID NOs: 192-233, and the targeting RNA is at the 3'-end of the RNA associated with the RNA-guided endonuclease and is identical or complementary to the targeting sequence of a genome of interest. The endonuclease complex of the present application can be used for efficient gene cleavage and gene editing.

## Description

The present disclosure claims the priority of the PCT Application No. PCT/CN2023/070108 titled "RNA-GUIDED ENDONUCLEASE SYSTEM AND USE THEREOF IN GENE EDITING" and filed on January 3, 2023.

### Technical Field

The present disclosure relates to the technical field of gene editing, in particular to an endonuclease complex and an application thereof.

### Background of the Disclosure

Genes are the most important genetic material in living individuals. They not only determine the phenotype of various lives, but also are highly associated with various diseases. With the progress of science and technology in recent years, the comprehensive development of sequencing technology and the completion of various genome projects, one has been able to analyze and study huge genetic data. What is hoped is not only to understand genes, but also to modify genes by means of technical methods to solve the problems of major genetic diseases and to optimize and improve the genes of animals and plants, etc. Against this background, gene editing technology is developing rapidly and innovating.

Gene editing technology refers to the technology of targeted modification of a gene achieved by changing the biological genome sequence by artificial means. In this technology, breaks in double-stranded DNA are produced at specific sites in the genome by using genetically engineered nucleases. Then, the DNA repair systems of the cell itself are used to repair the breaks in double-stranded DNA, including by means of non-homologous end joining or homologous recombination. Insertion and deletion of random bases may be induced during the DNA repair, leading to mutations in the genome at specific sites. The final functions of genes are changed due to sequence changes of the genome after the DNA repair.

The development of gene editing technology can be traced back to the 20th century. Prior to the 1990s, genetically mutated model organisms were predominantly procured through random mutagenesis. This approach was not only extremely time-consuming but also introduced random mutations, thereby rendering it arduous to achieve the desired gene modifications. After the 1990s, Chandrasegaran et al. developed the first generation of gene editing tools, zinc finger nucleases (abbreviated as ZFNs). In gene editing technology, ZFNs are the first new endonucleases formed by artificially fusing zinc finger proteins with Fok I endonuclease. In recent years, ZFNs have been used for gene editing in crops, fruit flies, zebrafish, mice, human cells, etc. However, zinc finger protein modules, which recognize DNA sequences, operate on the principle that each zinc finger recognizes three consecutive bases. This characteristic makes the design process for targeting extremely time-consuming and labor-intensive. Moreover, zinc finger nuclease technology faces significant limitations in large-scale applications. This is due to issues like non-specific cleavage by Fok I enzyme, a shortcoming that restricts its broader use in various fields. In 2009, the Ulla Bonas team discovered that the transcription activator-like effector (abbreviated as TALE) is closely associated with the base correspondence to DNA. Transcription activator-like effector nucleases (abbreviated as TALENs) were developed by fusing TALE and Fok I. TALENs are similar to ZFNs in principle, but the preparation steps of TALENs are relatively simple and their specificities are higher than those of ZFNs. In 2010, TALEN protein was firstly used in yeast. Since then, TALEN technology has been widely used in crops, mice, zebrafish, human cells and other areas. In 2012, TALEN technology was named one of the top ten scientific breakthroughs by the top scientific journal Science. In the same year, the Clustered Regularly Interspaced Short Palindromic Repeat sequences (CRISPR) and CRISPR associated proteins (Cas) (CRISPR/Cas) system began to attract widespread attention from worldwide researchers. Emmanuelle Charpentier and Jennifer A. Doudna established a system for CRISPR/Cas9 to cut double-stranded DNA *in vitro* for the first time. As reported by Feng Zhang in the journal Science, CRISPR/Cas9 has been used for gene editing in human cells. The gene editing technology of CRISPR/Cas9 has been applied to the researches in eukaryotes. Thus, the third generation of gene editing technology is unveiled. CRISPR/Cas, like ZFNs and TALENs, can produce breaks in double-stranded DNA at specific sites in genes, and thus can be applied in gene editing. Compared with the other two technologies, CRISPR/Cas technology shows obvious advantages with its simpler operation, lower cost, higher efficiency and stronger specificity. CRISPR/Cas is considered to be a gene editing tool with broad application prospects and is an alternative to the first two gene editing technologies.

The CRISPR/Cas gene editing technology comprises two important components: RNA acting as a guide and a Cas effector protein that acts to cleave DNA. Guided by the RNA, the effector protein recognizes the protospacer adjacent motif (PAM), binds to a specific location on the genome in a targeting manner, and exerts its endonuclease function, thereby causing a break in double-stranded DNA and enabling gene editing. Currently, the most widely used CRISPR/Cas system is the well-studied CRISPR/Cas9 system. In the CRISPR/Cas system, systems involving for example Cas12a also continue to receive attention.

Gene editing technology has extensive applications. It has important applications in research on gene function, plant breeding, modification and improvement of animal genes. But what is of most concern is undoubtedly the treatment of genetic diseases. Since the emergence of gene editing technologies, these technologies have played an important role in the treatment of genetic diseases in animal disease models and even in clinical settings, becoming a key component of the gene therapy field. Currently, gene therapy mainly involves two aspects: one is *in vivo* gene therapy, which involves delivering therapeutic genes to target organs or the whole body through viruses or materials; the other is *ex vivo* cell therapy, which uses cells from a patient or a healthy subject as carriers to deliver cells successfully repaired *in vitro* or cells capable of expressing therapeutic proteins to patients.

*In vivo* gene therapy methods have been developed for many years. Before the advent of gene editing technology, researchers had already begun using gene overexpression for gene therapy. After the discovery of adeno-associated virus (AAV), AAV-mediated gene therapy achieved success in animal models of various diseases, particularly demonstrating favorable effects in the clinical treatment of genetic diseases such as hemophilia. However, due to the inability of AAV to completely correct pathogenic genes at the genetic level, patients require multiple viral injections to alleviate symptoms, which may trigger systemic immune responses and pose life-threatening risks. The emergence of gene editing technologies, particularly CRISPR/Cas9, has significantly accelerated the progress of gene therapy. Using this technology, long-term disease treatment can be achieved by destroying pathogenic genes or repairing mutant genes *in situ.* This approach has been successfully applied to treat muscle diseases such as Duchenne muscular dystrophy and liver metabolic disorders such as type I tyrosinemia, hemophilia, and phenylketonuria.

Cell therapy has developed fast in recent years, mainly focusing on hematopoietic stem cells and immune cells. Infusing the *in vitro* edited and functionally restored cells back into patients can successfully treat anemic diseases and eliminate tumor cells. At present, many studies have been successfully applied to the clinic. Hematopoietic stem cell transplantation is currently the most promising method for treating thalassemia and sickle anemia. The combination of immune cells, especially T-cell, with gene editing technology is a hot spot in the field of tumor treatment currently. Chimeric antigen receptor T-cell (abbreviated as CART) therapy has saved the lives of many cancer patients in clinical practice.

Gene editing technology has greatly promoted the development of gene therapy, but it also faces various problems, mainly focusing on the efficiency of gene therapy and the cellular safety *in vivo.* The efficiency of gene therapy depends on the efficacy of gene editing, which is constrained by two factors: the delivery vector and the rate of gene integration. How to efficiently deliver gene editing tools to cells or the body has always been challenge for researchers. Taking the current AAV delivery method suitable for clinical treatment as an example, since the maximum packaging capacity of AAV vectors is less than 5kb, it is currently difficult to integrate both a CRISPR/Cas system and a repair template into a single AAV vector. The CRISPR/Cas system and repair template are mainly delivered separately, which greatly affect the effect of gene therapy. The smaller the gene editing tool system, the more efficient it becomes, making the construction of delivery vectors considerably easier. Currently, mainstream systems such as CRISPR/Cas9 and Cas12a exceed 5 kilobases (kb) in size, posing significant challenges for vector packaging and delivery. Consequently, the development of more compact gene editing tool systems has been a major focus for researchers in the field. Gene integration efficiency refers to the efficiency of the integration of the repair template into the cell. Efficient and targeted integration is beneficial to treat various genetic diseases, but integration efficiency is closely tied to the inherent DNA repair mechanisms in cells. Researchers have been optimizing strategies to enhance DNA damage repair efficiency, but there is still no effective technology to fully address this issue. The safety of gene editing technology is the most important issue. Since the emergence of gene editing technology, its off-target problem has been constantly appearing in the research process. The off-target problem has been solved neither in the ZFN nor in the CRISPR/Cas9 system. In addition to predictable off-target cleavage, deletions of genomic fragments can also occur in vivo, leading to unpredictable impacts on cellular functions and posing safety risks. Furthermore, the introduction of exogenous gene editing tool proteins can affect cells due to the intrinsic properties of these proteins. These challenges have driven researchers to develop more precise and safer gene editing tools.

In the past decade, the CRISPR/Cas system, as the mainstream gene editing technology, has developed a variety of different gene editing systems, including single-base editing technology based on the CRISPR/Cas system, which may induce specific base conversions to modify genes without introducing breaks in double-stranded DNA. Although gene editing technologies are becoming increasingly sophisticated, the challenges they face in practical applications remain unresolved. Researchers continue to explore novel gene editing tools and to develop innovative technologies, aiming to address these issues through the advancement of new gene editing systems. In 2021, Zhang Feng et al. speculated that TnpB of the IS605/IS200 family has RNA-guided nuclease activity. Subsequently, through a series of biochemical experiments, Virginijus et al. confirmed that TnpB is a reprogrammable RNA-guided functional nuclease. TnpB does not have clustered regularly interspaced short palindromic sequences (abbreviated as CRISPR), and it is guided by a new category of reRNAs to cleave human genomic DNA.

Furthermore, in the development of gene editing technology, it is worth noting that whether it is the zinc finger nuclease (ZFN), the transcription activator-like effector nuclease (TALEN) or the CRISPR/Cas system, they are basically optimized by Chinese researchers based on foreign developments.

There is a requirement for new gene editing technologies in this field.

### Summary of the Invention

In view of the lack of gene editing tools with improved performance in this field, the inventors provide the use of an endonuclease complex (also referred to herein as RAD or IS607 TnpB system) and an endonuclease (also referred to herein as RAD or IS607 TnpB protein) having a smaller molecular weight and enabling a wider range of applications as compared to the products in the gene editing field. The RAD (IS607 TnpB) system of this present disclosure is an RNA-guided endonuclease system.

The novel gene editing system provided by this disclosure, derived from the IS607 transposase system, consists of two coding sequences ORFA and ORFB encoding proteins, upstream and downstream transposition-related motifs. It has been proven that ORFB can be used in the IS605 system for gene editing. Although the names of IS605 and IS607 are similar, the mechanism of transposition between them is different. Similarly, ORFA plays transposase activity in the IS607 system, but there has been no research showing the specific function of ORFB. Thus, whether ORFB in the IS607 system has the characteristics of gene editing function has not been concerned and proved through biochemical experiments by those skilled in the field of gene editing.

In one aspect, the present disclosure provides an endonuclease complex comprising an RNA-guided endonuclease and an RNA.

In one embodiment, the RNA-guided endonuclease comprises an amino acid sequence selected from the group consisting of SEQ ID NOs: 1-57 and SEQ ID NOs: 150-191, or an amino acid sequence having at least 30%, 40%, 50%, 60%, 70%, 80%, 85%, 90%, 95%, 96%, 97%, 98%, 99% or 100% identity to any one of SEQ ID NOs: 1-57 and SEQ ID NOs: 150-191.

In one embodiment, the guide RNA comprises an RNA-guided endonuclease-associated RNA and a targeting RNA. The RNA-guided endonuclease-associated RNA is located at the 5' end of the guide RNA and the targeting RNA is located at the 3' end of the guide RNA, both of which may be directly linked or be adjacent to each other.

In one embodiment, the RNA-guided endonuclease-associated RNA comprises the sequence of any one of SEQ ID NOs: 58-114, and SEQ ID NOs: 192-233 or a sequence having at least 30%, 40%, 50%, 60%, 70%, 80%, 85%, 90%, 95%, 96%, 97%, 98%, 99% or 100% identity to the sequence of any one of SEQ ID NOs: 58-114, and SEQ ID NOs: 192-233. The RNA-guided endonuclease may be derived from IS607 TnpB.

In one embodiment, the targeting RNA is at the 3' end of the RNA-guided endonuclease-associated RNA. In one embodiment, the targeting RNA is at least 70%, 80%, 85%, 90%, 95%, 96%, 97%, 98%, 99%, or 100% identical or complementary to a targeted sequence in a gene of interest, such as the genome.

In one embodiment, the targeting RNA has a length of 12-40 bp. In one embodiment, the targeting RNA has a suitable length, such as 18-40 bp in length, and the first 14, 15, 16, 17 or 18 nucleotides of the targeting RNA are completely complementary to the targeted sequence in the gene of interest, such as the genome.

In one embodiment, the RNA-guided endonuclease protein comprises a RuvC domain. In one embodiment, the RNA-guided endonuclease protein comprises an amino acid selected from the group consisting of Q18, G27, R30, N34, F50, W73, A92, F97, P104, K107, Y117, E145, P150, S173, G192, D194, G196, K198, A201, S204, N211, I212, N213, R227, Q229, S233, R234, E237, G242, N249, K252, N266, V280, K283, P284, E290, L292, N293, G296, M297, M298, K299, L303, S304, K305, Q310, K322, P338, S339, S340, C343, C346, L353, L355, R358, C362, C364, D369, R370, D371, A374, N377 and L378 at a position corresponding to the amino acid sequence of SEQ ID NO: 6.

In one embodiment, the gene of interest (such as the genome of interest) is a single-stranded or double-stranded DNA. In one embodiment, the gene of interest (such as the genome of interest) comprises a motif selected from the group consisting of NGGAG, NGGNN, AGGAG, GAGGG, GGGGG, NNGGG, NNAGG, AGGNN, NTAAA, NGAGG, NNGGN or GGGGN. In one embodiment, for systems numbered 1-57, the gene of interest (such as the genome of interest) comprises a motif selected from the group consisting of AGGAG or GAGGG.

In another aspect, the present disclosure provides a polynucleotide comprising a polynucleotide of an RNA-guided endonuclease described herein and a polynucleotide for an RNA molecule. The polynucleotide for the RNA molecule may be a polynucleotide that transcribes the RNA molecule.

In another aspect, the present disclosure provides a vector comprising a polynucleotide molecule described herein. The vector comprises an expression control sequence, such as a promoter or terminator. In one embodiment, the vector is an expression vector.

In another aspect, the present disclosure provides a viral particle such as an adeno-associated viral particle, comprising an endonuclease complex, a polynucleotide or a vector described herein.

In another aspect, the present disclosure provides a method for specifically cleaving genomic DNA, comprising the step of contacting an endonuclease complex described herein with the genomic DNA.

In one embodiment, the DNA is genetic DNA of a eukaryotic cell, a prokaryotic cell, or a virus, such as the genome of interest.

In one embodiment, the DNA is a single-stranded or double-stranded DNA.

In one embodiment, the method comprises the step of introducing a polynucleotide or vector described herein into a cell.

In one embodiment, the targeted sequence of DNA, such as genomic DNA, comprises a motif selected from the group consisting of NGGAG, NGGNN, AGGAG, GAGGG, GGGGG, NNGGG, NNAGG, AGGNN, NTAAA, NGAGG, NNGGN or GGGGN. In one embodiment, for systems numbered 1-57, the gene of interest (such as the genome of interest) comprises a motif selected from the group consisting of AGGAG or GAGGG. In one embodiment, the 3' end of the motif is DNA that is at least 70%, 80%, 85%, 90%, 95%, 96%, 97%, 98%, 99%, or 100% identical or complementary to the targeting RNA.

In one embodiment, the method is performed *in vivo* or *in vitro.*

In one embodiment, the method is performed under conditions selected from the group consisting of 0.5 to 10 mM Mg²⁺, 25 to 250 mM Na⁺, the reaction temperature between 27 and 57 °C and the reaction time between 3 and 60 minutes.

In another aspect, provided is a method for gene editing comprising the step of contacting the endonuclease complex described herein with genomic DNA of a cell for gene editing.

In one embodiment, gene editing results in an insertion and/or a deletion in genomic DNA.

In one embodiment, the gene editing is performed in a mammalian cell to treat or prevent a disease, or to introduce a desired trait. In one embodiment, the gene of interest (such as the genome of interest) of the mammalian cell comprises a motif selected from the group consisting of NGGAG, NGGNN, AGGAG, GAGGG, GGGGG, NNGGG, NNAGG, AGGNN, NTAAA, NGAGG, NNGGN or GGGGN. In one embodiment, for systems numbered 1-57, the gene of interest (such as the genome of interest) comprises a motif selected from the group consisting of AGGAG or GAGGG.

In another aspect, the present disclosure provides a kit comprising one or more of following: an endonuclease complex described herein, a viral particle described herein, a polynucleotide described herein, and a vector described herein.

In another aspect, the present disclosure provides a method for inactivating or identifying a virus, comprising contacting the virus with an endonuclease complex or an endonuclease described herein. In one embodiment, the endonuclease comprises an amino acid sequence selected from the group consisting of SEQ ID NOs: 1-57 and SEQ ID NOs: 150-191, or an amino acid sequence having at least 30%, 40%, 50%, 60%, 70%, 80%, 85%, 90%, 95%, 96%, 97%, 98%, 99% or 100% identity to any one of SEQ ID NOs: 1-57 and SEQ ID NOs: 150-191. In one embodiment, the gene of interest (such as the genome of interest) of the virus comprises a motif selected from the group consisting of NGGAG, NGGNN, AGGAG, GAGGG, GGGGG, NNGGG, NNAGG, AGGNN, NTAAA, NGAGG, NNGGN and GGGGN. In one embodiment, the gene of interest (such as the genome of interest) of the virus comprises a motif selected from the group consisting of AGGAG or GAGGG, for example, for systems numbered 1-57.

In another aspect, the present disclosure provides an endonuclease complex or an endonuclease as described herein for use in inactivating or identifying a virus, comprising contacting the virus with an endonuclease complex or an endonuclease as described herein. In one embodiment, the endonuclease comprises an amino acid sequence selected from the group consisting of SEQ ID NOs: 1-57 and SEQ ID NOs: 150-191, or an amino acid sequence having at least 30%, 40%, 50%, 60%, 70%, 80%, 85%, 90%, 95%, 96%, 97%, 98%, 99% or 100% identity to any one of SEQ ID NOs: 1-57 and SEQ ID NOs: 150-191. In one embodiment, the gene of interest (such as the genome of interest) of the virus comprises a motif selected from the group consisting of NGGAG, NGGNN, AGGAG, GAGGG, GGGGG, NNGGG, NNAGG, AGGNN, NTAAA, NGAGG, NNGGN or GGGGN. In one embodiment, the gene of interest (such as the genome of interest) of the virus comprises a motif selected from the group consisting of AGGAG or GAGGG, for example, for systems numbered 1-57.

In another aspect, the present disclosure provides use of an endonuclease complex or an endonuclease described herein in the manufacture of a medicament for inactivating or identifying a virus. In one embodiment, the endonuclease comprises an amino acid sequence selected from the group consisting of SEQ ID NOs: 1-57 and SEQ ID NOs: 150-191, or an amino acid sequence having at least 30%, 40%, 50%, 60%, 70%, 80%, 85%, 90%, 95%, 96%, 97%, 98%, 99% or 100% identity to any one of SEQ ID NOs: 1-57 and SEQ ID NOs: 150-191. In one embodiment, the gene of interest (such as the genome of interest) of the virus comprises a motif selected from the group consisting of NGGAG, NGGNN, AGGAG, GAGGG, GGGGG, NNGGG, NNAGG, AGGNN, NTAAA, NGAGG, NNGGN or GGGGN. In one embodiment, the gene of interest (such as the genome of interest) of the virus comprises a motif selected from the group consisting of AGGAG or GAGGG, for example, for systems numbered 1-57.

In another aspect, the present disclosure provides a method for treating a viral infection, comprising administering an endonuclease complex described herein to a patient infected with a virus. In one embodiment, the gene of interest (such as the genome of interest) of the virus comprises a motif selected from the group consisting of NGGAG, NGGNN, AGGAG, GAGGG, GGGGG, NNGGG, NNAGG, AGGNN, NTAAA, NGAGG, NNGGN or GGGGN. In one embodiment, the gene of interest (such as the genome of interest) of the virus comprises a motif selected from the group consisting of AGGAG or GAGGG, for example, for systems numbered 1-57.

In another aspect, the present disclosure provides an endonuclease complex as described herein for use in treating a viral infection, the use comprising administering an endonuclease complex as described herein to a patient infected with a virus. In one embodiment, the gene of interest (such as the genome of interest) of the virus comprises a motif selected from the group consisting of NGGAG, NGGNN, AGGAG, GAGGG, GGGGG, NNGGG, NNAGG, AGGNN, NTAAA, NGAGG, NNGGN or GGGGN. In one embodiment, the gene of interest (such as the genome of interest) of the virus comprises a motif selected from the group consisting of AGGAG or GAGGG, for example, for systems numbered 1-57.

In another aspect, the present disclosure provides use of an endonuclease complex as described herein in the manufacture of a medicament for the treatment of a viral infection. In one embodiment, the gene of interest (such as the genome of interest) of the virus comprises a motif selected from the group consisting of NGGAG, NGGNN, AGGAG, GAGGG, GGGGG, NNGGG, NNAGG, AGGNN, NTAAA, NGAGG, NNGGN or GGGGN. In one embodiment, the gene of interest (such as the genome of interest) of the virus comprises a motif selected from the group consisting of AGGAG or GAGGG, for example, for systems numbered 1-57.

In another aspect, the present disclosure provides a viral vector comprising a polynucleotide or vector described herein. In one embodiment, the viral vector is an adeno-associated virus.

In another aspect, the present disclosure provides a gene therapy method for treating a disease comprising using a viral vector or a viral particle or an adeno-associated viral particle described herein.

In another aspect, the present disclosure provides a viral vector or a viral particle or an adeno-associated viral particle described herein for use in gene therapy for the treatment of a disease.

In another aspect, the present disclosure provides the use of a viral vector or a viral particle or an adeno-associated viral particle described herein in the manufacture of a medicament for use in a gene therapy for the treatment of a disease.

In another aspect, the present disclosure provides a cell comprising one or more of the following: an endonuclease complex, a polynucleotide, a vector, or a viral particle described herein. In one embodiment, the cell is a eukaryotic cell or a prokaryotic cell. In one embodiment, the eukaryotic cell is a fungal cell, a plant cell, or an animal cell.
The disclosure offers the following advantageous effects:
1. The applicant independently found and verified the RNA sequences in the endonuclease complexes described herein (i.e., RAD or IS607 TnpB systems) that are responsible for guiding a cleavage, which are discovered for the first time worldwide. Additionally, the functions of the RAD (IS607 TnpB) proteins within the endonuclease complexes are also discovered for the first time worldwide. (The sequence information of each component of the system is presented in Table 2 in detail);
2. The RAD proteins in the RAD (IS607 TnpB) systems are relatively smaller in size. For instance, they are made up of only 387 amino acids, which are less than one-third the size of the Cas9 protein in the currently most widely used CRISPR/Cas9 system. This small size gives the proteins greater advantages when it comes to the delivery of gene therapy vectors.
3. The RAD (IS607 TnpB) systems have an ability to specifically cleave double-stranded DNA, which is an essential prerequisite for their development into a gene editing tool. The systems recognize 5' terminal motif sequences such as NGGAG, NGGNN, AGGAG, GAGGG, GGGGG, NNGGG, NNAGG, AGGNN, NTAAA, NGAGG, NNGGN, or GGGGN (for systems numbered 1-57, the motifs are AGGAG or GAGGG). Currently, there are no gene editing tools that can recognize this type of motif sequence. As a result, the RAD (IS607 TnpB) systems broaden the range of recognition for gene editing tools. Moreover, they have a higher specificity compared to the most commonly used CRISPR/Cas9 system.
4. The RAD (IS607 TnpB) systems have a non-specific activity for cleaving single-stranded DNA and an activity for trans-cleaving single-stranded DNA. This property allows the RAD systems to cleave and eliminate single-stranded DNA viruses present in nature. They can also be used as a tool for identifying single-stranded DNA viruses.
5. The RAD (IS607 TnpB) systems have successfully induced DNA interference in both prokaryotic cells (Escherichia coli) and eukaryotic cells (human cells). This property indicates that the RAD systems can serve as a new type of gene editing tool, laying the groundwork for their development into an efficient and precise gene editing tool.
6. The RAD (IS607 TnpB) systems from 65 strains belonging to 55 different genera have *in vitro* DNA cleavage activity. The RAD systems from 16 strains belonging to 11 different genera have intracellular DNA cleavage activity. These diverse RAD systems are representative, demonstrating that the DNA cleavage activity in RAD systems is ubiquitous. This also shows that more RAD systems can be developed as gene editing tools.

### Description of the Drawings

Fig. 1 shows the acquisition process of the RAD (IS607 TnpB) systems.
Fig. 2 shows the results of matching RNA sequencing to the locus.
Fig. 3 shows the results after treatment of RAD complexes with DNase and RNase, respectively.
Fig. 4 shows the results of the analysis of transposon-associated motif (abbreviated as TAM) sequences required by RAD systems from different species.
Fig. 5 shows the TAM sequence of FbRAD1 protein and the results of the cleavage of double-stranded DNA.
Fig. 6 shows the results of the cleavage of DNA by FbRAD1 protein with a mutation in the active center of the RAD protein.
Fig. 7 shows a schematic diagram of the predicted secondary structure of RAD-RNA.
Fig. 8 shows the analysis of the alignment of RNA sequences in RAD systems with DNase activity.
Fig. 9 shows the analysis of the alignment of protein sequences in RAD systems with DNase activity.
Fig. 10 shows the results of the analysis of cleavage-occurring positions.
Fig. 11 shows the results of the cleavage of single-stranded DNA.
Fig. 12A shows cleavage results in presence of different motif sequences and mismatched bases.
Fig. 12B shows the results of the conditions of double-stranded DNA cleavage by RAD proteins.
Fig. 13 shows the results of an intracellular DNA interference experiment in *E*. *coli.*
Fig. 14 shows the DNA interference by different RAD systems in *E. coli* (with a TAM sequence of AGGAG).
Fig. 15 shows the DNA interference by different RAD systems in *E. coli* (with a TAM sequence of GAGGG).
Fig. 16 shows the results of DNA cleavage by RAD systems in human 293F cells.
Fig. 17 shows the results of DNA cleavage by different RAD systems in 293F cells.
Fig. 18 shows a process of the expression and purification of RAD protein nucleic acid complexes and RNA sequencing.
Fig. 19 shows a process of the digestion of a plasmid library by a RAD protein and high-throughput sequencing.
Fig. 20 shows a process of the cleavage of DNA by RAD protein in *E. coli* and screening.
Fig. 21 shows a process of the DNA editing by RAD protein in 293F cells and detection.

### Detailed Description of the Disclosure

Unless specifically indicated otherwise, all technical and scientific terms used herein have the same meaning as commonly understood by one of ordinary skill in the art. Furthermore, any methods or materials similar or equivalent to those described herein can be used in the practice of the present disclosure. All publications cited herein are incorporated by reference in their entirety.

Unless specifically defined herein, all terms used herein have the same meaning as they would have to those skilled in the art of the present disclosure. For definitions and terminology in the art, practitioners pay particular attention to Sambrook J. et al. (eds.), Molecular Cloning: A Laboratory Manual, 3rd Edition, Cold Spring Harbor Press, Plainsview, New York (2001); Ausubel, F.M., et al. (eds.), Current Protocols in Molecular Biology, John Wiley & Sons, New York (2010); and Coligan, J. E. et al., (eds.), Current Protocols in Immunology, John Wiley & Sons, New York (2010). In addition, definitions of common terms in molecular biology can be found in Benjamin Lewin, Genes IX, 2008, published by Jones and Bartlet (ISBN 0763752223); Kendrew et al. (eds.), The Encyclopedia of Molecular Biology, 1994, published by Blackwell Science Ltd. (ISBN 0632021829); and Robert A. Meyers (ed.), Molecular Biology and Biotechnology: A Comprehensive Desk Reference, 1995 (ISBN 9780471185710), published by VCH Publishers, Inc. In case of conflict, the terminology in the present disclosure shall prevail.

The term "gene editing" refers to a type of genetic engineering in which DNA is inserted, replaced, or removed from a target DNA (such as the genome of a cell) using one or more nucleases. Nucleases produce specific double-strand breaks (DSBs) at desired locations in the genome. Subsequently, using the endogenous machinery of the cell, the induced breaks are then repaired by homology-directed repair (HDR) (such as homologous recombination) or non-homologous end joining (NHEJ). Any suitable nuclease including, but not limited to, a CRISPR-associated protein (Cas) nuclease, a zinc finger nuclease (ZFN), a transcription activator-like effector nuclease (TALEN), a meganuclease, other endo- or exo- nucleases, variants thereof, fragments thereof, and combinations thereof, may be introduced into a cell to induce gene editing of a target DNA sequence. Nuclease-mediated gene editing can be performed by using the RNA with a stem-loop structure in combination with the RAD protein as described herein.

The term "nucleic acid", "nucleotide" or "polynucleotide" refers to deoxyribonucleic acid (DNA), ribonucleic acid (RNA), and their polymers in single-stranded, double-stranded or multi-stranded form. These terms include, but are not limited to, single-stranded, double-stranded, or multi-stranded DNA or RNA, genomic DNA, cDNA, DNA-RNA hybrids, or polymers comprising purine and/or pyrimidine bases or other natural, chemically modified, biochemically modified, non-natural, synthetic, or derivatized nucleotide bases. The term nucleic acid may be used interchangeably with gene, cDNA, and the mRNA encoded by the gene.

The term "gene" or "nucleotide sequence encoding a polypeptide" refers to a segment of DNA involved in the production of a polypeptide chain. DNA segments may include regions involved in transcription/translation of gene products and regulation of transcription/translation upstream and downstream of coding regions (leader and tail sequences), as well as intermediate sequences (introns) between individual coding segments (exons).

The term "complementary" refers to the ability of one nucleic acid to form hydrogen bonds with another nucleic acid sequence by conventional Watson-Crick or other non-conventional types. Complementarity percentage represents the percentage of residues in a nucleic acid molecule that are capable of forming hydrogen bonds (such as Watson-Crick base pairing) with a second nucleic acid sequence such as 50%, 60%, 70%, 80%, 90%, and 100% complementarity. "Completely complementary" means that all contiguous residues of a nucleic acid sequence will be hydrogen bonded to the same number of contiguous residues in a second nucleic acid sequence.

The term "expression vector" is a recombinantly or synthetically produced nucleic acid construct having a series of specific nucleic acid elements that allows transcription of the specific polynucleotide sequence in a host cell. The expression vector may be part of a plasmid, a viral genome, or a nucleic acid fragment. Typically, an expression vector includes a polynucleotide to be transcribed operably linked to a promoter.

In this disclosure, a RAD (IS607 TnpB) system refers to the RAGATH-18 containing RNA Associated DNase system (RAD), which is an abbreviation for RAGATH18-Associated DNA endonuclease. Usually, a system newly discovered by the inventors is named according to its characteristics. In the present disclosure, a RAD (IS607 TnpB) system is used interchangeably with an endonuclease complex or an RNA-guided endonuclease system. In the disclosure, a RAD (IS607 TnpB) protein is a DNase in the RAD (IS607 TnpB) system and can also be used interchangeably with an endonuclease herein.

A "guide RNA" refers to a polynucleotide comprising: 1) a guide sequence (also referred to as a "targeting RNA") capable of hybridizing with or being complementary to a target sequence (also referred to herein as a "targeted sequence"); and 2) a scaffold sequence capable of interacting with an endonuclease (referred to herein as "RNA-guided endonuclease-associated RNA sequence"). The guide nucleic acid may be RNA. The guide RNA may be encoded by a DNA sequence on a polynucleotide molecule.

As used herein, the term "RuvC domain" refers to a conserved domain or motif of amino acids having a nuclease activity (such as endonuclease activity). As used herein, a protein having a split RuvC domain refers to a protein having two or more RuvC motifs at sequentially different sites within the sequence, wherein the RuvC motifs interact with each other in a tertiary structure to form a RuvC domain.

The term "subject" includes a human or an animal. For example, an animal subject may be a mammal, a primate (such as a monkey), a livestock animal (such as a horse, a cow, a sheep, a pig, or a goat), a companion animal (such as a dog or a cat), a laboratory test animal (such as a mouse, a rat, a guinea pig, a bird), an animal of veterinary significance, or an animal of economic significance.

The term "sequence identity" or "identity" is used to describe the correlation between two amino acid sequences or between two nucleotide sequences. The sequence identity between two amino acid sequences can be determined as the output of the "longest identity" using the Needleman-Wunsch algorithm (Needleman and Wunsch, 1970, J. Mol. Biol. 48: 443-453), as implemented in the Needle program of EMBOSS software package (EMBOSS: The European Molecular Biology Open Software Suite, Rice et al., 2000, Trends Genet. 16: 276-277, preferably version 6.6. 0 or later). The parameters used are a gap open penalty of 10, a gap extension penalty of 0.5, and an EBLOSUM62 (EMBOSS version of BLOSUM62) substitution matrix. In order to make the Needle program report the longest identity, the nobrief option must be specified on a command line. The output of the "longest identity" marked by Needle is calculated as follows:
(the number of identical residues x 100)/(the length of the alignment - the total number of gaps in the alignment)

The sequence identity between two polynucleotide sequences can also be determined as the output of the "longest identity" using the Needleman-Wunsch algorithm (Needleman and Wunsch, 1970, supra), as implemented in the Needle program of EMBOSS software package (EMBOSS: The European Molecular Biology Open Software Suite, Rice et al., 2000, supra), preferably version 6.6. 0 or newer. The parameters used are a gap open penalty of 10, a gap extension penalty of 0.5, and an EDNAFULL (EMBOSS version of NCBI NUC4.4) substitution matrix. In order to make the Needle program report the longest identity, the nobrief option must be specified on a command line. The output of the "longest identity" marked by Needle is calculated as follows:
(the number of identical deoxyribonucleotides x 100)/(the length of the alignment - the total number of gaps in the alignment)

Provided herein is an endonuclease complex comprising an RNA-guided endonuclease and an RNA. The RNA-guided endonuclease comprises an amino acid sequence selected from the group consisting of SEQ ID NOs: 1-57 and SEQ ID NOs: 150-191, or an amino acid sequence having at least 30%, 40%, 50%, 60%, 70%, 80%, 85%, 90%, 95%, 96%, 97%, 98%, 99% or 100% identity to any one of SEQ ID NOs: 1-57 and SEQ ID NOs: 150-191. The guide RNA may comprise an RNA-guided endonuclease-associated RNA and a targeting RNA. The RNA-guided endonuclease-associated RNA may comprise the sequence of any one of SEQ ID NOs: 58-114 and SEQ ID NOs: 192-233, or a sequence having at least 30%, 40%, 50%, 60%, 70%, 80%, 85%, 90%, 95%, 96%, 97%, 98%, 99% or 100% identity to the sequence of any one of SEQ ID NOs: 58-114 and SEQ ID NOs: 192-233. The endonuclease system of the present disclosure may comprise: an endonuclease of SEQ ID NO: 1 and an RNA guide sequence comprising SEQ ID NO: 58; an endonuclease of SEQ ID NO: 2 and an RNA guide sequence comprising SEQ ID NO: 59; an endonuclease of SEQ ID NO: 3 and an RNA guide sequence comprising SEQ ID NO: 60; an endonuclease of SEQ ID NO: 4 and an RNA guide sequence comprising SEQ ID NO: 61; an endonuclease of SEQ ID NO: 5 and an RNA guide sequence comprising SEQ ID NO: 62; an endonuclease of SEQ ID NO: 6 and an RNA guide sequence comprising SEQ ID NO: 63; an endonuclease of SEQ ID NO: 7 and an RNA guide sequence comprising SEQ ID NO: 64; etc. In other words, the endonuclease system of the present disclosure may comprise an endonuclease comprising SEQ ID NO: N paired with an RNA guide sequence comprising SEQ ID NO: N + 57, wherein the N is an integer of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, or 57. The endonuclease system of the present disclosure may comprise an endonuclease comprising SEQ ID NO: M paired with an RNA guide sequence comprising SEQ ID NO: M + 42, wherein the M is an integer of 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, or 191. The endonuclease system of the present disclosure may comprise 99 types of RNA-guided endonucleases paired with RNAs listed herein.

The RNA guide sequence may comprise a RAGATH18-associated RNA sequence or an endonuclease-associated RNA sequence and a targeting RNA sequence. There may be sequence changes in the endonuclease and RNA guide sequence.

In one embodiment, the endonuclease complex comprises an RNA-guided endonuclease of SEQ ID NO: 6 and a guide RNA of SEQ ID NO: 63; an RNA-guided endonuclease of SEQ ID NO: 11 and a guide RNA of SEQ ID NO: 68; an RNA-guided endonuclease of SEQ ID NO: 13 and a guide RNA of SEQ ID NO: 70; an RNA-guided endonuclease of SEQ ID NO: 21 and a guide RNA of SEQ ID NO: 78; an RNA-guided endonuclease of SEQ ID NO: 43 and a guide RNA of SEQ ID NO: 100; an RNA-guided endonuclease of SEQ ID NO: 51 and a guide RNA of SEQ ID NO: 108; an RNA-guided endonuclease of SEQ ID NO: 52 and a guide RNA of SEQ ID NO: 109; an RNA-guided endonuclease of SEQ ID NO: 54 and a guide RNA of SEQ ID NO: 111; or an RNA-guided endonuclease of SEQ ID NO: 55 and a guide RNA of SEQ ID NO: 112.

The guide RNA may exhibit a stem-loop structure. The targeting RNA may be at the 3' end of the RNA-guided endonuclease-associated RNA. The targeting RNA may be at least 70%, 80%, 85%, 90%, 95%, 96%, 97%, 98%, 99%, or 100% identical or complementary to a targeted sequence in the genome. The targeting RNA has a length of 12-40 bp. For example, the targeting RNA has a length of 13, 14, 15, 16, 17, 18, 19, 20, 21, 22, 23, 24, 25, 26, 27, 28, 29, 30, 31, 32, 33, 34, 35, 36, 37, 38, 39, or 40 bp.

The RNA-guided endonuclease protein may contain a functional RuvC domain. The RNA-guided endonuclease protein may comprise an amino acid selected from the group consisting of Q18, G27, R30, N34, F50, W73, A92, F97, P104, K107, Y117, E145, P150, S173, G192, D194, G196, K198, A201, S204, N211, I212, N213, R227, Q229, S233, R234, E237, G242, N249, K252, N266, V280, K283, P284, E290, L292, N293, G296, M297, M298, K299, L303, S304, K305, Q310, K322, P338, S339, S340, C343, C346, L353, L355, R358, C362, C364, D369, R370, D371, A374, N377 and L378 at a position corresponding to the amino acid sequence of SEQ ID NO: 6.

The genome of interest may be single-stranded or double-stranded DNA. The genome of interest may comprise a motif selected from the group consisting of NGGAG, NGGNN, AGGAG, GAGGG, GGGGG, NNGGG, NNAGG, AGGNN, NTAAA, NGAGG, NNGGN, or GGGGN.

The present disclosure provides a method for specifically cleaving genomic DNA, comprising the step of contacting an endonuclease complex described herein with the genomic DNA. The genomic DNA may be the genomic DNA of a eukaryotic cell, a prokaryotic cell or a virus. The eukaryotic cell is not particularly limited and includes, but is not limited to, a mammalian (such as human) cell, or a yeast cell. The prokaryotic cell is not particularly limited and includes, but is not limited to, *an E. coli* cell. The genomic DNA may be a single-stranded or double-stranded DNA. The method may comprise the step of introducing a polynucleotide or vector described herein into a cell. The targeted sequence of genomic DNA may comprise a motif selected from the group consisting of NGGAG, NGGNN, AGGAG, GAGGG, GGGGG, NNGGG, NNAGG, AGGNN, NTAAA, NGAGG, NNGGN, or GGGGN. The 3' end of the motif may be DNA that is at least 70%, 80%, 85%, 90%, 95%, 96%, 97%, 98%, 99%, or 100% identical or complementary to the targeting RNA. The method may be performed *in vivo* or *in vitro.* The method is carried out under conditions selected from the group consisting of 0.5 to 10 mM of a divalent metal ion selected from Mg²⁺, Mn²⁺ and Ca²⁺, 25 to 250 mM Na⁺, a reaction temperature between 27 and 57°C, and a reaction time between 3 and 60 minutes.

The concentration of the divalent metal ion may be 0.5 to 10 mM, for example 1, 2, 2.5, 3, 4, 5, 6, 7, 8, 9, 10 mM. The type of salts providing the divalent metal ion is not particularly limited, and includes, but is not limited to, a chloride salt.

The concentration of the Na⁺ may be 25 to 250 mM, such as 50, 100, 150 or 200 mM. The type of sodium salts is not particularly limited, and include, but are not limited to, chloride salts.

Provided is a method for gene editing comprising the step of contacting the endonuclease complex described herein with genomic DNA of a cell for gene editing. The gene editing may result in an insertion and/or a deletion in genomic DNA. The gene editing may be performed in mammalian cells to treat or prevent a disease, or to introduce a desired trait.

Provided is a kit comprising one or more of the following: an endonuclease complex described herein, a polynucleotide described herein, and a vector described herein. Alternatively, a kit may comprise any of the components used herein.

Provided is a method for inactivating or identifying a virus, comprising contacting the virus with an endonuclease complex or an endonuclease described herein. In one embodiment, the endonuclease comprises an amino acid sequence selected from the group consisting of SEQ ID NOs: 1-57 and SEQ ID NOs: 150-191, or an amino acid sequence having at least 30%, 40%, 50%, 60%, 70%, 80%, 85%, 90%, 95%, 96%, 97%, 98%, 99% or 100% identity to any one of SEQ ID NOs: 1-57 and SEQ ID NOs: 150-191.

The RAD (IS607 TnpB) system of the present disclosure comprises an effector protein of Table 1. However, an effector protein with a similar function can also be used as endonuclease in the system of the present disclosure.

**Table 1: Source strains and effector proteins of 57 RAD (IS607 TnpB) systems**

| **Numb er** | **Name of strain** | **Strain ID** | **Protein ID** |
|---|---|---|---|
| **1** | Catenibacterium sp. AM22-6LB | QTUM01000055.1 | RGE94338.1 |
| **2** | Mitsuokella sp. AF33-22 | QRQA01000020.1 | RHM53169.1 |
| **3** | Blautia sp. AF14-40 | QUHK01000008.1 | RHS04595.1 |
| **4** | Ruminococcus sp. AF17-12 | QUGY01000003.1 | RHR70509.1 |
| **5** | Clostridium sp. AF27-5AA | QUFH01000002.1 | RHQ35082.1 |
| **6** | Firmicutes bacterium AM43-11BH | QUII01000032.1 | RHS75625.1 |
| **7** | Lachnospiraceae bacterium AM26- 1LB | QUJW0100002.1 | RHU00256.1 |
| **8** | Dorea formicigenerans strain AF19- 4AC | QRWH01000001.1 | RGT12103.1 |
| **9** | [Eubacterium] hallii strain AF31- 17AC | QRQO01000101.1 | RHN04853.1 |
| **10** | Clostridiaceae bacterium AF18- 31LB | QUGS01000002.1 | RHR46218.1 |
| **11** | Roseburia inulinivorans strain AF24-4 | QRUN01000005.1 | RGR69673.1 |
| **12** | Anaerobutyricum hallii strain AM34-3LB | QSID01000021.1 | RHC60416.1 |
| **13** | [Eubacterium] rectale strain AM42- 1 | QSGF01000003.1 | RHB06265.1 |
| **14** | Bacillus cereus strain OM04-20 | QSUV01000008.1 | RGN78093.1 |
| **15** | Weizmannia coagulans strain AF24- 21 | QRUS01000007.1 | RGR87203.1 |
| **16** | Blautia sp. TF11-31AT | QUKH01000003.1 | RHU47384.1 |
| **17** | Blautia sp. TM10-2 | QUJZ01000004.1 | RHU18041.1 |
| **18** | Blautia sp. OM07-19 | QULA0100001 | RHV03091.1 |
| | | 0.1 | |
| **19** | Butyricicoccus sp. AM28-25 | QUJL0100000 1.1 | RHT78732.1 |
| **20** | Clostridiaceae bacterium AF18-31LB | QUGS0100001 0.1 | RHR42937.1 |
| **21** | Clostridiales bacterium AM23-16LB | QWVV010000 23.1 | RGD88909.1 |
| **22** | Clostridium perfringens strain AF30-3 | QRQT0100000 1. | RHN30353.1 |
| **23** | Coprococcus catus strain AM28-39 | QVFD0100003 6.1 | RGC42877.1 |
| **24** | Coprococcus comes strain AF18-12LB | QRXJ0100002 6.1 | RGT87174.1 |
| **25** | Coprococcus sp. AM25-15LB | QVGH010000 02.1 | RGC76021.1 |
| **26** | Dorea formicigenerans strain AF25-11 | QRUK0100002 5. | RGR57390.1 |
| **27** | Dorea formicigenerans strain AF12-11 | QSAJ0100000 4.1 | RGW55202.1 |
| **28** | Dorea sp. AF36-15AT | QUDV010000 04. | RHP08869.1 |
| **29** | Firmicutes bacterium AM55-24TS | QTVU0100002 1.1 | RGF95087.1 |
| **30** | Firmicutes bacterium AF16-15 | QTXI0100003 0.1 | RGH01875.1 |
| **31** | Firmicutes bacterium TM09-10 | QUKC0100000 1.1 | RHU31723.1 |
| **32** | Lachnospiraceae bacterium AM48-27BH | QUFC0100001 0.1 | RHQ17086.1 |
| **33** | Megamonas funiformis strain AF12-42 | QSAH0100009 2.1 | RGW42851.1 |
| **34** | Megamonas rupellensis strain AF29-2 | QRST0100000 1.1 | RGQ08395.1 |
| **35** | Mitsuokella multacida strain AM25-21AC | QRHE0100000 9.1 | RHF50970.1 |
| **36** | Mitsuokella multacida strain AM25-21AC | QRHE0100000 3.1 | RHF52474.1 |
| **37** | Mitsuokella sp. AF33-22 | QRQA0100000 8.1 | RHM55346.1 |
| **38** | Peptoclostridium sp. AF21-18 | QUGE0100000 2.1 | RHQ99195. |
| **39** | Ruminococcus sp. AF17-12 | QUGY010000 08.1 | RHR68682.1 |
| **40** | Ruminococcus sp. AF17-12 | QUGY010000 02.1 | RHR71212.1 |
| **41** | Ruminococcus sp. AF18-29 | QTWT010000 12.1 | RGG64615.1 |
| **42** | Firmicutes bacterium TM09-10 | QUKC0100001 0.1 | RHU26542.1 |
| **43** | Firmicutes bacterium AM41-5BH | QTXW010000 19.1 | RGH37793.1 |
| **44** | Monoglobales | GUT_GENOM E084711_28 | GUT_GENOME08471 1_28_1717_2925_+ |
| **45** | Eisenbergiella sp | GUT_GENOM E11227339 | GUT_GENOME11227 3_39_14283_15425_+ |
| **46** | Anaerostipes hadrus | _ SRR7352028_ 380524_48115 | SRR7352028_380524_ 48115_816_1829_- |
| **47** | Eisenbergiella sp | GUT_GENOM E129951_100 | GUT_GENOME12995 1_100_5873_7024_+ |
| **48** | Mageeibacillus indolicus UPII9-5 | NC_013895.2 | WP_005609868.1 |
| **49** | [Eubacterium] rectale ATCC 33656 | NC_012781.1 | WP_012741946.1 |
| **50** | Erysipelotrichaceae bacterium SG0102 | AP019309.1 | BBH26708.1 |
| **51** | Dorea formicigenerans | GUT_GENOM E239804_2 | GUT_GENOME23980 4_2_85784_86938_+ |
| **5 2** | Agathobacter rectalis | GUT_GENOM E047820_36 | GUT_GENOME04782 0_36_478_1632_- |
| **54** | Dorea sp | GUT_GENOM E001213_4 | GUT_GENOME00121 3_4_160075_161238_+ |
| **54** | Hungatella | GUT_GENOM E022845_45 | GUT_GENOME02284 5_45_3955_5109_+ |
| **55** | Agathobacter faecis | GUT_GENOM E112567_131 | GUT_GENOME11256 7_131_1855_3009_- |
| **56** | Clostridium sp | GUT_GENOM E034484_15 | GUT_GENOME03448 4_15_4023_5177_- |
| **57** | Fusicatenibacter saccharivorans | GUT_GENOM E040537_31 | GUT_GENOME04053 7_31_7690_8853_+ |
| | | | |

| **New Numb er** | Strain Name | Strain ID | Protein ID |
|---|---|---|---|
| **58** | NA | ERR3307045_ 63958_30277 | ERR3307045_63958_3 0277_4897_6000_- |
| **59** | Fusobacterium | GUT _GENOM E151180_45 | GUT_GENOME15118 0_45_9178_10326_+ |
| **60** | NA | ERR2017626_ 1022590_1286 3 | ERR2017626_1022590 _12863_8034_9173_+ |
| **61** | Clostridioides difficile | GUT_GENOW E142162_18 | GUT_GENOWE14216 2_18_64466_65659_- |
| **62** | Desulfofarcimen acetoxidans DSM 771 | NC_013216.1 | NC 013216_1_421908_422 |
| | | | 903_+ |
| **63** | Clostridium tetani E88 | NC_004557.1 | WP_035125047.1 |
| **64** | Gracilibacillus timonensis | GUT _GENOM E096560_11 | GUT_GENOME09656 0_11_244135_245259_ |
| **6 5** | Mitsuokella multacida | GUT_GENOM E083533_37 | GUT_GENOME08353 3_37_11359_12597_- |
| **66** | Enterococcus mundtii QU 25 | NC_022878.1 | WP_023518930.1 |
| **67** | Anoxybacillus flavithermus strain DSM 2641T | NZ_CP020815. 1 | WP_012574395.1 |
| **68** | Fusobacterium pseudoperiodonticum strain KCOM 1277 | NZ_CP024701. 1 | WP_099988772.1 |
| **69** | Clostridia | GUT_GENOM E228548_94 | GUT _GENOME22854 8_94_18418_19641_+ |
| **70** | Lachnoclostridium phytofermentans ISDg | NC_010001.1 | WP_012201916.1 |
| **71** | Petrotoga mobilis SJ95 | NC_010003.1 | WP_081429131.1 |
| **72** | Lachnospira | GUT_GENOM E249453_33 | GUT_GENOME24945 3_33_14687_15901_- |
| **73** | NA | GUT_GENOM E046003_7 | GUT _GENOME04600 3_7_13445_14386_- |
| **74** | NA | GUT_GENOM E043660_1 | GUT _GENOME04366 0_1_131655_132716_- |
| **75** | Clostridium argentinense strain 89G | NZ_CP014176. 1 | WP_039636020.1 |
| **76** | Acetoanaerobium sticklandii strain DSM 519 | NC_014614.1 | WP_013360293.1 |
| **7 7** | Mitsuokella jalaludinii | GUT_GENOM E243333_13 | GUT_GENOME24333 3_13_9207_10436_+ |
| **78** | Mitsuokella multacida | GUT_GENOM E228909_131 | GUT _GENOME22890 9_131_318_1547_- |
| **79** | Mitsuokella sp. AF21-1AC AF21-1AC.Scaf4 | QRVW010000 04.1 | RGS74140.1 |
| **80** | Eisenbergiella sp | GUT_GENOM E029159_118 | GUT_GENOME02915 9_118_4969_5874_- |
| **81** | Finegoldia magna ATCC 29328 plasmid pFMC | NC_010371.1 | WP_012289953.1 |
| **8 2** | Clostridium aceticum strain DSM 1496 | NZ_CP009687. 1 | WP_044824965.1 |
| **83** | Eubacterium sp | GUT_GENOM E100288_478 | GUT_GENOME10028 8_478_819_1994_- |
| **84** | Monoglobales | GUT_GENOM E089678_61 | GUT_GENOME08967 8_61_13091_14269_- |
| **85** | Intestinibacter bartlettii | GUT_GENOM E190643_17 | GUT_GENOME19064 3_1780689183- |
| **86** | NA | ERR1539620_ 45264_57524 | ___ ERR1539620_45264_5 7524_39821_41020_- |
| **87** | Clostridium sporogenes strain CDC_1632 | NZ_CP013243. 1 | WP_072587064.1 |
| **88** | Clostridium novyi strain 150557 | CP029458.1 | AYF55140.1 |
| **89** | Clostridium sporogenes strain AM1195 plasmid pRSJ11_1 | CP013700.1 | AUM93701.1 |
| **90** | NA | SRR5892202 233507_16022 | SRR5892202_233507_ 16022_11195_12361_+ |
| **91** | Bacillus thuringiensis BMB171 | NC_014171.1 | WP_000978855.1 |
| **92** | Bacillus thuringiensis serovar chinensis CT-43 plasmid pCT14 | NC_017209.1 | WP_000604588.1 |
| **93** | Anaerostipes hadrus strain BPB5 | NZ_CP012098. 1 | WP_077326813.1 |
| **94** | Erysipelotrichaceae | GUT_GENOM E170581_2 | GUT_GENOME17058 1_2_14431_15588_- |
| **95** | Paenibacillus durus ATCC 35681 | NZ_CP011114. 1 | WP_025697990.1 |
| **96** | Clostridium sp. JN-9 | CP035280.1 | QAT40560.1 |
| **97** | Selenomonas ruminantium subsp. lactilytica TAM6421 plasmid pSRC1 | NC_017078.1 | WP_014431089.1 |
| **98** | Blautia wexlerae | GUT_GENOM E088555122 | GUT_GENOME08855 5_122_512_1693_+ |
| **99** | Brevibacillus brevis strain DZQ7 | _ CP030117.1 | AWX54000.1 |

Exemplary endonucleases of the present disclosure and their associated RNA sequences are listed in Table 2. The endonuclease system of the present disclosure may comprise: an endonuclease of SEQ ID NO: 1 and an RNA guide sequence comprising SEQ ID NO: 58; an endonuclease of SEQ ID NO: 2 and an RNA guide sequence comprising SEQ ID NO: 59; an endonuclease of SEQ ID NO: 3 and an RNA guide sequence comprising SEQ ID NO: 60; an endonuclease of SEQ ID NO: 4 and an RNA guide sequence comprising SEQ ID NO: 61; an endonuclease of SEQ ID NO: 5 and an RNA guide sequence comprising SEQ ID NO: 62; an endonuclease of SEQ ID NO: 6 and an RNA guide sequence comprising SEQ ID NO: 63; an endonuclease of SEQ ID NO: 7 and an RNA guide sequence comprising SEQ ID NO: 64; etc. That is, the endonuclease system of the present disclosure may comprise an endonuclease comprising SEQ ID NO: N and an RNA guide sequence comprising SEQ ID NO: 57 + N, wherein the N is an integer of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, or 57. The RNA guide sequence may comprise a RAGATH18-associated RNA sequence or an endonuclease-associated RNA sequence and a targeting RNA sequence. There may be sequence changes in the endonuclease and RNA guide sequence. The endonuclease may comprise an amino acid sequence selected from the group consisting of SEQ ID NOs: 1-57 and SEQ ID NOs: 150-191, or an amino acid sequence having at least 30%, 40%, 50%, 60%, 70%, 80%, 85%, 90%, 95%, 96%, 97%, 98%, 99% or 100% identity to any one of SEQ ID NOs: 1-57 and SEQ ID NOs: 150-191. A guide RNA may comprise an RNA-guided endonuclease-associated RNA and a targeting RNA, wherein the RNA-guided endonuclease-associated RNA may comprise the sequence of any one of SEQ ID NOs: 58-114 and SEQ ID NOs: 192-233, or a sequence having at least 30%, 40%, 50%, 60%, 70%, 80%, 85%, 90%, 95%, 96%, 97%, 98%, 99% or 100% identity to the sequence of any one of SEQ ID NOs: 58-114 and SEQ ID NOs: 192-233. The targeting RNA is at the 3' end of the RNA-guided endonuclease-associated RNA and is at least 70%, 80%, 85%, 90%, 95%, 96%, 97%, 98%, 99% or 100% identical or complementary to the targeted sequence in the gene of interest.

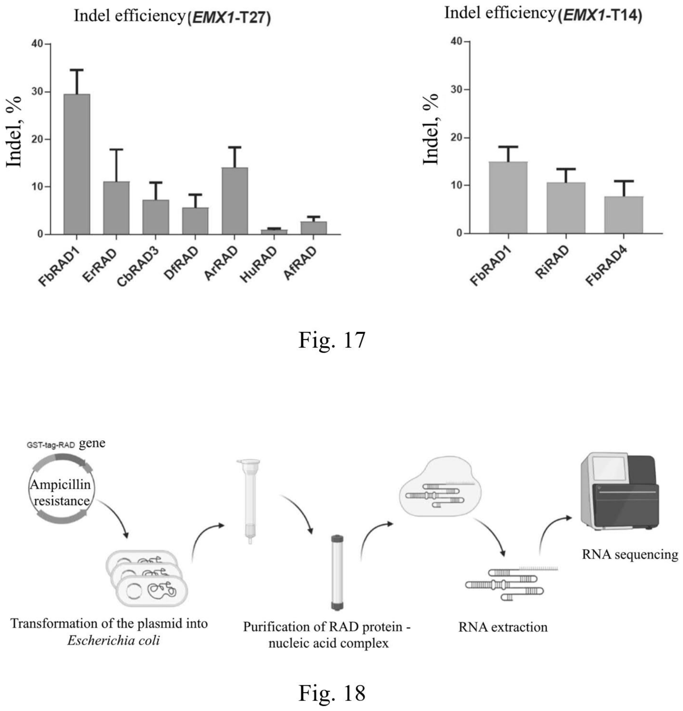

Under the conditions where the TAM sequence is AGGAG or GAGGG, a total of 12 RAD (IS607 TnpB) systems are found to successfully achieve a cleavage in prokaryotes. Those 12 RAD (IS607 TnpB) systems are from 7 different species. They are (6) FbRAD1/ISFba1 (RHS75625.1), (10) CbRAD1/ISCba1 (RHR46218.1), (11) RiRAD/ISRin (RGR69673.1), (13) ErRAD/ISEre1 (RHB06265.1), (15) WcRAD (RGR87203.1), (18) BsRAD/ISBsp4 (RHV03091.1), (20) CbRAD2/ISClsp2 (RHR42937.1), (21) CbRAD3/ISClsp3 (RGD88909.1), (24) CcRAD/ISCco (RGT87174.1), (30) FbRAD2/ISFba2 (RGH01875.1), (42) FbRAD3/ISFba3(RHU26542.1), or (43) FbRAD4/ISFba4 (RGH37793.1), respectively. Different RAD systems are expressed in 293F cells to verify whether they all have an *in vivo* editing function in mammalian cells. It is found that 9 RAD (IS607 TnpB) systems are able to edit *in vivo,* and they are from 8 different species. They are: (6) FbRAD1/ISFba1 (RHS75625.1), (11) RiRAD/ISRin (RGR69673.1), (13) ErRAD/ISEre1 (RHB06265.1), (21) CbRAD3/ISClsp3 (RGD88909.1), (43) FbRAD4 (RGH37793.1), (51) DfRAD/ ISDfo4 (GUT_GENOME239804_2_85784_86938_+), (52) ArRAD/ ISAre (GUT_GENOME047820_36_478_1632_-), (54) HuRAD/ ISHun (GUT_GENOMEO22845_45_3955_5109_+) and (55) AfRAD/ ISAfa (GUT_GENOME112567_131_1855_3009_-) (Fig. 17).

**Table 2 Sequences of the proteins and RNAs of 57 RAD (IS607 TnpB) systems**

| **SE Q ID NO** | **Protein ID** | **The amino acid sequence of the protein** | **SEQ ID NO** | **RAGATH18-associated RNA sequence** |
|---|---|---|---|---|
| **1** | RGE943 38.1 | MRKLLKSFKTEINPTEEQKVRIRKTIGTCRFIYNFYLAH NKELHESGKKFMSSSQFRVWLNNEYLPSHPEYSWIKEA YSKSVTQAVNDGQTAFTRFFNHKSAFPKFKKKGKSDV KMYFVRNNPKDCSCERHRIKIPSLGWVRIKEKGYIPTTK DGYVIKSGHVSIKADRYYVSVLIEIPDKTSASNSSEGIGI DLGLKDFAIASNGKTYKNINKSAKLKKLEKKLIREQRS LSRKYENIKKGGSTQKRNIQKQRLKIQKLHHRIDNIRTD YINKIIAEIVKTKPSYITIEDLNISGMMKNRHLSKAVASQ KFYEFKTKLQAKCRENGIELRVVDRWFPSSKTCHCCKS IKKDLKLSDRLFRCDCGYIEDRDFNAALNLRDATTYEV A | 58 | AUGAACGCAAACGUAAGUAUGU ACCGAAGGCUAUUUCGGGAAUU UACGACUAUGGAGUGCACUAGA ACUUGUGAGUAGAUAUGAUUUC GGUCAAUCCAAAGCAUGCACAG UGACAUAGUAAGUAAUGUUCGU GAGAACUUACAUUAUCUCGAUG UGAGUAUAUUUAAUCACAUUUU GAGUGGCAG |
| **2** | RHM531 69.1 | MKRLLKSFKTELRLTESQTDKVRRSLGICRFLANQYIAR NIWLCRLYQRGVLDEKQPHFITANDFDKYVNHVLKKK FPWIDQCGSKARKKVLVNAEQAFRRFFKGPARFPRFKR KNQQNVKLYFPKNNKGDWTIWRHKIQIPTIGRVRLKEF GYLPQGVTIKNGHVSCEAGRYYVSVTAEIEETSRYNND LEASYHPQSDGIGVDLGVKSLAIVSDGRIFENINTSSRAR RLEKRLRREQRRLSRKYEIRKKKGGSTATASANIEKKR LSVQKLHQRLTNLRRNYENQVIHALVEQKPRFITMEDL NVTGMMKNRHLSKSVAQQRFYSLREKLRRKAEIIGIEF RIADRFYPSSKTCHACGHVHGGLKLKDRIYVCPVCGYT EDRDLNAALNLRDTKNYRIA | 59 | UUCACGUUUUCACGUCUUGAUA CAUGUACCGAUGGCUAGUCGGG AACAGGCCGUUGCCUUGUGAGG CAAGGAAUACGGCCAACGCCUUU GGAGUGUACAAGAACUUGUGAG UAGUCUCAGGCUUGCCUGGACA AAAGCAUACACGAUGAAUAAGG AAUUUUUCUAGGCAUAGAUAAU UUCUAUGUUAUUCUAUGUUUUU AGUAGCAG |
| **3** | RHS045 95.1 | MRKLQKSFKTEINPTDEQKLKIRRTIGTCRYVYNFYLD QNRKRYEKGEKFMSGKSFSVWLNNEYLPQNPEYAWIK | 60 | GAAACGCAGAGAUACAUAUGUA CCGAAGGCCAUUUCGGGAAUUA |
| | | EVSSKSVKRSLENACTAFSRFFKGQSHFPRYKKKNKSN VKMYFVKNNRTDCFCERHRVNIPTLGWVRIKEKGYLP TTKDGYVIKSGTVSMRAGRYYVSVLVEVPDPPKEENR NEGIGIDLGIKSFAVLSDGTVYQNINKSAKIKKLEKQLR REQRSLSRKYENLKKGVSTQRANIQKQKLKVQRLYQRI DNIRTDYIKKTIAGIVKTKPSFVTIEDLNVKGMMKNRHL SKATASQKFYEFRTRLQGKCAEKGIELRIADRWYPSSR RCHNCGQIRKDLKLSNRVYQCSCGYCEDRDLNAALNL RDVKTYQSA | | ACGACUGUGGAGUGUACCAGAA CUUGUGAGUAGAUACAGCUCCG GCUGGUCAAAAGCAUACACGUU UGAAACAGUAAGUAGGAUUCGU GAGAACUACAAUAUCUCGAUAU GAGUAUAUUUGAUCAUAUUUUG AGUGGCAG |
| **4** | RHR705 09.1 | MRRLLKSFKTEIDPTEEQKIKIHKTIGTCRYIYNFYLFHN KERYDAGERFMSGKSFSVWLNNEYLPGHPEYFWIREV SSKSVKNTIENACTAFSRFFHHQSSFPRYKKKGRSDVK MYFVKNNPKDCLCERHRIKIPTLGWVRLKEKGYIPASK DGFVIRSGAVSMKAGRYYVSALVDIPAPEADGNFTDGI GIDLGLKDFAVVSNGTVYRNINKTARIRKLEKQLRRAQ RKLSRKYENLKKGESTQRANICKQKLKVQKLHHRIENI RTDHINKVISEIVKTKPSHITIEDLNISGMMKNRHLSKAV ASQKFYEFRTKLKIKCAEYGIELRVVDRWYPSSRICHCC SCIKKDLKLSDRIYRCTCGYIEDRDLNAARNLRDAETY EVA | 61 | UCCCGCAAACGUAAGUAUGUACC GAAGGCUAUUUCGGGAAUUAAC GACUGUGGAGUGUACUUAGGAU CUGUGAGUAGAGAUAACUCCGG UUACUCAAAAGCAUACACAAAG AAACAGUAAGCGGUAUUCGUGA GAAUCCGCAUUAUCUCGAUGUG AGUAUGUUUAAUCACAUUUUGA GUGGCAG |
| **5** | RHQ350 82.1 | MWKLLKSFKTEINPTEEQKVKIRKTIGTCRYIYNFYLAH NKELHDNGKKFMSGKSFSVWLNNEYLPQNPDKLWIKE VSSKSVKRSIENGCVAFTRFFKHQSAFPNFKKKGKSDV KMYFVKNNPKDCRCERHRINIPSLGWVRIKEKGYIPTT KDGYVIKSGTVSIKAGRYYVSVLVEIPDMLIDSSNNDGI GIDLGLKNFAIVSNGKTYKNINKSARLKKLEKQLRREQ KCLSRKYEYLKKGESTQRANIQKQKLKVQKLYHRLDN IHTDYINKIIAEIVKTKPSYITIEDLNVSGMMKNRHLSKA VASQKFYEFRTTLKSKCDDNNIELRVVDRFYPSSKLCH CCGSIKKDLKLSDRIYRCDCSYVADRDLNAALNLKDA | 62 | UGAAAAGCAAAUGUAAGUAUGU ACCGAAGGCUAUUUCGGGAAUU AACGACUGUGGAGUAUACAAGA ACUUGUGAGUAGCAUAUCUGUU UACAGAUUGUAAAAGCAUAUAC GAUGAAGCAGUAAGUAAUGUUC GUGAGAACUUACAACAUCUCGA UGUGAGUAUAUUUACACACAUU UUGAGUGGCAG |
| | | KTYKIA | | |
| **6** | RHS756 25.1 | MWKLLKSFKTEINPSEEQKVKIHKTIGTCRFIYNFYLAH NKELYDKGEKFMSGKSFSVWLNNEYLPQNPDKLWIKE VSSKSVKHSIENGCIAFTRFFKHQSAFPNLKKKGKSDVK MYFVKNNPKDCRCERHRINIPSLGWVRIKEKGYIPTTK DGYVIKSGTVSMKADRYYVSVLVEISNNKIANNSNAGI GIDLGLKDFAIVSNGKTYKNINKSARLKKHEKQLIREQR SLSRKYENLKKGESTQKANIQKQRLKVQKLHHRMDNI RTDYINKTIAEIVKTKPSYITIEDLNVKGMMKNRHLSKA VASQKFYEFRTKLQAKCNENGIELRVVDRWYPSSKTC HCCGAVKKDLKLSDRIFKCSCGYVEDRDFNAALNLRD AITYEVA | 63 | CUAAAACGCAAACGUAAGUAUG UACCGAAGGCUAUUUUCGGGAA UUUACGACUAUGGAGUGUACAA GAACUUGUGAGUAGAUAUGAUU UCGGUCAAUCCAAAGCAUACACG AUGAAAUAGUAAGUAAUGUUCG UGAGAACUUACAUUAUCUCGAU GUGAGCAUAUUUAAUCACAUUU UGAGUGGCAG |
| **7** | RHU002 56.1 | MRKLLKSFKTEINPTEEQKAKIRKTIGTCRYIYNFYLAH NKELYNKGEKFMSSNQFRVWLNNEYLPKHPECFWIKE AYSKAVTQAVNNGQAAFIRFFKHQSSFPRFKKKNRSDV KMYFVKNNPKDCRCQRHRINIPSLGWVRIKEKGYIPTT KDGYVIKSGHVSIKAGRYYVSALVEIPNNKIANSFNEGI GIDLGIKDFAIVSNGKIYKNINRSVRLKKLGKQLIREQK NPSRKYENLKKGESTQKANIQKQRLKVQKLHHKIDNIR TDHINKTIAEIVKTKPSYITIEDLNISGMMKNKHLSKAV ASQKFYEFRIKLQAKCNENGIELRVVDRWYPSSKTCHC CGAIKKDLKLSDRIFKCDCGYSEDRDFNAALNLRDATT YEIA | 64 | AUAAAUGCAAACGUAGGUAUGU ACCGAAGGCUAUUUCGGGAAUU UACGACUAUGGAGUGUACAAGA ACUUGUGAGUAGAUAUGAUUUC GGUCAAUCCAAAGCAUACACGA UGAAGUAGUAAGUAGAACUCGU GAGAGUCUACAUUAUCUCGAUG UGAGUAUGUUUAAUCACAUUUU GAGUAGCAG |
| **8** | RGT121 03.1 | MWKLLKSFKTEINPSEEQKVKIHKTIGTCRFIYNFYLAH NKELYDNGEKFMSSNKFRVWLNHEYLSEHPEYSWIKE VYSKAVTQAVNNGQTAFARFFNHESAFPNFKKKGKSD VKMYFVKNNPKDCRCERHRINIPSLGWVRIKEKGYIPT TKDGYVIKSGTVSMKADRYYVSVLVEILNNKIANNSNA GIGIDLGLKEFAIVSNGKTYKNINKSARLKKLEKQLIRE QRSLSRKYENLKKGESTQKTNIQKQRLKVQKLHHRIDN | 65 | CUAAAACGCAAACGUAAGUAUG UACCGAAGGCUAUUUUCGGGAA UUUACGACUAUGGAGUGUACAA GAACUUGCGAGUAGAUAUGAUU UCGGUCAAUCCAAAGCAUACACG AUGAAAUAGUAAGUAAUGUUCG UGAGAACUUACAUUAUCUCGAU |
| | | IRTDYINKTIVEIVKTKPSYITIEDLNVKGMMKNRHLSK AVASQKFYEFRTKLQAKCNENGIELRVVDRWYPSSKT CHCCGAVKKDLKLSDRIFKCSCGYVEDRDFNAALNLR DAITYEVA | | GUGAGCAUAUUUAAUCACAUUU UGAGUGGCAG |
| **9** | RHN048 53.1 | MARLLKSFKTEINPSEEQKVRIYKTIGTCRFIYNFYLSHN KELYDKGEKFMSSNKFRLWLNNEFLPDNPDKMWIKEA YSKAVTQAVNHAQTAFKKFFDHKTAFPRFKKKGKSDV KMYFVRNNPKDCRCERHRIHIPSLGWIRIKEKGYIPTTK DGYIIKSGTVSIKAGRFYVSALVEVPDRKITEPFNEGIGI DLGLKEFAVVSNGRIYKNINRSARIRKLEKQLIREQRRL SRKYEDLKKGETTQKANIQKQKRKVQKLHQRIQNIRTD YMNKIIAEIVKTKPSYITIEDLNVKGMMKNRHLSKAVA SQKFYEFRRKLQAKCTEKGMELRIADRWYPSSKKCHC CGCINKELKLSDRIYRCGCGYVEDRDFNAALNLRDART YKTA | 66 | AAAAGCAGAUGUAAGUAUGUAC CGAAGGCUAUUUCGGGAAUUUA CGACUGUGGAGUGUACAAGAAC CUGUGAGUAGAUAUGAUUUUGG UCAAUCAAAAGCAUACACAAAG AAGCAGUAAGUAGAACUCGUGA GAGUUUACAUUAUCUCGAUGUG AGUAUAAUUACACACAUUUUGA GUGGCAG |
| **10** | RHR462 18.1 | MRKLLKSFKTEINPTEEQKTKIRKTIGTCRFIYNFYLAH NKKLHEDGKKFMSSNKFRIWLNNEYLPQHPEYLWIKE AYSKAVTQSVNNGQTAFTRFFNHESAFPNFKKKGKSD VKMYFVKNNPKDCHCERHRIKIPSLGWVRIKEKGYIPT TKDGYVIKSGSVSIKADRYYVSVLVEVSDNKTINHFDE GIGIDLGLKDFAIVSNGRTYQNINKSAKLKKLERQLIRE QRCLSRKYENLKKGDATQRANIQKQKLKVQKLHHRID NIRTDYINKTIAEMVKIKPSYITIEDLNVKGMMKNRHLS KAVASQKFYEFRAKLLTKCNKSGIELRVVDRWYPSSKI CHCCGSIKKDLKLSDRIFKCRCGYIEDRDFNASLNLRDA ETYSIA | 67 | UUAAAACGCAAAUGUAAGUAUG UACCGAAGGCUAUUUCGGGAAU UUACGACUGUGGAGUGUACAUG AACUUGUGAGUAGAUAUGACUA CGGUCAAUCAAAAACAUACACA AUGAAGCAGUAAGUAAUGUUCG UGAGAACUUACAUUAUCUCGAU GUGGGUAUAUUUAAUCACAUUU UGAGUGGCAG |
| **11** | RGR696 73.1 | MRKLLKSFKTEINPTVEQKIKINKTIGTCRYVYNFYLGH NKALYDKGEKFMTGKSFSVWLNNEYIPNNPDKIWIKE AYSKAVKKSIEDGCTAFTRYFKHQSAFPNFKKKGKSDV KMYFVKNNPKDCRCERHKLNIPTLGWVRMKEKGYIPT | 68 | AACGCAAACGUAAGUAUGUACU GCGGGCUAUCGCAGGAAUUUAC GACUGUGGAGUGUACAAGAACU UGUGAGUAGCGUAUUGUUUACA |
| | | TKDGWKIKSGTVSIKADRYYVSVLVEIPDAKITDNSND GMGIDLGLKDLAIVSNGKTYKNINKSARVKKLEKKLRR EQRCLSRKYENLKKGESTQKNIQKQKLKVQRLHHKID NIRTDYINKSIAEIVKAKPSYITIEDLNVSGMMKNRHLS KAVASQKFYEFRTKLKAKCDENGIELRVVDRWYPSSKI CHCCGAVKKDLKLSDRIYRCDCGYIEDRDFNAALNLR DALTYEVA | | AUCGCCAAAGCAUACACAAUGA AGCAGUAAGAAGUAUCCGCAAG GACUUCAAUUUCUCGAUGUGUU UGAGUAUAUUUCAACACAUUUU GAGUGGCAG |
| **12** | RHC604 16.1 | MRKLLKSFKTEIDPTEEQKVRIHKTIGTCRFIYNFYLAH NKELHKNGERFMSSSKFRVWLNNEYLPNHPEYSWIKE AYSKAVTQAVNNGQTAFTRFFNHESNFPNFKKKGRSD VKMYFVKNNPKDCCCERHRIKIPSLGWVRIKEKGYIPT TKDEYVIKSGHVSIKADRYYVSVLVEIPDDKMVNNSNE GIGIDLGLKDLAIISNGKTYKNINKSAKLKKLEKQLIRG QRSLSRKYENSKKGESTQRANIQKQKIKVQKLYQKIDN IRTDYINKTIAEIVKTKPSFITIEDLNVKGMMKNRHLSK AVASQKFYEFRIKLQAKCNENGIELRVVDRWYPSSKTC HCCKNIKKDLRLSDRIFKCDCGYIEDRDLNAALNLRDA MTYEVA | 69 | AUAAACGCAAACGUAGGUAUGU ACCGAAGGCUAUUUCGGGAAUU UACGACUAUGGAGUGUACAAGA ACUUGUGAGUAGAUAUGAUUUC GGUCAAUCCAAAGCAUACACGA UGAAAUAGUAAGUAAAACUCGU GAGAGUUUACAUUAUCUCGAUG UGAGUAUAUUUAAUCACAUUUU GAGUGGCAG |
| **13** | RHB062 65.1 | MRKLLKSFKTEINPTIEQKIKINKTIGTCRYVYNFYLGH NKTLYDNGEKFMTGKSFSVWLNNEYIPNNPDKIWIKEA YSKAVKKSIEDGCTAFTRFFKHQSAFPNFKKKGKSDVK MYFVKNNTKDCRCERHRLNIPTLGWVRIKEKGYIPTTK DGWKIKSGTVSVKADRYYVSVLVEIPNVKIANNSNGGI GIDLGLKDLAIVSNGKTYKNINKSTRIKKLEKKLRREQR CLSRKYENLKKGESTQKNIQKQKLKVQRLHHKIDNIRT DYINKSIAEIVKTKPSYITIENLNVSGMMKNRHLSKAVA SQKFYEFRTKLKAKCDENGIELRVVDRWYPSSKICHCC GAIKKDLKLSDRIYRCDCGYVEDRDFNAALNLRDALT YEVA | 70 | UAAACGCAAACGUAAGUAUGUA CUGCGGGCUAUCGCAGGAAUUU ACGACUGUGGAGUGUACACGAA CUUGUGAGUAGCGUAUUGUUUA CAAUCGCCAAAGCAUACACAUCG AAGCAGUAAGAAGUAUCCGCAA GGACUUUAAUUUCUCGAUGUGU UUGAGUAUAUUUGAACACAUUU UGAGUGGCAG |
| **14** | RGN780 | MKRAYLIEIKPTTEQVSKIHQTIGVCRYVVYNLYISKNKE | 71 | AUAGAUUGUAUAUAUGUACGGU |
| | 93.1 | AYESEGKFLSGYDFSKWLNNIHTKECDQWIKEVSSKAV KQAIMNGDKAFKRFFKGLSKFPRYKKKRKQDVKCYFP KNNKTDWTVERHRVKVPTIGWVRLKEYGYIPKNATVK SGTVYQRAGRYFVSVLCERAEIRVKPMLNKVGLGIDLG LKDFAVRSDGKTHKNINKTVQVKKIEKRLKREQRSLSR KFENRKKRGEQSVTNKRANIDKNILRVQKLHARLANIR LAYVKSIVNDVVKTKPTFITVEDLNVKGMMKSKHLSK TIAKQSFFAFKTWLLTKCEEYGIELRQVDRFYPSSKLCS CCSQKKVDLKLSDRVYTCECGNVMDRDLNAAINLLQA KEYTILT | | GGGCUACAUCGGAAUUUACGCC UGUGGAGUGUCACAGCAAACUG UAGUAGCAAGUGAUUAGCGAGG CAGGGCACGUAGAAGCAGGAAU ACUCUAAGAAUAUACAUUUGUC UAUAUUUUUAGUAGCAG |
| **15** | RGR872 03.1 | MILAKKVRLNPTEEQERQLWKSVGTARWVYNWTLSK QEESYKNDGKFISDGELRKELTQLKKTEEYSWLLDVSN NVAKQAVKDACDAYKKFFKKLANKPRFKSRKRSKPSF YNDNVKLKVKEMLVLIEKVGWVKTSEQLPINTKYVNP RVSFDGKYWYLSVGIQQKHVHNDLTDNIIGIDVGVKEL AVCSNGMVFKNINKTKTVKKIEKRLRRLQRKVSRKYE MNKEGNRFVKTCNIIKTEKKIRLLHRRLMNIRTNHIHQT TNAIVKTKPSTVVMEKLNIKGMMKNKYLSKAVQNQKL YEFKRQMKYKCEFNGIKFIEADKWYPSSKTCSNCGQIK KDLKLSERIFRCDCGLEIDRDLNASINLAKLAN | 72 | CACCUAAAACGUUAACGCUAAU AUGUACGAUUCGUUGCAUCGGA AUUUACGCCUGUGGAGUGUUAC ACCAAACGAAAGUAGCUUCGGC AAAAUCGGACACGAUGAAUCAG GAAGCAAACAAGAAUUUAUAAG UUUUUAUAAUUUCUUGGCAACG G |
| **16** | RHU473 84.1 | MVKAIKVMLIPNNVQKTKMFQYAGASRFAYNWALAR EKENYEKGGRFIPDAELRKEFTKLRHSDEYAWLLSISN NVTKQAIKDACTAYKNFFKGLQKFPRFKYRKRSMPKF YQDNVKIRFSNTHVKFEGFSSGRKANKQKMNWVRLAE HGRIPTDAKYMNPRISFDGLNWWISVCVELPDCRETPN DDGVGIDLGIKNLAVCSDAVKYKNINKSQKVKKLEKQ KRGLQRSISRSYEKNKRGESYCKTNNVIKKKKLLLKRN HRLTNIRKNYLNQTISEIVNRKPRFICIEDLNVSGMMKN RHLSKAVQEQGFFRFRKQLEYKCSDKGIQLIVADRFYP SSKLCSCCGNIKKDLKLSDRIYRCECGNRIDRDFQASIN | 73 | CACUGAAACGUUAAUGCAAAUA UGUACGGAUACGUUAGUCCGGA AUUUACGCCUGUGGAGAGUACA AGAACUUGUGAGUAGAUUGAUA UUUAUAUCAUCAAAAGCAUACU CGUUGAAGCAGGAAUGAAACAU GGAAGUUUAUAACUUUUUAUAA GUUUUCAGUAACGG |
| | | LKIYGERFAS | | |
| **17** | RHU180 41.1 | MLKSFKTEIHPTEEQKVRIHKTIGTCRFIYNFYLAHNKE LYQNGEKFMSSSKFRVWLNHEYLPQHPEYLWIKEAYS KAVTQAVNHGQTAFKKFFNHESAFPKFKKKGRSDVKM YFVKNNPKDCRCERHRVNIPSLGWVRIKEKGYIPTTKE GYIIKSGHVSMKAGRYYVSALIEIPDNKTADLSNEGIGI DLGLKDFAIVSNGKTYKNINKSARLKKLEKRLVREQRC LSRKYENLKKGESTQRANIQKQKLKVQKLHHKIDNIRT DYINKTIAEIVKTKPSYITIEDLNVSGMMKNKHLSRAVA SQKFYEFRIKLKAKSRENGIELRVADRWYPSSKICHCCG TIKKDLKLSDRTFRCHCGYVEDRDFNAALNLRDVKTY KIA | 74 | GGAAGCAAAUGUAAGUAUGUAC CGAAGGCUAUUUCGGGAAUUAA CGACUGUGGAGUGUACAUGGAC CUACGAGUAGAUAUGAUUUCGG UCAAUCAAAAGUAUACACACUG AAGCAGUAAGUAAGAUUCGUGA GAACUUACAUUAUCUCGAUGUG AUAAGUAUAUUUAAUCACAUUU UGAGUGGCAG |
| **18** | RHV030 91.1 | MRRLLKSFKTEINPTEEQKIKIHKTIGTCRYIYNFYLFHN KERYDIGEKFMSGRAFSVWLNNEYLPKHPEYSWIREVS SKSVKNAIENACTAFSRFFHHQSGFPRYKKKGRSDVKM YFVRNNPKDCFCERHRIKIPTLGWVRLKEKGYIPTSRDG YVVRSGAVSMKAGRYYVSALIEVPTPKVDGNFSDGIGI DLGLKNFAVVSNGTVYRNINKTVQIRKLEKQLTRAQR KLSRKYENLRKGEFTQKANIQKQKLKVQKLYDRIENIR TDHINKTISEIVKTKPSHITIEDLNVSGMMKNRHLSKAV SSQKFYEFRTKLKAKCAEYGIELRVVDRWYPSSKTCHC CGCIKKDLKLSDRIYKCTCGYVEDRDFNAALNLRDART YKVA | 75 | UCCUGCAAACGUAAGUAUGUAC CGAAGGCUAUUUCGGGAAUUAA CGACUGUGGAGUGUACUUAGGA UCUGUGAGUAGAGAUAACGCAG GUUACUCAAAAACAUACACGAA GAAACAGUAAGCGGUAUUCGCG AGAAGCCGCAUUAUCUCAAUGU GAGUAUGUUUAAUCACAUUUUG AGUGGCAG |
| **19** | RHT787 32.1 | MRELLKSFKTEINPTAEQKVKINKTIGTCRFVYNFYLDH NKALHNKGEKFMTGKSFSVWLNNEYIPNNPDKAWIKE AYSKAVKKSIENGCIAFTRFFKHQSAFPNFKKKGKSDV KMYFVKNNPKDCRCERHRLNIPTLGWVRIKEKGYIPTT KDGWRIRSGTISIKAGRYYVSVLIEVPDVKNTNNNSKGI GIDLGLKDFAIISNGKIYKNINKSARVKKLENKLRREQR CFSRKYENLKKGESTQKNIQKQELKVQRLHHKIVNIRT | 76 | UUAACGCAACGUAAGUAUGUAC CGAAGGCUAUUUCGGGAAUUAA CGACUGUGGAGUGUACAAGAAC UUGUGAGUAGCGUAUUGCUUGC AAUCGCCAAAGCAUACACAUUG AAGCAGUAAGAAGUAUCCGUGA GGACUUCAGUUUCUCGAUGUGU |
| | | DYINKTIAEIVKTKPSYITIEDLNVSGMMKNKHLSKAVA SQKFYEFRTKLKAKCDENGIELRIVNRWYPSSKICHCCG AIKKDLKLSDRIYRCGCGYVEDRDFNAALNLRDALTYE IA | | UUGAGUAUAUUUAAACGCAUUU UGAGUGGCAG |
| **20** | RHR429 37.1 | MKKLLKSFKTEINPTVEQKIKINKTIGTCRYVYNFYLVH NRNLHDQCEKFMTGKSFSVWLNNEYIPNHPDKAWIKE AYSKAVKKSIEDGCTAFTRFFKHQSDFPNFKKKGKSDV KMYFVKNNPKDCRCERHRLNIPTLGWVRIKEKGYIPTT KDGWKIKSGTVSIRAGRYYVSALVEVPDATIYNNNND GVGIDLGLKDLAIVSNGKTYKNINKSARVKKLEKQLRR EQRCLSRKYESLKKGESTQKNIQKQKRKVQKLHHKIDN IRTDYINKTVAEIVKTKPSYITIEDLNISGMMKNRHLSK AIASQKFYEFRIKLKAKCDENGVELRVADRWYPSSKIC HCCGTIKRDLKLSDRIYRCSCGYIEDRDFNASLNLRDAI TYKIA | 77 | UGAACGCAAAUGUAAGUAUGUA CCGAAGGCUAUUUCGGGAAUUA ACGACUGUGGAGUGUACCAGAA CUUGUGAGUAGUGAGUUACUUG UAACCACAAAAGCAUACAUGGU GAAGCAGUAAGAAGUAUCCGUG AGGACUUCAAUUUCUCGAUGUG AUUGAGUAUGUUCAAACACAUU UUGAGUGGCAG |
| **21** | RGD889 09.1 | MRKLLKSFKTEINPTEEQKTKIRKTIGTCRFIYNFYLAH NKKLHEDGKKFMSSNKFRIWLNNEYLPQHPEYSWIKE AYSKAVTQSVNNGQTAFTRFFNHESAFPNFKKKGKSD VKMYFVKNNPKDCHCERHRIKIPSLGWVRIKEKGYIPT TKDGYVIKSGSVSIKADRYYVSVLVEVSDNKTINHFDE GIGIDLGLKDFAIVSNGRTYQNINKSAKLKKLERQLIRE QRCLSRKYENLKKGDATQRANIQKQKLKVQKLHHRID NIRTDYINKTIAEMVKIKPSYITIEDLNVKGMMKNRHLS KAVASQKFYEFRAKLLAKCNESGIELRVVDRWYPSSKT CHCCGSIKKDLKLSDRIFKCRCGYIEDRDFNAALNLRD AETYSIA | 78 | UUAAAACGCAAAUGUAAGUAUG UACCGAAGGCUAUUUCGGGAAU UUACGACUGUGGAGUGUACAUG AACUUGUGAGUAGAUAUGACUA CGGUCAAUCAAAAACAUACACA AUGAAGCAGUAAGUAAUGUUCG CGAGAACUUACAUUAUCUCGAU GUGGGUAUAUUUAAUCACAUUU UGAGUGGCAG |
| **22** | RHN303 53.1 | MKKLKRAYKIEINPTAEQKSKIHQTIGVSRFIYNFYIAH NKEIYAREGKFVSGMDFSKWLNNKYIPNNKDMKWIKE VSSKATKQAIMNGDKAFRDFFKKAKGFPKFKKKKNQD VKAYFPKNNKTDWTLERHKVKIPTLGWVRLKEFGYIP | 79 | AAUCAAAAGCACUUAUAUAUGU ACGGAGGGCUAACUUCGGAAUU UAAGCCUUUGGAGAGCCAUACA AAUCGUAGUAGCUUAGGCAAGG |
| | | VNSIVKSGTVSQKADKYYVSILVEEDDKKVYKSTNEGL GIDLGVKEFVVCSNGIKFKNIN | | CGAGGCUUUAUGAAGAAGGAAU AAUCUCCAUAUGGAUAUAUUUG ACCAUAUUUUGAGUAGCAG |
| | | KTSTVKKVEKKLKREQRKLSRKYESLKIRNKNLKEGRA TSQNIQKQLVKVQKLHQRLTNIRTDYINKIVSSIIKQKPS YITIEDLAVSNLMKNKHLSKAIASQKFFEFKTKLMFKC KENHIELRIVDRFYPSSKTCSSCGKVKKDLKLSDRVYK CDCGLTIDRDLNASINLKNAKEYKIA | | |
| **23** | RGC428 77.1 | MVKAIKVMLIPNNVQKTKMFQYAGASRFAYNWALAR EKESYEKGGRFISDSELRKEFTKLRHSDEYAWLLNISNN VTKQAIKDACTAYKNFFKGLQKFPRFKSRKRSMPKFYQ DNVKIQFSNTHVKLEGFSSSRKANKQKKNWVRLAEHG RIPTDVKYMNPRISFDGLNWWISVCVEFPDCKETLNDD GVGIDLGIKDLAVCSDAVKYKNINKSQKVKKLEKQKR RLQRSISRSYEKNKKGESYCKTNNVIKKEKLLLKRNHR LTNIRKNYLNQTISEIVNRKPRFICIEDLNVSGMMKNRH LSKAVQEQGFFWFRKQLECKCNDKGIQLIVADRFYPSS KLCSCCGNIKEDLKLSDRVYRCECGNMIDRDFQASINL KAYGERFAS | 80 | CACUGAAACGUUAAUGCAAAUA UGUACGGAUACGUUAGUCCGGA AUUUACGCCUAUGGAGAGUACA AGAACUUGUGAGUAGAUAGAUA UUUAUAUCAUCAAAAGCAUACU CGUUGAAGUAGGAAUGAAACAU GGAAGUUUAUAACUUUUUAUAA GUUUUCAGUAACGG |
| **24** | RGT871 74.1 | MVKAIKVMLIPNNVQKTKMFQYAGASRFAYNWALAR EKENYEKGGKFISDSELRKEFTKLRHSDEYAWLLNISN NVTKQAIKDACTAYKNFFKGLQKFPRFKSKKRSMPKF YQDNVKIQFSNTHVKFEGFSSSRKANKQKMNWVRLAE HGRIPTDVKYMNPRISFDGLNWWISVCVEFPDCKETLN DDGVGIDLGIKDLAVCSDAVKYKNINKSQKVKKLEKQ KRRLQRSISRSYEKNKKGESYCKTNNVIKKEKLLLKRN HRLTNIRKNYLNQTISEIVNRKPRFICIEDLNVSGMMKN RHLSKAVQEQGFFWLRKQLEYKCSDKGIQLIVADRFYP SSKLCSCCGNIKKDLKLSDREYRCECGNIIDRDFQASIN LKGYGERFAS | 81 | CACUGAAACGUUAAUGCAAAUA UGUACGGAUACGUUAGUCCGGA AUUUACGCCUAUGGAGAGUACA AGAACUUGUGAGUAGAUAGAUA UUUAUAUCAUCAAAAGCAUACU CGUUGAAGUAGGAAUGAAACAU AGAAGUUUGUAACUUUUUAUAA GUUUUCAGUAACGG |
| | | | 82 | CACUUUCAAGUUAAUACAGAUA |
| **25** | RGC760 | MIKSIKVRLCPNNKQLTKLFQYAGCARFAYNWAIAREQ | | |
| | 21.1 | ENYKQGDKFLSDNELRKEFTQLKKQSEYQWLSEVSNN VTKQAIKDACNTYKKFFKGQCKYPKFKSRKHSSPSFYQ DNIKIQFSDTHVKVEGFSMSKKKNKQKLNWIRLCEKGR IPTNCKYMNPRFTYDELHWYISVGIEVEDSTTIPLNNGI GIDLGIKDLAVCSDGNIYKNINKTTTVNKLEKRKRRLQ RSISRRYEKNKKGGIYCKTCNIIKKEKELLKVTKRLTNI RQNYLHQTTSEIIKRKPSFICIEDLNVSGMMKNKHLSKA VQQQCFYEFRKQMEYKSAWNNIPVIIADRFFPSSKLCSC CESIKKDLKLSDRIYKCECGNVIDRDYQASLNLKMYGE NVLKQQSVA | | UGUACUGAUUCGUUAGUCAGGA AUUUACGCCUAUGGAGAGUACA AGAACUUGUAAGUAGUGAUUGA CUUACCAACACGAAAGCAUACUC GUUGAAAUAGGAAUGAAACAUA AAAGUUUAUAACUUUUUAUAAG UUUUCAGUAACGG |
| **26** | RGR573 90.1 | MVKAIKVMLLPNNVQKTKMFQYAGASRFAYNWALAR EKESYEKGGKFISDSELRKEFTKLRHSDEYAWLLNISNN VTKQAIKDACTAYRNFFKGLQKFPRFKSRKRSMPKFYQ DNVKIQFSNTHVKLEGFSSSRKANKQKKNWVRLAEHG RIPTDVKYMNPRISFDGLNWWISVCVEFPDCKETLNDD GVGIDLGIKDLAVCSDAVKYKNINKSQKVKKLEKQKR RLQRSISRSYEKNKKGESYCKTNNVIKKEKLLLKRNHR LTNIRKNYLNQTISEIVNRKPRFICIEDLNVSGMMKNRH LSKAVQEQGFFGFRKQLEYKCNDKGIQLIVADRFYPSS KLCSCCGNIKKDLKLSDRVYRCECGNMINRDFQASINL KTYGERFAS | 83 | CACUGAAACGUUAAUGCAAAUA UGUACGGAUACGUUAGUCCGGA AUUUACGCCUAUGGAGAGUACA AGAACUUGCGAGUAGAUAGAUA UUUAUAUCAUCAAAAGCAUACU CGUUGAAGUAGGAAUGAAACAU GGGAGUUUAUAACUUUUUAUAA GUUUUCAGUAACGG |
| **27** | RGW55 202.1 | MYSKAVKKSIEDGCAAFTRFFKHQSDFPKFKKKGKSD VKMYFVKNNPKDCQCERHRLKIPTLGWVRIKEKGYIPT TKAGYMIRSGTVSVKAGRFYVSVLVEIPDVNINNNLNE GIGIDLGLKDFAIISNGKTYRNINKSAGLKKLEKQLIREQ RSLSRKYENLKKGESTQRANIQKQKLKVQKLHHKMDN IRTDYINKTIAEIVKTKPSYITIEDLNVKGMMKNRCLSK AVASQKFYEFRKRLKAKCDEKGIELRIADRFYPSSKTC HHCGSIRKNLKLSDRIYRCECGYVADRDLNAALNLKD AKTYKIA | 84 | UUAAAAGCAAAUGUAAGUAUGU ACCGAAGGCUAUUUCGGGAAUU AACGACUGUGGAGUGUACAAGA ACCUGUGAGUAGCGUAUUGCCU GCAAUCGUCAAAACAUACACAA UGAAGCAGUAAGAAGUAUUCGU GAGGACUUCAAUUUCUCGAUGU GUUUGAGUAUAUUUAAACAUAU UUGGAGUGGCAG |
| **28** | RHP088 69.1 | MRKLLKSFKTEINPTEEQKVKIRKTIGTCRFIYNFYLAH NKELHKNGEKFMSSNKFRVWLNNEYLPNHPEYSWIKE AYSKAVTQAVNNGQTAFTRFFNHKSSFPNFKKKGKSD VKMYFVKNNPKDCRCERHRINIPSLGWVRIKEKGYIPT TKDGYVIKSGHVSIKADRYYVSVLIEIPNVKIANNSNAG IGIDLGLKDFAIVSNGKTYKNINKSAKLKKLEKQLIREQ RSLSRKYENLKKGESAQKPNIQKQRLKVQKLHHRIDNI RTDYINKTIAEIVKTKPSYITIEDLNISGMMKNKHLSKA VASQKFNEFRTKLQAKCNENGIELRVVDRWFPSSKTCH CCKNIKKDLKLSDRIFKCDCGYIEDRDFNAALNLRDAT TYEVA | 85 | AUAAACGCAAACGUAGGUAUGU ACCGAAGGCUAUUUCGGGAAUU UACGACUAUGGAGUGUACAAGA ACUUGUGAGUAGAUAUGAUUUC GGUCAAUCCAAAGCAUACACGA UGAAAUAGUAAGUAAUGUUCGU GAGAACUUACAUUAUCUCGAUG UGAGUAUAUUUAAUCAUAUUUU GAGUGGCAG |
| **29** | RGF950 87.1 | MIKSIKVRLNPNNKQSTKLFQYAGCARFAYNWAISREQ DNYKQGNKFLSDSELRKEFTQLKKQSEYQWLNEVSNN VTKQAIKDACNTYKRFFKGQCKYPKFKSKKHSTPSFYQ DNIKIQFTDTHVKVESFSMSKKQNKQKLNWIKLCEKGR IPTGCKYMNPRFTYDGLYWYVSIGIEVNDNTTLPSNEGI GIDLGIKHLAICSDGNTYKNINKTQTVKKLEKKKRRLQ RSISRKYEKNKKGANYCKTSNIIKREKELLKLNHRLTN VRQNYLHQTTSEIVKREPSFICIEDLNVSGMMKNKHLS KAVQQQGFYEFRRQIEYKAMWNNIPVVIADRFFPSSKL CSCCGSIKKDLKLSDRIYKCECGNVIDRDYQAALNLKQ YGENVLKQQSVAYHFQVITDMY | 86 | UACGUUAGUCAGGAAUUUAUGC CUAUGGAGUGUACAAGAACUUG CGAGUAGAUUAUGAGAAAUCAU GUCAAAAGCAUAUACGAUGAAG UAGGAAUGAAACAUAAAGGUUU AUAACUUUUUAUAAGUUUUCAG UAACGG |
| **30** | RGH018 75.1 | MVKAIKVMLIPNNVQKTKLFQYAGASRFAYNWALARE KENYEKGGKFISDSELRKEFTRLRHSDEYAWLLNISNN VTKQAIKDACTAYQNFFKGLQKFPRFKSRKRSMPKFYQ DNVKIQFSNTHVKFEGFSSSRKANKQKRNWVRLAEHG RIPTDAKYRNPRISFDGLNWWISVCVEFPDSKETLNEDG VGIDLGIKDLAVCSDAVKYKNINKSQKVKKLEKQKRR LQRSISRSYEKNKKGESYCKTNNVIKKEKFLLKRNHRL TNIRKNYLNQTISEIVNRKPRFICIEDLNVRGMMKNRHL | 87 | GAUUGAAACGUUAAUGCAAAUA UGUACGGAUACGUUAGUCCGGA AUUUACGCCUAUGGAGAGUACA AGAACUUGUGAGUAGAUAGAUA UUUAUAUCGUCAAAAGCAUACU CGUUGAAGUAGGAAUGAAACAU AGAAGUUUAUAACUUUUUAUAA GUUUUCAGUAACGG |
| | | SKAVQEQGFFGFRKQLEYKCSDKGIQFIVADRFYPSSKL CSCCGNIKKDLKLSDRIYRCECGNRIDRDFQASINLKAY GEQFAS | | |
| **31** | RHU317 23.1 | MVKAIKVMLIPNNVQQTKLFQYAGASRFAYNWALARE KENYEKVGRFIPDTELRKEFTRPRNSDEYAWLLNVSNN VTKQAIKDACSAYKNFFKGLQRYPRFKAKKKSMPKFY QDNIKIQFRDTNVKFEGFSSSRKANKQKLNWVKLAEH GRIPTDAKYRNPRVSFDGLNWWISVCVEFPDCRDRSNH DGIGIDLGIKDLAICSDGNTYKNINKSQTAKKLEKCKRR LQRSVSRKYEHNKKGGSYCKTNNIVKKEKLLLKVNHR LANIRKDYLNQTTSEIANRKPRFICIEDLNVSGMIKNRH LSKAVQNQGFFEFRKQLEYKCNDRDFQASLNLKAYGE QFAS | 88 | CACUUAAAUGUUAAUGCAAAUA UGUACGGAUGCGUUAAUUCGGA AUUUACGCCUAUGGAGAGUACA AGAACUUGUGAGUAGAUAGGUA UUUAUGCCAUCAAAAGCAUACU CGUUGAAGUAGGAAUGAAACAU AAAAGUUUAUAACUUUUUAUAA GUUUUCAGUAACGG |
| **32** | RHQ 170 86.1 | MVKAIKVMLIPNNVQKTKMFQYAGASRFAYNWALAR EKENYEKGGRFISDSELRKEFTKLRHSDEYAWLLSISNN VTKQAIKDACIAYKNFFRGLQKFPRFKSRKRSMPKFYQ DNVKIQFSNTHVKFEGFSSSRKANKQKMNWVRLAEHG RIPIDANYMNPRISFDGLNWWISVCVEFPDCRETLNDD GVGIDLGMKDFAVCSDAVKYKNINKSQKVKKLEKQKR RLQRSISRSYEKNKKGESYCKTNHVIKKEKLLLKRNHR LTNIRKNYLNQTVSEIVNRKPRFICIEDLNVSGMMKNR HLSKAVQEQGFFLFRKQLEYKCSDKGIQLIVADRFYPSS KLCSCCGNIKKDLKLSDRVYRCECGNMIDRDFQASINL KAYGERFAS | 89 | CACUGAAACGUUAAUGCAAAUA UGUACGGAUACGUUAGUCCGGA AUUUACGCCUACGGAGAGUACA AGAACUUGUGAGUAGAUAGAUA UUUAUAUCAUCAAAAGCAUACU CGUUGAAGUAGGAAUGAAACAU AGAAGUUUAUAACUUUUUAUAA GUUUUCAGUAACGG |
| **33** | RGW42 851.1 | MILAKKVRIYPTKEQEQKLWQSVGTARFIYNYTLAKQE ENYKNGGKFISDGIIRKELTQLKKSELIWLNEVSNNVTK QAVKDACNAYKRFFKGLAKKPKFKIRKKSKPSFYNDTS KLKVKEKKVLIEKVGWIKTNEQIPIDVKYNNPRVTYDN KYWYISVGVEVDKKQEELTDISLGIDLGLKDLAVCSDG KVFKNINKTKEVKNLEKRLKQKQRQISRKYEINKIKKG | 90 | UUUCACAAACAAGAAAAUGCUA AUGUGUAGGAUUCGUUGUAUCC GAAUUUAAGCCUUUGGAGAGUU AUAUUAAACGAAAGUAGCUACG GCAAAAUCGGAUUCUAUGAAGA AGGAAAUAAACAAAUUUCUAUA |
| | | GSCQFIKTKNIEKLENTTKLMHRKLTNIRDNYIHQVTTS IVKTKPYRIVIEDLNVSGMIKNKHLSDSVRKQCFNKFRQ YITYKAELYGIGLVIADRFYPSSKTCSECGSIKKDLKLK DRVYRCPHCGVVIDRDYNASLNLSMYKLA | | UUUUUAUAGAUUUUUGGCAACG G |
| **34** | RGQ083 95.1 | MILAKKVRIYPTKEQEQKLWQSVGTARFIYNYTLAKQE ENYKNGGKFINDGVIRKELTQLKKSELTWLNEVSNNIT KQSVKDACKAYKRFFKNLANKPKFKSRKKSKPSFYND PIKLKIKEKKVLIEKVGWIKINEQIPINVKYNNPRITYDN KYWYLSVGIEVDKKQEGLTNISLGIDLGLKELAICSDGK VFKNINKTKKIKKLEKRLKQKQRQISRKYEINKIKKEGG KRYQFIKTKNIKKLENTTKLIYRKLANIRNNYIHQVTTSI VKTKPYRIVIEDLNVSGMMKNKHLSDSVKKQCFHKFR QYITYKSELNGIELVVADRFYPSSKTCSKCGFIKKDLKL KDRVYRCPHCGAVIDRDLNASLNLSMYKLA | 91 | UUUCACAAGCAAGAAAAUGCUA AUAUGUAGGAUUCGUUGUAUCC GAAUUUAAGCCUUUGGAGAAUC AUAUCAAACGAAAGUAGCUACG GCAAAAUCGGAUUCAAUAAAGA AGGAAAUAAACAAAUUUUUAUA UUUUUAUAGAUUUUUGGCAACG G |
| **35** | RHF509 70.1 | MLKAFKTEIAPTKEQRDKIIRSIGVARFLYNQYISYNRR LYKMYRRGMLDERQKYFVTAVDFDKYVNHKLKQELP WIDQCGSKARKKALVNAEQAFRRFFAGISRFPTFKKKT NQDVKLYFPRNNKGDWKIWRHKLMIPTLKQVKLKEY GYLPVGMTATSGTVSYVAGRFYVSVVVDIDAKSKYNK DLDSCYAIQTEGIGIDLGVKDLAVVSDGRVFENINKSSK VKRLEKRLRREQRRLSRKYVGKVDTVSANIEKQKLKV QKLHQRITRIREDYENKTVHEIVKQKPRFITVEDLNVKG MMKNRHLAKAVAAQRFNHLLSKLKRKAKIIGIELREV DRFYPSSKTCHACGHVHRGLKLKDRVYICPECGYRMD RDYNASLNLRDAKEYRVV | 92 | UAGAGAAGUUCUGAUAUAUGUA CCGAUGGCUAGUCGGGAACAGG CUCUUAUCCUUGCAUUGCAAGG AGCGUGAAGCCAACGCCUUCGGA GUGUUAUACAAACGUCAGUAGC CUGUCUUUCGGACGGCGAGGGC GAACACGAUGAACAAGGAAUAU UUCUAAGAUACAUACAUUGAGG GUUAUGUUCUUAGUAGCAG |
| **36** | RHF524 74.1 | MRNLLKSFKTELRLTEGQRDKVRRSLGICRFLANQYIA RNIWLYRLYQRGVLDEKQRRFVTANDFDKYVNHVLK KKFPWIDQCGSKARKKILVNAEQAFRRFFRGQAHFPRF KRKNQQNVKLYFPKNNKGDWTIWRHKIQIPTIGKVRL KEFGYLPQGVTIKNGHVSYKAGRYYVSVTAEIEEMSRY | 93 | UUCACGCUCUCACAUUUGAUAA AUGUACCGAUGGCUAGUCGGAA ACAGGCUGUUGCCUUGCCUAGCA AGGAAUACAGCCAACGCCUUUG GAGUGUACAAGAACUUGUGAGU |
| | | NNDLEASYHPRSDGIGVDLGVKSLAIVSDGRIFENINRT SRARRLERRLRREQRRLSRKYEMRKKKGGSTATASANI EKKKLSVQRLHRRLANLRRNHENQVIHALVEQKPRFIT VEDLNVTGMMKNRHLSKAVVQQRFYTFREKLRRKAEI IGIEVRIADRFYPSSKTCHACGHVHSGLKLKDRTYVCPA CGYKKDRDFNAALNLRDTKNYRIA | | AGUCUCAGGCUUGCCUGGGCAA AAGCAUACACGAUGAAUAAGGA AUUUUUCUAAGACAUGCACAUU UUUGUCUAUGUUUUUAGUAGCA G |
| **37** | RHM553 46.1 | MIRSIRVQLLPNNKQKTKLFQFAGAARYAYNWALDKQ MDNFREGRKLQTDHELRKAFTLLRHSEENKWLLDISN NVTKQAIKDLCIAYKNFFHKQKQPIYIKYSSKKITHYNR IGKSLTVYDMNGHPKFRSKKNGDFRFYQDNVKIQFTGT HVKLESLAGSRRKNRQRLNWVRLAERNRIPVRAKYTN PRIAFDGERWWLSVGIQTARKLKPLRGLSFVCKKRRYA RSEGIGIDLGIKDLAVLSDATKVGNINKTRAIRKLKKKR RRLQRQVSQKYQMNKKGDSYCKTSNLIKSEKRLLRINH RLANIRQNHVHQATTSIIKRKPSFVCLEDLNVRGMMKN RYLSEKIQEQNLYEFRRQIEYKAHWAKIPVVIADRWYP SSKTCVICGHVKKELNLLERTYICPVCGNIIDRDFQAAL NLKRYGEAEQVKLANY | 94 | AAAGAAGUCCAUGCAGAUAUGU ACCGAUUCGUUAGUCGGGAACA GACGUGCCUUCUUGCGAUGGUA AGGAGUGUCAUGUCAAAGCCUC CGGAGCAUUAUAUCAAACGUGA GUAGCUUGUCCUUCGGGAUGGC AAAAACGAAUGCGAUGAACGAG GAAUGAAACGUAAAAGUCAGUU UUUCAUCAAUAGACUAUGGCUU UUUAUAAGUUUUCAGUAACGG |
| **38** | RHQ991 95. | MEKGFKVQIFPNKEQKELMFKSFGCARFAYNWALAKE KENYEKGGKFISDMDLRKEFTQLKKKEEFKWLNEINN NVTKQAIKDLCKAYKNFFKKKAEHPRFKSKKKSKKSF YNDVYKIKFDDYKVRIESIGWMDMAENRIPRNCKYQN PKISFDGVKFWLSITCIVEDEENNLPKTEPIGIDMGIKTL MTCSNGMNFNRVSTKKEKKKLKRLQKKASRHYDKM MKTKSSDKSKNLLKLETLIRKQYQRISNVRLNNIHQAT TKLVKLNPKAIVVEDLNVKGMIKNKYLSEKIADCSFYEI YRQLEYKCENKGMEFIVADRFYPSSKICSNCGHKKEKL SLSERTYRCECCGNVIDRDFNASLNLKNLAYN | 95 | ACUGUAAACUAAAUGUGUACCG AUACGUUAGUCGGGAAUUUAAG CCUUUGGAGAAGUAUACAAACU AUAGUAGCUAUUCUUUGAAUGG UGAAAUAGGCUUUGGUGAAUUA GGAAAUAGAGUUUGUAGUUUUU UAUAAGUAAUUUAUAGAAAUUU AUAGAUUUUAUGUAACGG |
| **39** | RHR686 82.1 | MVKAIKVMLIPNNIQKTKMFQYAGASRFAYNWALARE IENYEKGGKFISDAELRKEFTKLRHSDEYAWLLSISNNV | 96 | CACUAAAACGUUAGUGCAAAUA UGUACGGGUACGUUAAUUCGGA |
| | | TKQAIKDACTAYKNFFKGLQKFPRFKPKKRSIPKFYQD NIKIQFSNTHVKFEGFSSSRKRNKQKLNWVRLAEHGRIP TDAKYMNPRISFDGLNWWISVCVEFPDCIEKLNDDGIGI DLGIKDLAICSDGAKYKNINKSQKVKKLEKQKRRLQRS ISRSYEKNKKGESYCKTNNVIKKEKLLLKRNHRLTNIR KNYLNQTTSEIVSRKPRFICIEDLNVSGMMKNRHLSKA VQNQGFFEFRKQLEYKCSDKGIQLIVADWFYPSSKLCS CCGNIKKDLKLSDRIYKCDCGNVIDRDFQASINLKAYG ERVAS | | AUUUACGCCUAUGGAGAGUACA AGAACUUGUGAGUAGAUAGAUA UUUAUAUCAUCAAAAGCAUACU CGUUGAAGUAGGAAUGAAACAU AGAAGUUUAUAACUUUUUAUAA GUUUUCAGUAACGG |
| **40** | RHR712 12.1 | MWKLLKSFKTEINPSEEQKVKIHKTIGTCRFIYNFYLAH NKELYNNGEKFMSSNRFRVWLNNEYLSEHPEYSWIKE AYSKAVTQSVNNGQTAFTRFFNHESAFPKFKKKGRSD VKMYFVKNNPKDCRCERHRINIPSLGWVRIKEKGYIPT TKDGYVVKSGTVSMKADRYYVSVLVEISDNKIADNSN AGIGIDLGLKDFAIVSNGKTYKNINKSARLKKLEKQFIR EQRCLSRKYENLKKGEVTQRANIQKQKLKVQKLHHKI DNIRTDYINKTIAEIVKTKPSYITIEDLNVNGMMKNRHL SKAVASQKFYEFRTKLQIKCNENGIELRIVDRWYPSSKT CHCCGAIKKDLKLSDRIFKCSCGYVEDRDLNAALNLRD AITYEVA | 97 | CUAAAACGCAAACGUAAGUAUG UACCGAAGGCUAUUUUCGGGAA UUUACGACUGCGGAGUGUACAA GAACUUGUGAGUAGAUAUGAUU UCGGUCAAUCAAAAACAUACAC GGUGAAGCAGUAAGUAGAACUC GUGAGAGUCUACGUUAUCUCGA UGUGAGUAUAUUUACACAUAUU UUGAGUGGCAG |
| **41** | RGG646 15.1 | MLTTKKVRLKLTPEQEIQFRKSCGVARWAYNYLLSEK QRVYDEYISNGKTGKKTISEGEVRKYINNVLKPTTHQW LKEVGSNVMKQAVKDADNAYKNFFNGLSKRPKFKSK KSSKQSFYVNYESLTRINGGFKGEKLGFVKTSEPLPKLA DGEKYANPRITFDGKYWYLSVVYNIEPKSVQLTNESLG IDLGVKNLAICSNGVTYKNINKSKRVKALKRTLKREQR KLSRKIECNIIGYANNRKPIFRTPLQGCKNIQKQIHYIKLI NRKINSIRNNHLHQATAEIVKTKPFQIVMETLNITGMM KNKHLAKAIAEEKLFEFKRQIKYKAEMYGIKVVEVPTF YPSSKTCSVCGCINTNLKLSDRVYHCDSCGITLDRDLN | 98 | UUCACUAUAAAGAAUUUUAUAA CUAUGUACCUAUCGUUACUGGG GAAUUUAAGUCUACAGAGUGUU AUAACAAAUGAGAGUAGCUUAG GCAAAAUCAGACACGAUGAAGU AGAAAGUCUAAUUCGUGAGAAU AGACAUGGUGUAGAUAUUUAUA AAUUAUAAUAGAUUUGUACAUA UUUAUCGUAACGG |
| | | AAINLANYKVI | | |
| **42** | RHU265 42.1 | MRKLLKSFKTEINPTVEQKIKINKTIGTCRYVYNFYLGH NKALYDKGEKFMTGKSFSVWLNNEYVPDNPDKIWIKE AYSKAVKKSIEDGCTAFTRFFKHQSAFPNFKRKGKSDV KMYFVKNNPKDCRCERHRLNIPTLGWVRMKEKGYIPT TKDGWKIKSGTVSIKADRYYVSVLVEIPDAKIANNSND GIGIDLGLKDLAIVSNGKTYKNINKSARVKKLEKKLRR EQRGLSRKYENSKKGESTQKNIQKQKLKVQRLHHKID NIRTDYINKSIAEIVKTKPSYITIEDLNVSGMMKNRHLSK AVASQKFYEFRTKLKAKCDENGIELRVVDRWYPSSKV CHCCGAIKKDLKLSDRIYRCDCGYVEDRDFNAALNLR DALTYEVA | 99 | UGAACGCAAACGUAAGUAUGUA CUGCGGGCUAUCGCAGGAAUUU ACGACUGUGGAGUGUACAAGAA CUUGUGAGUAGCGUAUUGUUUA CAAUCGCCAAAGCAUACACAAUC AAGCAGUAAGAAGUAUCCGCAA GGACUUCAAUUUCUCCAUGUGU UUGAGUAUAUUUAAACAUAUUU UGAGUGGCAG |
| **43** | RGH377 93.1 | MWKLLKSFKTEINPSEEQKVKIHKTIGTCRFIYNFYLAH NKELYDNGEKFMSSNKFRVWLNHEYLSEHPEYSWIKE VYSKAVTQAVNNGQTAFARFFNHESAFPNFKKKGKSD VKMYFVKNNPKDCRCERHRINIPSLGWVRIKEKGYIPT TKDGYVIKSGTVSMKADRYYVSVLVEILNNKIANNSNA GIGIDLGLKDFAIVSNGKTYKNINKSARLKKLEKQLIRE QRSLSRKYENLKKRESTQKTNIQKQRLKVQKLHHRIDN IRTDYINKTIAEIVKTKPSYITIEDLNVKGMMKNRHLSK AVASQKFYEFKTKLQTKCNENGIELRVVDRWYPSSKTC HCCGAVKKDLKLSDRMFKCSCGYVEDRDFNAALNLR DAITYEVA | 100 | CUAAAACGCAAACGUAAGUAUG UACCGAAGGCUAUUUUCGGGAA UUUACGACUAUGGAGUGUACAA GAACUUGCGAGUAGAUAUGAUU UCGGUCAAUCCAAAGCAUACACC AUGAAAUAGUAAGUAAUGUUCG UGAGAACUUACAUUAUCUCGAU GUGAGCAUAUUUAAUCACAUUU UGAGUGGCAG |
| **44** | GUT_G ENOME 084711_ 28_1717 _2925_+ | MIKSIKVRLNPNNKQSTKLFQYAGCARFAYNWAISREQ DNYKQGNKFLSDSELRKEFTQLKKQSEYQWLNEVSNN VTKQAIKDACNTYKRFFKGQCKYPKFKSKKHSTPSFYQ DNIKIQFTDTHVKVESFSMSKKQNKQKLNWIKLCEKGR IPTGCKYMNPRFTYDGLYWYVSIGIEVNDNTTLPSNEGI GIDLGIKHLAICSDGNTYKNINKTQTVKKLEKKKRRLQ RSISRKYEKNKKGANYCKTSNIIKREKELLKLNHRLTN | 101 | UACGUUAGUCAGGAAUUUAUGC CUAUGGAGUGUACAAGAACUUG CGAGUAGAUUAUGAGAAAUCAU GUCAAAAGCAUAUACGAUGAAG UAGGAAUGAAACAUAAAGGUUU AUAACUUUUUAUAAGUUUUCAG UAACGG |
| | | VRQNYLHQTTSEIVKREPSFICIEDLNVSGMMKNKHLS KAVQQQGFYEFRRQIEYKAMWNNIPVVIADRFFPSSKL CSCCGSIKKDLKLSDRIYKCECGNVIDRDYQAALNLKQ YGENVLKQQSVAYHFQVITDMY | | |
| **45** | GUT_G ENOME 112273_ 39_1428 3_15425 _+ | MLIPNNVQKTKLFQYAGASRFAYNWALAREKETYEKG GKFISDSELRKEFTRLRHSEEYAWLLNISNNVTKQAIKD ACTAYQNFFKGLQKFPRFKSRKRSMPKFYQDNVKIQFS NTHVKFEGFSSSRKANKQKRNWVRLAEHGRIPTDVKY RNPRISFDGLNWWISVCVEFPDSKETLNEDGVGIDLGIK DLAVCSDAVKYKNINKSQKVKKLEKQKRRLQRSISRSY EKNKKGESYCKTNNVIKKEKLLLKRNHRLTNIRKNYLN QTISEIVNRKPRFICIEDLNVRGMMKNRHLSKAVQEQGF FLFRKQLEYKCSDKGVQLIVADRFYPSSKLCSCCGNIKK DLKLSDRVYRCECGNRIDRDFQASINLKAYGEQVAS | 102 | GACUGAAACGUUAAUGCAAAUA UGUACGGAUACGUUAGUCCGGA AUUUACGCCUAUGGAGAGUACA AGAACUUGUGAGUAGAUAGAUA UUUAUAUCAUCAAAAGCAUACU CGUUGAAGUAGGAAUGAAACAU AAAAGUUUAUAACUUUUUAUAA GUUUUCAGUAACGG |
| **46** | SRR735 2028_38 0524_48 115_816 _1829_- | MSSNQFRVWLNNEYISKHPECFWIKEAYSKAVTQAVN NGQAAFTRFFKHQSSFPRFKKKNRSDVKMYFVKNNPK DCRCQRHRINIPSLGWVRIKEKGYIPTTKDGYVIKSGHV SIKAGRYYVSALVEIPNNKIANSSNEGIGVDLGIKDFAV VSNGKVYKNINRSARLKKLEKQLIREQRSLSRRYENLK KGESTQKANIQKQRLKVQKLHHRIDNIRTDHINKTIAEI VKTKPSYITIEDLNISGMMKNRYLSKAVASQKFYEFRIK LQAKCNENGIELRVVDRWYPSSKTCHCCGAIKKDLKLS DRIFKCDCGYTEDRDLNAALNLRDAMTYEVA | 103 | AUAAACGCAAACGUAGGUAUGU ACCGAAGGCUAUUUCGGGAAUU UACGACUAUGGAGUGUACAAGA ACUUGUGAGUAGAUAUGAUUUC GGUCAAUCCAAAGCAUACACGA UGAAGUAGUAAGUAGAACUCGU GAGAGUCUACGUUAUCUCGAUG UGAGUAUAUUUACACAUAUUUU GAGUGGCAG |
| **47** | GUT_G ENOME 129951_ 100_587 3_7024_ + | MLKSFKTEIDPTPEQKVKINKTIGTCRYVYNVYLSHNK ALYDKGEKFMTGKSFSVWLNNEYIPNNPDKAWIKEVS SKAIKKSIEDGCTAFRRFFKHQSSFPSCKKKGRSDVKM YFVKNNPKDCKCERHRISIPTLGWMRMKEKGYIPTTKD GYAIKSGTVSMKAGRYYVSALVEVPNVNVRNNTNDG VGIDLGLKDLAIVSNGKIYKNINKSARLKKFEKRLRREQ RCLSRKYDNRKKGESTQKNIQKQRLKVQRLHHKIENIR | 104 | AUAAACGCAAACAUAAGUAUGU ACCGAAGGCUAUUUCGGGAAUU AACGACUGUGGAGUGUACAAGA ACUUGUGAGUAGCGUAUUUUUU AUUUCUUGCAAUCGCCAAAACA UACACAAUGAAGCAGUAAGAAG AAUCCGUGAGGACUUCAAUUUC |
| | | TDHINKTIAEIVKTKPSYITIEDLNVSGMMKNRHLSKAV ASQKFYEFKIKLKAKCEDNGIELRVVDRFYPSSKLCHC CGRIKKDLKLSDRIYKCECGYIEDRDYNAALNLRDAVT YEVA | | UCGAUGUGUUUGUGUAUAUUUA AUCACAUUUUGAGUGGCAG |
| **48** | WP_005 609868.1 | MTGKSFSVWLNNEYIPNNPEKVWIKEAYSKAVKKSIED GYTAFTRFFKHQSAFPNFKKKGKSDVKMYFVKNNPKD CRCERHRLNIPTLGWVRIKEKGYIPTTKDGWKIKSGTLS IKAGKYYVSVLVEIPDAKIANNSNDGIGIDLGLKDLAVV SNGKTYKNINKSARVKKLEKKLRREQRCLSRKYENIKE GESTQKNIQKQKLKVQRLHHKIDNIRTDYINKSIAKIVK TKPSYITIEDLNVSGMMKNRHLSKAVASQKFYEFRTKL KAKCDENGIELRVVDRWYPSSKICHCCGAIKKDLKLSD RIYRCDCGYIEDRDLNAALNLRDASTYEIA | 105 | UGAAAGCAAACGUAAGUAUGUA CUGCGGGCUAUCGCAGGAAUUU ACGACUGUGGAGUGUACAAGAA CUUGUGAGUAGCGAAUUGUUUA CAAUCGCCAAAGCAUACACAUUG AAGCAGUAAGAAGUAUCCGCAA GGACUUCAAUUUCUCGAUGCGU UUGAGUAUAUUUCAACACAUUU UGAGUGGCAGA |
| **49** | WP_012 741946.1 | MLKSFKTEISPTIEQKIKINKTIGTCRYVYNFYLDHNKTL YDNGEKFMTGKSFSVWLNNEYIPNNPDKIWIKEAYSKA VKKSIEDGCTAFTRFFKHQSAFPNFKKKGKSDVKMYFV KNNTKDCRCERHRLNIPTLGWVRIKEKGYIPTTKDGWK IKSGTVSVKADRYYVSVLVEIPDVKIANNSNGGIGIDLG LKDLAIVSNGKTYKNINKSTRIKKLEKKLRREQRCLSRK YENLKKGESTQKNIQKQKLKVQRLHHKIDNIRTDYINK SIAEIVKTKPSYITIENLNVSGMMKNRHLSKAVASQKFY EFRTKLKAKCDENGIELRVVDRWYPSSKICHCCGAIKK DLKLSDRIYHCDCGYVEDRDFNAALNLKDALTYEVA | 106 | UCAACGCAAACGUAAGUAUGUA CUGCGGGCUAUCGCAGGAAUUU ACGACUGUGGAGUGUACAAGAA CUUGUGAGUAGCGUAUUGUUUA CAAUCGCCAAAGCAUACACAAUG AAGCAGUAAGAAGUAUCCGCAA GGACUUUAAUUUCUCGAUGUGU UUGAGAAUAUUUAAACACAUUU UGAGUGGCAGA |
| **50** | BBH267 08.1 | MLKSFRTEIHPTPEQKVKINKIIGTCRYIYNFYLSNNKAL HDQGEAFMSGKAFSVWLNNEYIPDNPDKAWIKEVYSK AVKKSIEDGCLAFTRFFKHQSGFPKFKKKGKSDVKMYF VKNNPKDCICERHRIKIPALGWVRLKEKGYIPTTKDGW KIKSGTVSMKADRYYVSVVVEIPDTKPFCDSGEGIGIDL GLKDFAIVSNGKTYKNINKSAKLKKLEKQLRRAQRCLS RKYENLKKGESTQKNIQKQRLKVQKLYQRIDNIRTDY | 107 | ACAAGCAAAUGUAAGUAUGUAC CGAUGGCUUAGUCGGGAAUUUA CGACUGUGGAGUGUACAAGAAC UUGUGAGUAGUAUGUCACUUGU GACUACAAAAGCAUACACGAAG AAGCAGUAAGUAAUGUUCGUGA GAACUUACAUAUCUCGAUGUGU |
| | | MNKTIAEIVKTKPSYITIEDLNVKGMMKNRHLSKAVAS QKFYEFRTKLKNKCDENGIELRVVDRWYPSSKLCHCC GAIKKDLKLSDRIYKCNCGYIADRDYNASLNLRDAKTY KIA | | UUGAGUAUAUUUAAACACAUUU UGAGUAGCAGA |
| **51** | GUT_G ENOME 239804 2_85784 _86938_ + | MLKSFKTEINPTEEQKVKIRKTIGTCRYIYNFYLAHNKE LYDKGEKFMSGKSFSVWLNNEYLPQNPDKLWIKEVSS KSVKRSIENGCIAFTRFFKHQSAFPNFKKKGKSDVKMY FVKNNPKDCRCERHRINIPSLGWVRIKEKGYILTTKDGY AIKSGHVSIKADRYYVSVLVEIPNNKIANNSNEGIGIDL GLKDFAIVSNGKTYKNINKSAKLKKLEKQLIKEQRSLSR KYENLKKGESTQKTNIQKQRLKVQKLHHRIDNIRTDYI NKTIAEIVKTKPSYITIEDLNISGMMKNKHLSKAVASQK FYEFRIKLQAKCKENGIELRVVDRWFPSSKTCHCCGAV KKDLKLSDRIFKCSCGYVEDRDFNAALNLRDAATYEV A | 108 | AUAAAACGCAAACGUAGGUAUG UACCGAAGGCUGUUUCGGGAAU UUACGACUAUGGAGUGUACAAG AACUUGUGAGUAGAUAUGAUUU CGGUCAAUCCAAAGCAUACACGA UGAAGUAGUAAGUAGAACUCGU GAGAGUCUACGUUAUCUCGAUG UGAGUAUAUUUAAUCACAUUUU GAGUGGCAG |
| **52** | GUT_G ENOME 047820_ 36_478_ 1632_- | MLRSFKTEINPSEEQKVKIHKTIGTCRFIYNFYLAHNKE LYDNGEKFMSSNRFRVWLNNEYLSEHPEYLWIKEAYS KAVTQSVNNGQTAFTRFFNHESAFPKFKKKGRSDVKM YFVKNNPKDCRCERHRINIPSLGWVRIKEKGYIPTTKDG YVIKSGTVSIKADRYYVSVLVELPDNKTADNSNEGIGID LGLKDFATVSNGKTYKNINKSAKLKKLEKQLIREQRSL SRKYENLQKGESTQRANIQKQKLKVQKLHHRIDNIRTD YINKTIAEIVKTKPSYITIEDLNVKGMMKNRHLSKAVAS QKFYEFRIKLQAKCTENGIELRVADRWYPSSKTCHCCK NIRKDLKLSDRIFKCDCGYIEDRDLNAALNLRDAMTYK VA* | 109 | AUAAACGCAAACGUAGGUAUGU ACCGAAGGCUAUUUCGGGAAUU UACGACUGUGGAGUGUACAAGA ACUUGUGAGUAGAUAUGAUUUC GGUCAAUCCAAAGCAUACACGA UAAAGCAGUAAGUAGAACUCGU GAGGGUCUACAUUAUCUCGAUG UGAGUAUAUUUAAUCACAUUUU GAGUGGCAG |
| **53** | GUT_G ENOME 001213 4_16007 | MRKLLKSFKTEINPTEEQKVKIRKTIGTCRFIYNFYLAH NKELHKNGEKFMSSNKFRVWLNNEYLPNHPEYSWIKE AYSKAVTQAVNNGQTAFTRFFNHKSSFPNFKKKGKSD VKMYFVKNNPKDCRCERHRINIPSLGWVRIKEKGYIPT | 110 | AUAAACGCAAACGUAGGUAUGU ACCGAAGGCUAUUUCGGGAAUU UACGACUAUGGAGUGUACAAGA ACUUGUGAGUAGAUAUGAUUUC |
| | 5_16123 8_+ | TKDGYVIKSGHVSIKADRYYVSVLIEIPNVKIANNSNAG IGIDLGLKDFAIVSNGKTYKNINKSAKLKKLEKQLIREQ RSLSRKYENLKKGESAQKPNIQKQRLKVQKLHHRIDNI RTDYINKTIAEIVKTKPSYITIEDLNISGMMKNKHLSKA VASQKFNEFRTKLQAKCNENGIELRVVDRWFPSSKTCH CCKNIKKDLKLSDRIFKCDCGYIEDRDFNAALNLRDAT TYEVA | | GGUCAAUCCAAAGCAUACACGA UGAAAUAGUAAGUAAUGUUCGU GAGAACUUACAUUAUCUCGAUG UGAGUAUAUUUAAUCAUAUUUU GAGUGGCAG |
| **54** | GUT_G ENOME 022845_ 45_3955 _5109_+ | MLKSFKTEINPTEEQKVKIRKTIGTCRYIYNFYLAHNKE LHDNGEKFMSGKSFSVWLNNEYLPKNPDKLWIKEVSS KSVKRSIENGCVAFTRFFKHQSAFPNFKKKGKSDVKMY FVKNNPKDCRCERHRMNIPSLGWVRMKEKGYVPTTKD GYVIKSGHVSIKADRYYVSVLIEIPNSKTADNSNEGIGID LGLKDFAIVSNGKTYKNINKSAKLKKLEKQLIREQRSLS RKYENLKEGESTQRANIQKQKLKVQKVHHKIDDIRTDY INKTIAEMVKTKPSYITIEDLNVSGMMKNKHLSKAVAS QKFYEFRTKLQAKCKENGIELRVVDRWYPSSKTCHCC GAIRKDLKLSDRIFECDCGYIEDRDFNAALNLRDATTYE VA | 111 | AUAAACGCAAACGUAGGUAUGU ACCGUGGGCUUACACGGGAAUU AACGACUGUGGAGUGUACAAGA ACUUGUGAGUAGAUAUGAUUUU GGUCAAUCCAAAGCAUACACGA UGAAACAGUAAGUAAAACUUGU GAGAGUUUACAUUAUCUCGAUG UGAGUAUAUUUACACACAUUUU GAGUGGCAG |
| **55** | GUT_G ENOME 112567_ 131_185 5_3009_ | MLKSFKTEINPTEEQKVKIRKTIGTCRYIYNFYLAHNKE LHDNGEKFMSGKSFSVWLNNEYLPKNPDKLWIKEVSS KSVKRSIENGCVAFTRFFKHQSAFPNFKKKGKSDVKMY FVKNNPKDCRCERHRMNIPSLGWVRMKEKGYVPTTKD GYVIKSGHVSIKADRYYVSVLIEIPNNKIANHSKEGIGID LGLKDFAIVSNGKTYKNINKSAKLKKLEKQLIREQRSLS RKYENLKKGESTQRANIQKQKLKVQKLHHKIDDIRTDY INKTIAEMVKTKPSYITIEDLNVSGMMKNKHLSKAVAS QKFYEFRTKLQAKCKESGIELRVVDRWYPSSKTCHCCG AIKKNLKLSDRIFQCDCGYIEDRDFNAALNLRDATTYE VA | 112 | AUAAACGCAAACGUAGGUAUGU ACCGUGGGCUUACACGGGAAUU AACGACUGUGGAGUGUACAAGA ACUUGUGAGUAGAUAUGAUUUU GGUCAAUCCAAAGCAUACACGA UGAAACAGUAAGUAAAACUUGU GAGAGUUUACAUUAUCUCGAUG UGAGUAUAUUUACAUACAUUUU GAGUGGCAG |
| **56** | GUT_G | MLKSFKTEINPTEEQKVKIRKTIGTCRYIYNFYLAHNKE | 113 | GGACGCAAAUGUAAGUAUGUAC |
| | ENOME 034484_ 15_4023 _5177_- | LHDNGEKFKSGKSFSVWLNNEYLPQNPDKLWIKEVSS KSVKHSIENGCVAFTRFFKHQSAFPNFKKKGRSDVKMY FVKNNPKDCRCERHRVNIPSLGWVRIKEKGYIPTTKEG YIIKSGHVSMKAGRYYVSALIEIPDNKTADLSNEGIGIDL GLKDFAIVSNGKTYKNINKSARLKKLEKRLVREQRCLS RKYENLKKGESTQRANIQKQKLKVQKLHHKIDNIRTDY INKTIAEIVKTKPSYITIEDLNVSGMMKNKHLSRAVASQ KFYEFRIKLKAKSRENGIELRVADRWYPSSKICHCCGMI KKDLKLSDRIFRCHCGYVEDRDFNAALNLRGVKTYKIA | | CGAAGGCUAUUUCGGGAAUUAA CGACUGUGGAGUGUACAUGGAU CUACGAGUAGAUAUGAUUUCGG UCAAUCAAAAGUAUACACAAGG AAGCAGUAAGUAAGAUUCGUGA GAACUUACAUUAUCUCGAUGUG AUAAGUAUAUUUAAUCACAUUU UGAGUGGCAG |
| **57** | GUT_G ENOME 040537_ 31_7690 _8853_+ | MRTLLKSFKTEINPTEEQKVKIRKTIGTCRYIYNFYLAH NKELHENGKKFMSGKRFSVWLNNEYLPKNPDKLWIKE VSSKSVKRSIENGCVAFTRFFKHQSDFPNFKKKGKSNV KMYFVKNNPKDCRCERHRINIPSLGWVRIKEKGYVPTT KDGYVIKSGHVSIKADRYYVSVLVELPDNKTADNSNE GIGIDLGLKDFAIVSNGKTYKNINKSTKLKKLEKQLIRE QRSLSRKYENLKKGESTQRANIQKQKLKVQKLHHKID NIRTDYINKTIAEIVKTKPSYITIEDLNVSGMMKNKHLS RAVASQKFYEFRIKLKAKSRENGIELRVADRWYPSSKIC HCCGMIKKDLKLSDRIFRCHCGYVEDRDFNAALNLRG VKTYKIA | 114 | GGACGCAAAUGUAAGUAUGUAC CGAAGGCUAUUUCGGGAAUUAA CGACUGUGGAGUGUACAUGGAU CUACGAGUAGAUAUGAUUUCGG UCAAUCAAAAGUAUACACAAGG AAGCAGUAAGUAAGAUUCGUGA GAACUUACAUUAUCUCGAUGUG AUAAGUAUAUUUAAUCACAUUU UGAGUGGCAG |

For RNA sequences, the "U" in a sequence (such as any one of SEQ ID NOs: 58-114) may be shown as "T" in the sequence listing.

The sequences of the proteins of additional 42 RAD (IS607 TnpB) systems and corresponding RNAs are described below. For RNA sequences, the "U" in the following sequences may be shown as "T" in the sequence listing.

No. 58 (Numbering continues sequentially after Table 2):
> ERR3307045_63958_30277_4897_6000_-(SEQ ID NO: 150) 
MVYKIEISPTQEQKSIIFRTFDATRLVYNYFIEVNKARHENGYPYMGNLEF
SKTFNNYMSRLEWVRDKSLKISSKAVKEAMKDADKAYQRFFKGISRYPRFKSRREP
RKSYYLCGSIHVKRHAVRLPNLGWVLLKEKNYIPAGSGVVSCRLIYEGGRFYVAILT
QDNNSVNNQLSNDGLGVDVGIRFLATDSDKDVYPNINKSSRVKHLKKRLKREQRK
LVRRNKKSLRREKTRQIINRIHYKISCICIDHKNKVVSALVKKKPSYITVETLNIKGML
KNRHLSRVIHEQGLHDFMTRLANKARQYGIELRRVSQYFASSKLCSTCGAKNDPKH
KTFWVCTSCGTRHDRDINASLNLLNATQYTIDK*
Associated RNA sequence (SEQ ID NO: 192)

UAAAACCAUAGUUAUAAGGACUUCACAUGACCUAAAACAUUAUAG
UACAAUGAAUAAUGAGACCAAUUAACCCGUUAUGUCUCCAAAAACUACCGUU
GGUUCUACCGUGGGUUGGCAGGUAAGUUACGCACGUGGACUGUUACAUCAAA
CCGUAGUAGACACUCCUUGCAGUUUCGAGACGGGACAGGAAGAAGCGUGAAA
AAGCUCAACCAGAUAAUGAUAAUAUGCGUAUGGUUGAGUUACUGGCAUGGAA
CAG

No. 59:
> GUT_GENOME151180_45_9178_10326_+(SEQ ID NO: 151) 
MIRGYRIKIFPNEEQKKMIHKSFGCSRFIYNWCIDRISENYNETKKTLSSIE
LQKEIVILKKQIEYSWLNEVSANMLKQVTIDCSNAYKRWFKLIKQNNNIKGKPRYKS
RKSKQNCPTRTDRMTFNGRYVHLEKIGVVKLSKVKIDLDGKLMNARLNFDGIDYW
LSFSVEYKDVQLEEKPKAEPIGIDLGLKTLVYCSNGNIYKKPHTKMIDKKIKHLQRRI
SKIYQPMIDYCKETRTKFSTLKKSNNLTKLEKELRKYQIRKTNILDSNIHRITSDLIKIN
PEKIVIEDLNVKGMMSNHKLARSIQSSKFYEIRRQLMYKCKNNNIELIVADRFYPSSK
TCSSCGKIKDDLKLKDRVYRCPQCGAVIDRDLNAAINLSRIS*
Associated RNA sequence (SEQ ID NO: 193)

UUUAUAUAAAAUAGUGAGACUGCUCUCACGAAUUUAAGCCUAUGG
AGAGUUAAAACCACUUGAGUAGUUGCGACGAAAAAGAAUUCGAAGAAGUAGG
AAAUAUUAGAAUAUACAACUUUGUAUAAGUUUAAUAUAGCAG

No. 60:
> ERR2017626_1022590_12863_8034_9173_+(SEQ ID NO: 152) 
MNDVIRSYKIKLYPTKEQEQLMHKHIGCCRYIWNYMINLQCSNYESGNK
YMSAFTMINLLKPLKNDGEHKWLCEVSNQSLATVCQDVDKAYQRLFKKQAKKPKF
KSKKRSKQAFPVRGTIWFKDNVVTIEKLGKVKFKSDFEFPQGRGNKFTNTRISEING
KWMLSFGMECENQAPELNDYSMGIDLGVKDLAIAECNGQIVFHNINKSKKMRALN
KKLKHLQRAVSRKYEANKQGKKYIKTKNIEREEQKVRNIHNRISNIRNNYIHQCTHA
LISMLPKRVVVEDLNVKGMMKNKYLSRAIQEQCFGEFLRQMQYKCEWNGIEFVMA
DRFYPSSKTCSCCGVVKTNLKLSDRIYRCESCGLVIDRDYNAAINLSRYVA*
Associated RNA sequence (SEQ ID NO: 194)

AUAGAGAGGCUAUAACCUCAAGGUGUCGUUGCACCUUUAAGCUGU
GGAGUGUUAUAUAAACUUGAGUAGGAUUACCCAAAAGAGGACGCUGAGAAGC
AGUAAGUUAGAUUGGCUAACACAACGGAAAAGGAAUGAUUUAUAUGGUUAA
AAUCUCGCAGGAGGAAGCUUUUAAGAUUAGAAAGAAGUUUCCGGGAACGCAU
AUAACGG

No. 61:
> GUT_GENOME142162_18_64466_65659_-(SEQ ID NO: 153) 
MIKAIKVRLYPTKKQEVLMFKSAGISRFSYNWGLAFLNKYYEENNKFLQ
IDKLRKEFTRLRNNNEFSWLKEVSSEIPQQALKDLGESFKKFFKKESLYPRFKKKGK
CEVSFFHLNNKFVVKENYIKLEKIGYVKMKDEGRLPIGNYKKDKIKVLNPRVKFNG
KYWYLSLAMEVEEKTNIKLTNESVGVDLGIKDLAIVSNIDEPFKNINKSKEVKRLNK
KLRKLQRQVSRKYDMLKSGNTFQKGEKLVKTKNIIKVEKQIKLVYKKLSDIRLNHIH
QTTNTIVKTKPCRIVVEDLNVRGLLKNKHLSKSISEQCFNKFINILEYKCKWNKIEFV
KADRFYPSSKMCSCCGTIKKDLKLKDRTYVCSNEKCNLVIDRDKNASINLANYKSN
YKCGYLN*
Associated RNA sequence (SEQ ID NO: 195)

CAUAGAAAAUCAAAUAAAAAGAUUGUGCUAAUAUGUAGACAUUGA
CGAGGUCGAAUUUAAGCCUUUGGACUGCUACAUUAAACUAGAGUAGAAAUUG
AAUUUCAAAAUAGAGCAGGAGGAAAAAGGAAAUAAAACUAUAAAAAAUAUA
AAGUAUUUAUAUAAAUUUAUAGAGUUUAUACGUCAG

No. 62:
> NC_013216_1_421908_422903_+(SEQ ID NO: 154) 
MYAGTCVTSIFKLIKKGISKAYLLCLQMILTNGLTMSILNSKDLSGLRVY
HLKHVRSLLSMRRGLLKDSLKNWVNSIPTIGWMRLKEFGYIPYGVTVKSGTVSYKA
GRYYVSVIVDIQLVGVEKKCSEGIGVDLGVKVLATVSSGIKFKNINKTSKVKKLERR
LKREQRKLSRKYHMRKQRGGTATALRCANIDKQRLKVQKLHQRLTNIQTDYVNKT
VNELVKTKPEYITVENLNVSGMMKNRHLSKAIAQQKFYEFNIKLKSKTQWHNIELR
VVDRFYPSSKICSCCGHIKWDLKLSDRIYICDNCGFVEDRDVNASINLRNAKQYKIS*
Associated RNA sequence (SEQ ID NO: 196)

UUAACAAAGUUCUUGUAUAUGUACCGAUGGCUAGUCGGGAAUUUA
AGACUGUGGAGUGUUACAUCAAAUGCGAGUAGCUUUGUGGCAAAAGCAGACA
UUAUGAAGCAGUAAAACUCUCUUGAUAUAGAUUUUUGUCUAUAUUUAAAGUA
GCAG

No. 63:
> NC_004557_1_WP_035125047.1(SEQ ID NO: 155) 
MIKALNIRLYPTQEQAALMYRHIGCMRFVYNWALARQIDSYKHSGKKL
SIAELGKELTVLKNTEGYEWLYEVSNATLKEGIRDLDKAYGNFFKGRGFPKFKSKK
KSEPKFYSRYEKAYFKDNLVNLEKIGKVKCKVDYDIDLTTIKKFKNPRVKFNGRVW
VLSVGIEIPAKKVKLNDFSLGVDLGIKQLAITNIDNLDTLNINKTNKVKKLNKKLKK
LQRQCSKKYIINKKGGSYQKTKNIGKLELKIKKLHSKLKNIRLNHTHQTTSKMVKA
KPYRVVMEDLKVSNMIKNKHLSKAISEQGFNIFINQMKYKCQKYGIEFIQVSTFYPSS
KTCSHCGTIKKDLKLSDRTYKCDCGFTCDRDKNASYNLANYGLEISI*
Associated RNA sequence (SEQ ID NO: 197)

AAAGAUAUUCUAAAUUGGGUAAUCGUUACAACCCAUUACCUUGCU
AGGUAACAAAGCCUAUGGAGUGUUAUAGCAAACGAAAGUAGCUUAGGCAAAA

UCGGAUAUUAUGAAGUAGGAAAGAAACAAAAAUCUUUAAUUUAUAGAUUUU
UAUAGGUUUUUGGUAACGG

No. 64:
> GUT_GENOME096560_11_244135_245259_-(SEQ ID NO: 156) 
MMKAYKIKLNPNNKQRTLLERSAGVSRWAYNWALEQKKKRYEETGKT
VSQADLRKRLTKLKQTDDYQWLFQFSNNITKQAIKDSDEAFKRFFKGQARYPRFKS
KRKSKWSFYNDNHKVKRSDTHIRLEKIGWIRFAETGYLPIEFKPLSYRVSREGLHWF
VSITVEIPDSSDYSTPVNEPIGIDLGIKTLATVSDGSVYVNINKKHEVRQMEKRLRRL
QRQISRKYKMNKEGSRYRKTCNIIKTEKEIARLQTRLNHIRTNYIHQMTSEIVRTKPS
HIVMEDLNVRGMMKNKHLADHIAKCHFREIRRQLTYKANQSGVSLVVADRWYPSS
RICCECGVMDKTLKLSDRKMKCECGNRIDRDMNASINLSRLAV*
Associated RNA sequence (SEQ ID NO: 198)

UCACGAACAAGACACGGCUAAUAGGUACCGUGAGUUACACGGGAA
UGAACGCCCUCGGAGUGUUACAGCAAACGAGAGUAGUAUCUGAGACAGAUAC
AAAAUCGGACACGGUGAACAGGGAAGAAAACAAAAAAUCUCUUUUACAGGUU
UUUAUAAGUUUUUCGUAACGG

No. 65:
> GUT_GENOME083533_37_11359_12597_-(SEQ ID NO: 157) 
MFRGRKIRLLPTKEQELLFWKSAGTARWAYNYFLAENEAAYRAYLKRG
KTGPRRISADDVRKYINNNLKKTTHQWLKEVSNNVMKMAVKKADYAVKKFLKGL
AGKPKFKTKHRTKPSFYVNYASLARKNGGFHGERLGFIRTAEPLPRLPKGKRYSDP
WISFDGKYWYLGLSYEVSASEAKELSGDTIGIDLGIKTLATCYVSGTKKKMTFQNIN
RTKKVRRLEKKLRREQRKQSRKLVANTARHKSIQREGKTIGRKPIWKRPLHECLNL
KRQRRKIALIHRRLANIRQNHVHQTTATIVKTKPSRVVLENLNVKGLMKNKHLAKA
IAGAKFYEFRRQMIYKCEEHGIQVIIADRFYPSSKTCSYCGYLKKNLKLNDRVYVCP
SCGKAIDRDLNAAINLANYFQSDTACTA*
Associated RNA sequence (SEQ ID NO: 199)

AACUUAGAGAAAUUCUCAGACAGGUACCUAUCGCUACUAGGGAACC
GGCUUUCCUAAAAAGAUUGUCUAAGCCUGCGGAGCGUUAUAUCAAACGUGAG

UAGCCAUCGAGCUUGCUUGAGGCCAAAACGGACGCGAUGAACCAGGAAGAAA
ACAUCGCAAGAGUUUUCGAAGUAUAACUCUGUAUGAUAAUUUAUACAGAUUU
AUCGUAGCGA

No. 66:
> NC_022878_1_WP_023518930.1(SEQ ID NO: 158) 
MSHLKIDKYNKEIEGDKDVSGVQNRNSSNRRTKKIINRTIGVARFVKNFY
IYHNQEVYKSGGKFVSGIDFSKWLNNDYIPNHPDKLWIKAVYAKSTKHAIMETEKA
FRNFFQGKANFPKFKKKHQDVSMYFVKNDKKTIIRCERHRIKVPSLNWVKLNEKG
YIPKHDDKHSIKSGTISQQANRYYLSVIVEQDCTAQVLNDTPTEGVGIDLGLKDFAIL
STGKTYKNINKSGKIRKIEKQLKRQQRKLSRKYESEKKRVNEKQSKRGEATRKNIEK
QVLKVQKLHKRLTDIRRDRVNKIVLELVKTKPAYITIEDLNVKGMMKNRHLSKAVR
EQSFYAFRKKLADKCKVSTIELRLADRFYPSSKLCHECGHKKVALKLSERTYRCENC
GYVADRDYNASMNLKDVKEYNVIA*
Associated RNA sequence (SEQ ID NO: 200)

GCACGCAACUUUGUAUAUGUACCGAUGGCUUAGUCGGGAAUCAAC
GACUGUGGAGAACGAGACAAACCGUAGUAGUGUCGAAGUGACGAGGCGGCGU
UCGGUGAAACAGUAAUUCUACCUACGUGAGUGGUAGAGAAAUCUCGAUAUAG
GUAUUUUUGUCUAUAUUUUGUGUAGCAG

No. 67:
> NZ_CP020815_1_WP_012574395.1(SEQ ID NO: 159) 
MIRTYKVMLLPNNKQKTKLFQCAGVARWAYNFALAQQQENDKQGGKF
LSDGELRKRLTQLKQTKEYSWLNQFSNNITKQAIKDAYQAYKNFFEGRAKFPKFKT
KKRTRPSFYQDTSKIKITDTHVKLEKLTPSKKKNRQKFNWIRLAERGRIPHGAHIKY
VNPRIVFDGLHWFLTVGVEEAEVKHGTYNEGIGIDLGVKSFATVSNGMTFPNIHHM
PQVKKLEKQMKRMQRKISRKYEMNKIQTERGEFRYRKTENIKKLEFLVLKKRRRLK
NTQLNYTHHITTTLVKTKPAYVVMEDLNTSGMLKNRKLSKAIQQQTFHEFKRQMT
YKCAWHGIELIVADRFYPSSKKCSRCGHVKEKLSLSERTYRCEACGIEMDRDLNASI
NLKNYAK
Associated RNA sequence (SEQ ID NO: 201)

AGCACAACAUGUACCCAUCCGUUAGUGGGGAAGUGAAGCCUUCGGA
GCGCCAAGGAAACGAGAGUAGCUUCGGCAAAAUCGGGCGCGAUGAACAAGGA
AGGAAACAUAGACUUUUAUAGGUUUCUUAUAAAGUCUUGUAAGUUUUCUGUA
ACGG

No. 68:
> NZ_CP024701_1_WP_099988772.1(SEQ ID NO: 160) 
MYYKALKIEIKLTEEQKIQVNKTIGTERFIYNEYIKYNQEQYELGNKFVS
ANDFSKYINNIYLPNNPDKKWIKDVSSKSVKQAMIYGEKAFKNFFKGLSSFPVFKKK
GKNELGAYFVKNNKTDFEFYRHKIKIPTLKFVRVKEYGYIPKNANIKSGTITKIADRY
FLSLIIEIEDTVKATNTNSKGLGVDLGIKDTAICSNGKVFKNINKTKKVKKLKKKLKR
EQRKMSRSVEYSKSKKIKLKECKNFNKKKLKVQKLFYRLNCIRDDYNNKIVDEITR
AKLKYIAIEDLKVSNMMKNKHLSKAIQEQNFYAIRTKLINKCKERNIELRVVNTFYP
SSKTCSCCGSIKKNLKLNDRIYKCCNCGLEIDRDYNASINLEKAKIYKVIA*
Associated RNA sequence (SEQ ID NO: 202)

CAAGUUAAAAUAUCUAUAUGUACCGAUGGCUAGUCGGGAAUUUAC
GACUAUGGAGAGUACAAGGAACUAUGAGUAGAUACUAAGUAUUGAAAAUAU
ACUCGUUGAAGUAGUAACAUUCUAGUGUAUUAAAUAUGUUUUAAUACAUUUA
UAGUAGCAG

No. 69:
> GUT_GENOME228548_94_18418_19641_+(SEQ ID NO: 161) 
MEFEEQPIILFKGGLKILKSYKTEIDPTEEQTKKIKKTIGTCRFIYNFYLAK
NKEIYEKSNQSMSAFSFMKWLNNEYLKENPDKLWIKEVSTVSIQKATQNATDAFRR
FFKGQNRFPKFKKKNKQDVKMYFCRQGKNDSIKCERHRIKIPTLGWVKLKEKGYIP
TNPNTHIIRKGTVSYKAGRYYVSVLVEEMQPMKERLNDFGIGIDLGVKEFATLSNGT
IYQNINKTNRVKDLEKKLNRERRKWSRKYKSYLRRKELDENCTKENLLKQQIKMQ
KVFHQLDNARTDYVNKCISEIVKTKPSYITIEDLDVMRMMKNKHLSMEVQKQKFFE
FRTKLADKCREYGIELRVVDRWYPSSKTCHNCGCIKHNLKLSERVYKCPECGFKIDR
DLNAALNLRDSKIYKIIP*
Associated RNA sequence (SEQ ID NO: 203)

CUAAUCUGUAAAUAUGUACCGAUGGUUUGUCGGGAAUUUACGACU
GUGGAGUAUACUAGAACUUGUGAGUAGCGUGUUAUUCGUAACCGCAAAAGCA
UAUACGAAGAAGCAGUAAGAGGUAUCUGUAAAACCCUCAAUUCUCAAUACUU
AUGUAUUUUAGAGAAACAG

No. 70:
> NC_010001_1_WP_012201916.1(SEQ ID NO: 162) 
MKRLQRAYKVELKPTNQQIQKINQCLGVCRWLYNAYLAKNQSLYQDFK
EGKLNKKKAFMSANDFDKYINNDIKVLDDCKWINDCGSKARKKAICNAEFAYKKF
FKGEAQFPRFKKKGKSKVGLYFPKNNKTDFKIERHKINIPTLKWVRLKEFGYIKANA
NVISGTVTEQAGKYYISIVCEVEEVKNSLPKTEPLGLDLGIKDFAIFSNEIVKKNINKT
SRVKKLEKKLKREQVRLSRKYECLKKQNKIRKGETTRQNIRKQIVKVQRLQQRLAN
IRTDYINKCVSEIVGQKPSSITIEDLNVKGMMKNRHLSKAVAGQKFYEFRIKLTNKC
KQNDIELRIVDRWYPSSKLCSCCGNVKKDLKLSDRIYVCKECGLVIDRDYQASINLR
EAKTYMIA*
Associated RNA sequence (SEQ ID NO: 204)

CCACGUUACUGUAAGUAUGUACCCAAGGCUAUUUGGGGAAUUUAC
GCCCUCGGAGUGUUAUAGCAAAUGAGAGUAGUGUAUUAUAACAAUCACCAAA
UCAGACACGUUGAACAGGGAAUAAUCUCGAUAUGGGUAGAUUUGUCCAUAUU
UUGAGUGGCAG

No. 71:
> NC_010003_1_WP_081429131.1(SEQ ID NO: 163) 
MMRLLKSYKTEINPTLEQEQQIKKTLGVCRFIYNFYIAHNKEIYEKERRF
VSGMEFSVWLNNEFIPNSQEFNWIKDVSSKAVKQSIMNGERAFKKFFKGKSKFPRFK
KKKNNDVKAYFPRNNKTDWTIERHRVKIPTLGFVRLKEKGYIPTNAKVGSGSVSFE
AGRYYVSVLVEEDSLTAVEKGIKQDNISFSEGIGIDVGIREFVVASNGIKKKNINKSK
EIKKLEKKLKREQRRLSKKYENKKKGGTATNSAKNIDKQVLKVQKLHQKLKNKRH
DYVNKVVCELVKTKPEYFTIEDLNVKGMMKNRHLSKAIAKQNFHYFKIRLENKCK
HLGIQLRIVSRFYPSSKICSHCGNIKKDLRLSDRVYICEECGSKIDRDLNAAINLKNAK
EYAIAC*
Associated RNA sequence (SEQ ID NO: 205)

AAUUGCUUUGCAUAUGUGUACCGAUGGCCAGUCGGAAUUCACGCC
UGUGGAGUGUCAAACAAACCGUAGUAGAUAUAUCGAGGCGGACACGUUG
AAGCAGGAAACUUCUGGAUAUAGGCAUGUUUGUCUAUGUUUCAGUGGCAG

No. 72:
> GUT_GENOME249453_33_14687_15901_-(SEQ ID NO: 164) 
MVWSLVKYKLEREVRKMIKTIRVMLVPNNKQKAKLFQYANTARFAYN
WALGREKENHENRGKFICEGDLRKEFTQLKKKEEYLWLKKISNNVPKQAIRDACTA
YQNFFNGVSKAPRFKSKKHSIPKFYQDTKKIKFTDTHVKVEGFATSKRHNKQKLNW
IRLAEHCRIPTDCKHFNPRIKYDGLNWYMTVGIEYKDKESIPSNEGIGIDLGIKDLAIC
SDKNKYKNINKIQKVKKLEKRKRRLQRCISRRYEKNKNGECYCKTSNIKRKEKELL
KVSRKLANIRLNYLHQITTEIIKRKPSFICIEDLNVKGIMKNKHLSKAIQQQCFGEFRR
QIEYKSKWNNTKVIIADRFFPSSRLCSCCNKIKKDLNLSDRIYRCECGNVIDRDYQAA
INLKNYGEMKISV*
Associated RNA sequence (SEQ ID NO: 206)

AACACGAAAAAGUUAAUACAGAUGUGUACGAAUACGUUAGUUCGG
AAUUUACGCCUAUGGAGUGUACAAGGAACUGUAAGUAGUAACUAAAUUUAUU
UAGUACAAAAGCAUACACGAUGAAAUAGGAAUGAAACAUAAAAGUUUAUAAC
UUGUUAUAAGUUUUCAGUAACGG

No. 73:
> GUT_GENOME046003_7_13445_14386_-(SEQ ID NO: 165) 
MRYYLIGEFAEKIGKAIQTLRNWHKNNTFKPAYVTAAGTRYYSQEQLN
NFLGLKIKTKIKDKKALIEKVGWIKINEQIPSDIKYSNPRITYDNKYWYISVGIEVNKK
QENLTDISLGIDLAICSNGQVFKNINKTIKVRKLEKRLKQKQRQISRKYEMNKIKKEG
GVRCQFIKTKNIEKLENTTKLIHRKLANIRNNYLHQVTTSIVKNKPYRIVIEDLNVSG
MMKNKHLSDSVRKQCFYKFRQYLTYKTELYGIKLVIADRFYPSSKTCSQYSSIKKDL
KLKDIVYRCPHCGAVIDRDYNAALNLSMYKSNM*
Associated RNA sequence (SEQ ID NO: 207)

GACACGUUGUAUCCAAAUUUAAGCCUUUGGAGAAUCAUAUCAAAC

GAAAGUAGCUAUGGCAAAAUCGGAUUCUAUGAAGAAGGAAGUAAACAAAUUU
AUAUAUUGUUAUAGAUUGUUGGCAACGG

No. 74:
> GUT_GENOME043660_1_131655_132716_-(SEQ ID NO: 166) 
MYNYMLDLQKKRYEAGEKHLSAFSMIKLITPLKQQEGYEWLCRVSTTS
MQRVCQDLANAYENFFSKRAKFPRFKSRKRSRASYPVRETVYFIDGKTQIEKIGKVV
YQTNYDLPQGRGHKFSNPRISNNNGKWILTVGIECESQAPELTDKPMGIDVGVKDL
AIVAFGEDQIVFHNINKSKRVRNLSRKKRKVQRTISRKYRTNGSYSKTKGIEKYERIL
KTIHYKLANIRSNYIHQSTHKLVSMLPKVVCVEDLNVTGMMKNRHLSRAIQEQCLG
EFLRQMEYKCAWNGIELVKVDRFYPSSRTCSCCGEIKEDLKLSDRTYVCPHCGLVID
RDYNAALNLMKYAASQTRAAA*
Associated RNA sequence (SEQ ID NO: 208)

GGGAAUCGUUGCACCCCUGAAUGCUGUGGAGUGUCAUACAAACCCG
AGUAGCUAAGGCCAAAGGGGACACAAUGAAGCAGUGAUUGGAAUUACCAAAC
GCAACGGUACAACGUGCUGGAUGUAGAGGUCAUGGCUAAGAUUCUCUAUCGG
GAAGCUCGUGGCAUCUCCAGCAAGACAGAACAGGCCUGCGUCGGCUGGGUGA
UCUGUAACAG

No. 75:
> NZ_CP014176_1_WP_039636020.1(SEQ ID NO: 167) 
MINLIKALNIRLYPTKEQIILMYKHIGCMRFVYNWALSKQIENYKINNKK
LSVSELAKELTILKNTKGYEWMYEISNATLKESIRDLDKAYKNFFNGSSFPKFKSKK
KSNHVFYTRYDRIYFKEDFVNLEKIGKVKCRVDYDIDLATITKFQNPRIHFNGRVWV
LSVGIETSVEKAKLNDFSLGIDLGITQLAITNIDNLDTKNINKTNSVKKLNKRLKRLQ
RQCSRKYEKNKEGGSYQKTKNIVRLEKKIKKLHSKLKNIRLNHIHQSTSKMVKAKP
FRVVMEDLKVSNMMKNKHLSKAIAEQGFNKFINQMKYKCEKYGIEFIQVPILYPSS
KTCSHCGYIKKDLKLSDRIYKCSCGFSCDRDKNASYNLANYGLV*
Associated RNA sequence (SEQ ID NO: 209)

UCAAUUAAAAGAUUAACUAAAUUGGGGAAUCGUUAUAACCCACUA
CCUUAUUAUUAGGUAGCAAAGUCUAUGGAGUGUUAUAUCAAACGAAAGUAGC

UUAGGCGAAAUCGGACAUUAUGAAGUAGGAAAUAAACAAAGCUAUAAACUUA
UAUAAUCUUUUUAUAGAGUUUUUAGUAUUUUGGUAACGG

No. 76:
> NC_014614_1_WP_013360293.1(SEQ ID NO: 168) 
MIKSIKIRLKPTKEQEILMFKSVGVARFAYNWGLAKWEEMYKKGLKPSK
AKIRKEFNNSVKKSDDFKWLYEVSGQITSQAFTDLEYAYKNFFNKTSDKPKFKTKK
KSKKSFYVRYDALKFKDAKVNIEKIGKVKYSSNYNIPQLDNYVNPRCHFDGKYWY
LTFGFEHSENKAKLDYNLSIGIDLGIKELAYVSCFNKPIKNINKSVKVKKLKKRLIRL
QKQVSRKYEANKSGDRYNKTSNIIKLEKQIKLLHRKLNNIRNNHLHQTTKMIVKLNP
YRIVMEDLNVTGMMKNKHLSKAIAEQKFYEFIRQIKYKCEFNNIEFLQVDRFYPSSK
TCSSCSGIKQDLKLKDRIYHCDKCGLTMDRDKNASINLANYKIV*
Associated RNA sequence (SEQ ID NO: 210)

UUUUGAAAAAUUAUACUAUAUGUAUCCUGCGUUGUGGGAGAAUUU
AAGUCCUUGGAGAGUUACACCAAACUAAAGUAGAGUGAGAAAUCAUUCAAAA
UAGAACUCGAUGAAUAGGAAAUGUUAUUUGGUAACAAAUAAUGUAAGUAAA
AUUUAUAUAUUUAUAAGUUUUAUGCAACGG

No. 77:
> GUT_GENOME243333_13_9207_10436_+(SEQ ID NO: 169) 
MLRGKKIRLLPTPEQEILFWKSAGTSRWAYNYFLARNEEQYKTYLANGR
KGKKSISEGVIRKEINNQLKPITHQWLKEVGSNVMKQAVKEAGRAMKKFLSGAAG
RPKFKTKRRTTPRFYVNYESLTRKNGGFHGEKIGFVRTAEPLPKIPKGCHYSNPWISF
DGKYWYIGFGYEVPDRLNVELTGDSIGIDLGVKELAVCYAFGERKKTVFKNINKTK
EVHRLEKKLRREQRRQSRKFTANIDHYETVQRNEKKTGRRPIWKRPIRECRNIERQR
QRIILLHRRLKNIRENHIHQTTAAIVKTKPSRIVMEDLNVKGMMKNRYLAKEIASEK
FYEFRRQMEYKCEPYGIQLVFADRFYPSSKTCSHCGHIKKDLKMNNRTYICPACGH
VIDRDLNAAINLAKYFKSEIA*
Associated RNA sequence (SEQ ID NO: 211)

CAUUUCGAGAAGCACUUAGAUAUGUACCUAUCGCUACUGGGGAAU
UUAAGCCUGUGGAGCGUUAUAUCAAACGAAAGUAGCUGCCUGCUUCGGCAGG

GCAAAAUCGAACGCAAUGAAACAGGAAGGAUGUUUGUGAAAACAUUCAAAGU
GUAAAUUUGUAUAGUAUAAAAUAGAUAUUUACAGAUUUAUCGUAGCGG

No. 78:
> GUT_GENOME228909_131_318_1547_-(SEQ ID NO: 170) 
MIVGKKIRLHPTSEQETLFWKSAGTARWAYNYFLIRQEEVYQEYLVNGQ
KGRRGISEGTVRKEINRVLKPTTHAWLKDIGSNVMKQAVKTADKAWKKYLKGLA
WKPKFKSKKMTKPNFYVNYESLSRKNGGFHGEKIGFVRTAEPLPKLPHKKHYLNP
WISFDGKYWYLGFSYEVEAHRESLTGDHIGIDLGVKSLAVLYSVDTGQSKVIKNINK
SRKVLRLEKKLKRAERKRTRQLKSNVLRYEPIQRDGKRPGRKPVWKRPVPECKNIE
KQRRKIKLIYRKLKNIRVNHLHQATTAIVKTKPSRVVLETLNIQWMMKNKHLAKAI
SKAKLYEFRRQIEYKCQKCGIEVVFADKFFPSSKQCSSCGEIHHELTLSDRMFSCSCG
LHIDRDLNAAINLARYTPSKIAHN*
Associated RNA sequence (SEQ ID NO: 212)

GAGCAGUUUCGAUAUGUACCUAUCGCUGCUGGGGAAUUUAAGCCU
GCAGAGCAUUAUAUCAAACGAGAGUAGCUUGCAAUGCGUUGUGGCAAAAUCG
GAUGCGAUGAAGCAGGAAGAGUGCUCGUGAGAGCAGUCAACGUAUUGAUUUA
UGAUAUAUAUCGGUAAAUCAAUCGCAGCGG

No. 79:
> ref_RGS74140_1_44001_45231_+QRVW01000004_1(SEQ ID NO: 171) 
>MLRGKKIRLLPTPKQEVLFWKSAGTARWAYNYFLGENQAVYKAYLEHGKVGPK
RISEGAIRKHINNELKKTTHQWLKEVGSNVMKMAVKEADRALKRFLKGIAGKPRFK
TKHKTKPSFYVNYESLVRKNGGFHGEKIGFVRTRETLPKLPKGNHYCNPWISFDGK
YWYLSFSYEVPEQETEKLTGDSMGIDLGIKVLATCYASGERKATVFKSVNKTKEVR
RLEKKLRREQRKQSRKILANTARYETVQRKGKKPGRKPIWKRPLRECQNIEKGRRKI
ALIHRRLANIRQNHIHQTTAAIVKTKPSRIVMEDLNVKGMMKNKHLAKGIAGEKFY
EFRRQMTYKCENNGIQLVFADRFYPSSKTCSCCGHVKENLKLNDRKYVCQACGTTI
DRDLNAAINLANYFQSETA
Associated RNA sequence (SEQ ID NO: 213)

AAUACAGAGAAGCACUCAGAUAUGUACCUAUCGCUACUGGGAAU

UCAAGCCUGUGGAGCGUUACAUCAAACGCGAGUAGCCAUCGAGCUUGCUCGG
GGGCAAAAACGGACGCAAUGAAACAGGAAGAAAAUAUCGUGAGAAUUUUCAU
CGUAUAAAUUUGUGCGAAUUAUAUUAUAUUUAUACAGAUUUAUCGUAGCGG

No. 80:
> GUT_GENOME029159_118_4969_5874_-(SEQ ID NO: 172) 
MPAQPIKTFSRVYNGTQGLRRKRNQCLNSIKIQFRDTHVKFEGFSFSRKA
NKQKLNWVKLAEHGRIPTDAKYRNPRVSFDGLNWWISVCVEFPDCRDRSNHDGIGI
DLGIKNLAICSDGNTYKNINKSQTVKKLEKCKRRLQRSVSRKYEHNKKGGSYCKTN
NIVKKEKLLLKVNHRLANIRKDYLNQTTSEIVNRKPRFICIEDLNVSGMIKNRHLSKA
VQNQGFFEFRKQLEYKCNDRGIQLIVADRFYPSSKRCSCCGKIKKDLRLSDRTYKCE
CGNVIDRDFQASLNLKAYGEQFAS*
Associated RNA sequence (SEQ ID NO: 214)

CACUUAAAUGUUAAUGCAAAUAUGUACGGAUGCGUUAAUUCGGAA
UUUACGCCUAUGGAGAGUACAAGAACUUGUGAGUAGAUAGGUAUUUAUGCCA
UCAAAAGCAUACUCGUUGAAGUAGGAAUGAAACAUAAAAGUUUAUAACUUUU
UAUAAGUUUUCAGUAACGG

No. 81:
> NC_010371_1_WP_012289953.1(SEQ ID NO: 173) 
MLITYKTEIKPNPEQVIKIKQTMGVCRFIYNFYIVENQKAYKNNMPFITAF
NFSKWLNNVFIPNNLNYSWIRDVSSKSVKQSIVCAERAFKRFFKKESKFPRFKKKNK
SNVKMYFVRNNKGDCLCERHRIKIPTLGWIKIKEKGYIPTSKDDYVVKSGAISYKAG
RYYISCLVDIEEPPKQILKPFGLGIDLGLEKFAVISNGNVYKNINKSKKIIKLEKKLRH
EQKCLSRKYEQLKNNKKGESTHKNILKQIAKVQKTHLKLSNIRTDYINKIINELVKT
KPAYIVIENLNISGMIKNRYLSKSISNQRFYEFRQKLFNKCNFHGIELRIADRFYPSSK
TCHSCGSIKKDLKLSDRIYKCPTCGEIIDRDLNASLNLRDTTSYKIA*
Associated RNA sequence (SEQ ID NO: 215)

UAAAAAAAGCUAUAUAAGAUAUGUACCGAUGGCUAGUCGGGAAUU
GACGACUGUGGAGUGUACAAGAACUUGUUAGUAGCAAAAUCUUUGCAAAAGC
AUACACAAUGAAGCAGUAAUUUAUCUCGAUGUAGACUUAUUUGUCUAUGUUU

UGAGUAGCAG

No. 82:
> NZ_CP009687_1_WP_044824965.1(SEQ ID NO: 174) 
MKKMIKGIKVMLIPNNKQNTKLFQYAGASRFAYNWGLAKQQENYKNS
GKFINDGELRKEFTQLKKQEGYEWLNNISNNVTKQAIKDACNAYKRFFKGESKFPR
FKSRKKSAPKFYQDVEKIQFNGTHVKFEGFSTSKKKNKQKLNWVRLAEYNRIPFGE
NEKYINPRVSFDGLNWWVSVGIEHEDEVKQPTNEGIGIDLGIKDLAICSDIDKPYKNI
NKTSKVKKIKKKLRRLQRKISKKYEMNKEGSRYKKTCNIIKLENKLKKVHHQLKSIR
HNYIHQTTCEIVNREPMFIVLEDLAVSNMMKNKHLAKAIGEQCFYEFYRQIEYKAL
WNNVKFIIADRFYPSSKMCSVCGAIDKDLKLSDRTYICECGNKIDRDKNASVNLKNF
GELAI*
Associated RNA sequence (SEQ ID NO: 216)

UCACUUAAAAGAUAUUGCUAAUAUGUACUCAUACGUUAAUGAGGA
AUUUAUGCCUUUGGAGUGUUAUAUCAAACGAAAGUAGCAUAGGCAAAAUCGG
ACACGAUGAACAAGGAAAGAAACACAAAAGUUUAUUUUAUAACUUUUUAUAA
GUUUUCUGUAACGG

No. 83:
> GUT_GENOME100288_478_819_1994_-(SEQ ID NO: 175) 
MLKSYKTEIDPTPEQKQHISRTIGVCRYVYNFYLAHNQEIYEVEKRFVSG
MDFSKWLNNEFIPNNPDFHWIKEVSSKSVKQSIMDAERAFKNFFKGKSRFPRFKKK
ARSDVKMYFVKTDAKVIIKCERHRIKIPTLGWVRLKEKGYIPTNPKTHIIKSGAVSCK
AERYYVSVLVEEQEPQKPVLNDFGIGIDLGLKDFAVCSNGKTYKNINKSSQIRKLEK
KLKREQRSLSRKYESYKKLNKNMKGVATRQNIQKQKLKVQELHQRLDNIRTDHIK
KVVSELAKTKPMWITIEDLNISGMMKNRHLSKAIAQQKLFEFRTKLLAKCNSYGIEL
RIVDRFYPSSKTCHNCGSVKSDLKLSDRTYHCCECGYAEDRDYNASLNLRDCRTYK
IA*
Associated RNA sequence (SEQ ID NO: 217)

ACAAGCUAAUGUAGGUAUGUACCGUAGGCUAUACGGGAAUUUACG
CCUGUGGACUAUACAAGAACUUGUGAGUAGUCUUAGGACAAAAGCAUAUAGG

UUGAAGCAGGAAUUUCUCAAUAUGGGUGUAUUUGUCCAUAUUUGAGUAGC
AG

No. 84:
> GUT_GENOME089678_61_13091_14269_-(SEQ ID NO: 176) 
MLITKKIRLKPTTEQEILFRKSAGVSRWAYNFFLGENKRIYREYIDNGKV
GKKSISEGDVRKYINNVLKPTTHSWLKEVGSNVMKQGVKDANLALQRYFKDLSGK
PKFKSKHKDRPSFYVNYESLSRKQGGFQGEKIGFVKTSESLPKLNKNEKYSNPRITFD
GKYWYLSVGYEAKENTVELTDESLGIDLGVKDLAICSNGKVYKNINKTQRVKNLE
KKLKREQRKLSRKIENNIQSYKSNRSPVYKRPLKECQNVQKQNRLIRNIHRKLANIR
QNYLHQTTTEIVKTKPSRIVMESLNVSEMMKNKHLSKAIQQQKFYEFKRQIEYKSKL
NGIEFIQVDKFYPSSKTCSCCGNIKKDLKLSDRMYKCDVCGLVIDRDYNASINLANY
EVI*
Associated RNA sequence (SEQ ID NO: 218)

AUUCACUUAAAAGAAUUAUAUAACUAUGUACCUAUCGCUAUUAGG
GAAUUUACGUCUAUGGAGUGUCAUAACAAAUGAAAGUAGCUACGGCAAAAUC
AGACACGUUGAAGUAGAAAGUCUUAAUCGUGAGAUAAGACACAGUAUAGAUU
UAUAUAGAUUAGUUGUAAUUUAUAUAUAUUUAUCAUAGCGG

No. 85:
> GUT_GENOME190643_17_8068_9183_-(SEQ ID NO: 177) 
MILAKKVRLCPSELQEEKLWQSVGTARFIYNWALNRQEENYKGGGKFIP
DSVLRKEITQLKKTDLSWLNEVSNNVAKQAVKDACDSYKRFFKGLSGKPRFKSRKR
SKKSFYHDNVKLKVKDNKLVKIEKIGWIKTNEQLSIGAKYSNPRISYDNKYWYLSV
GIEQEEIQEELTDASLGIDLGLKNLAICSDGTVYKNINKTCTVKKIEKKLKRLQRQVS
RKYEKNKKGKEYVKTKNIIKLEKQIQQTHRRLANIRNNYLHQTTTSIVKTKPYRVVI
EDLAVSNMMKNKHLSDAIRKQGFYEFRRQLEYKCNFRGIKLVVADRFYPSSKICSQ
CGEIKKDLKLSDRVYKCNCGLVIDRDLNASINLSKYKLA*
Associated RNA sequence (SEQ ID NO: 219)

UAUCAUUAAUAAGAUAAUGCUAAUAUGUAGGAUGCGUUGUAUCCG
AAUUUACGCCCUUGGAGAGUUAUAUCAAACGAAAGUAGGUUGUUAUUUAUAA

UUUCCAAAAUCGAAUUCGUAGAACAGGGAAUUAAACAAGAUUUAUAGAUAUU
UAUAGAUUUUUGGCAACGG

No. 86:
> ERR1539620_45264_57524_39821_41020_-(SEQ ID NO: 178) 
MNEEDKGDQERMLKSYKTEINPTMEQIHKINKTIGTCRFVYNFYLARNK
EVYEKEKKFVSGMEFSKWLNNVYLKENPEYVWIKEVSTKSVKQSIMNSERSFKRFF
KGQSGFPKFKKKGKSDVKMYFVKTNSQTIITCERHRIKISTLGWVRLKEKGYIPWDS
NTYIIKSGTVSCRAGRYYVSVLVEQPERERLQLQDFGFGIDLGVKNFAVISNGLVKK
NINKTNKVKKLEKQLKREQRCLSRKYESLKKNNKKGESSRENIQKQILKVKRLYCRI
DNIRTDYINKTIAEIVKTKPSYIAVEDLNVSGMMKNRHLSKAVASQKFYEFRVKLEA
KCKEYGIELRIVDRWYPSSKLCHECGCIRKDLKLSDREYVCECGYHADRDYNASLN
LRDATSYKVA*
Associated RNA sequence (SEQ ID NO: 220)

ACAAGCAUUUGCAGGUAUGUACCGAUGGCUAGUCGGGAAUUGACG
ACUGUGGAGUGUACAAGAACUUGUGAGUAGUAUCUGUAUUUACUCAGGGCGA
AAGCAUAUACAGAGAAGCAGUAAGAAAUGUUCGCAAGAACUUUCAAUAUCUC
GAUAUGGAUAUCUUUGUUCAUAUUUUGAGUAGCAG

No. 87:
> NZ_CP013243_1_4129900_4131015_+WP_072587064.1(SEQ ID NO: 179) 
MILAKKIRIIPNMEQEQQLWKSVGTARFIYNWTLARQEGNYKNGGKFIK
DGDLRKEITIMKQTEEYKWLKEVSNNVAKQAVKDGCNAYKRFFKGFADKPRFKSR
KKSRPSFYNDNLKLKVKPKMALIEKVGWIKTAEQVPMNIKYTNPRVSFDGKYWYL
SVGVEKENSKVELTGESIGIDVGIKDLAICSNGMTFKNINKTRLVKKLEKRLRRLQR
KISKKYELNKEGRKFVKTSNIIKLEKQIRLLHRRLSNIRNNYLHQATTKIVKNKPSRV
VMETLNIKGMMKNRHLSKAIAQQGLYEFKRQLQYKCEFYRIEFVEADKWYPSSKIC
SECGHKKTKLSLSERTYICEECGVVIDRDFNASINLSRYSA*
Associated RNA sequence (SEQ ID NO: 221)

CUACUUAAACAGUUAUGUUUGAGGUGUAGGAUUCGUUGUAUCCGA
AGUUAAGCCCUCGGAGUGCUAUACAAACCAAAGUAGCUACGGCAAAAUGGAG

UACGUUGAACAGGGAAUUAAACAAGCUUUAUAGAGUUUUAUAAGGUUUUGGC
AACGG

No. 88:
> CP029458_1_2205965_2207104_-AYF55140.1(SEQ ID NO: 180) 
MKMILLKKVRLYPTGIQEQKLWQSVGTARFIYNWTLNKQQENYKNGGK
FIKDSDLRKEITLMKKTDEYKWLSEVSNNVAKQAVKDCCNAYKNFFKGLSDKPRF
KSRKKSKPSFYNDNVKLKVKTKLVNIEKVGWIKTKEQIPINVKYTNPRISFDGKYWY
ITVGVEQEQSTIELTNKSIGIDVGIKDLAICSNGMTFKNINKSKEVKRLKKVLKRKQR
KVSRKYDMNKKIEGGENRCQYKKTNNIIKLEKEIRLLHRRLRNIRNNHIHQATNKIV
KTKPSRVVMETLNIKGMLKNKHLSKAISEQCLYDFKTKMQYKCKLYGIEFIEADKW
YPSSKTCSCCGAIKKDLKLSDRVYKCKCGLVIDRDLNASINLSRYKLA
Associated RNA sequence (SEQ ID NO: 222)

UAUCACUUAAAAAGAUAAUGCUAAUAUGUACCAUUCGUUGUAUGG
GAAUUUAAGCCCUUGGACUGUUAUACAAACCAAAGUAGCCUCGGCAAAAUGG
AACAGGUGGAACAGGGAAUUAAACAAGGUUUAUAGAUAUUUAUAGAUUUUU
GGCAACGG

No. 89:
> CP013700_1_58380_59501_-AUM93701.1(SEQ ID NO: 181) 
MILGKKVRLYPTKDHEQKLWQSVGTARFIYNWTLARQEESYKNGGKFIS
DNDLRKELTILKKTELNWLNEVSNNVAKQAVKDGCEAYKKFFKGLADRPRFKSRR
KSKPSFYNDTSKLKVKENSVLIEKVGWINIKKNSIPMNCKYSNPRISFDGKYWFIAV
GIEKEKPIVELTTESIGIDVGVKDLAICSNGMTFKNINKARVVKQLEKRLRRLQRRVS
RKYLKNKEGSKFVKTSNIIKIEKQIKLLHRKLANIRSNYNHQATNKIVKTKPSRVVM
ETLNIKGMMKNKYLSKAIAKQCLYEFKKQMQYKCEFNGIKFVEADKWYPSSKTCS
ECGHIKPKLSLSERTYICEECGCVIDRDYNASINLSRYELVG
Associated RNA sequence (SEQ ID NO: 223)

UCACUUAAAAGAUACUACUAAUAUGUACCAUGUGUUGUAUGGGAA
UUUAAGCCCUCGGAGUGUUAUAGCAAACGAAAGUAGCUUUAUAGCAAAAUCG
GACACGUUGAACAGGGAAUUAAACAAGCUUUAUAGAGUUCUAUAAUGUUUUG

GCAACGG

No. 90:
> SRR5892202_233507_16022_11195_12361_+(SEQ ID NO: 182) 
MIILLKSFKTEINPTAEQIIKINKTIGVCRFIYNFYIAHNKELYENGEKFISG
RRFNVWLNNEYLPNNPDKMWIKDVYTKAVAKSMDNACVAFTRFFMCKSKFPRFK
KKNKSDVKMYFVKNNKTDCLSERYRIKVPTLGWVRLKEKGYIPTSKDGFIIRSGTIS
HKAGRYYISVLVDIEKQDTEMSNNFGIGIDLGLKSLAVCSDGSTYCNINKTYNIRKV
EKILKREQRKLSRKVISIKKGESTRKNFVKQRLKVQKLYQRLDNIKTDYINKTISKVV
KTKPSYIVIEDLNVSGMMKNRHLSKAVAQQKFFEFKTKLISKCKENNIELRVVDRFY
PSSKLCHNCDNVKKDLKLSDRIYKCSECGYVEDRDINASLNLRDAKTYTIIQ*
Associated RNA sequence (SEQ ID NO: 224)

AUAUAGUGUAUAAGUAUGUACCAAUGGCUAGUUGGGAAUUUACGA
CUGUGGAGUAUAGAAGAACUUGUGAGUAAUAUAUUCAACUUUGUUGAAUUA
UGAAAGCAUAUACGUUGAAGCAGUAAGUAAAAAUCGUGAGGUUUUACAAUUC
UCAUUAUAGACAUUAGGUCUAUAAUUUGAGUAGCAG

No. 91:
> NC_014171_1_3506713_3507831_-WP_000978855.1(SEQ ID NO: 183) 
MMLAKKVRIKPNKGQELQLWKSVGTARWAYNWTLAKQEENYKNGGK
FISDVFLRKELTVLKQTEEYAWLYDVSNNIAKQAIKDACEAYKRFFKGLADKPRFKS
RRKAKLSFYNDNVKLKVKEKQVLIEKVGWVKTSEKLPVGVKYSNPRVSFDGKYW
YLSVGIEQEFTKEKLTAVSLGIDIGIKELAYCSDGQRKNNINKTKMVRQVEKRLRRL
QRQVSCKYEMNKEGNRFVKTSNIIKIEKEIRQLHRRLSNIRKNHLHQSTSEIVKTKPF
RIVMETLHMKGLMKNRYLAKAIAKQCLYEFKRQIQYKCEKYGIEFVEADTWYPSSK
MCSSCGSINKELKLSDRVYKCTCGHEMDRDLNAAINLSRYELAS*
Associated RNA sequence (SEQ ID NO: 225)

CAUCUAAAAAAGUUACGGCUAAUAUGUAGGAUUCGUUGUAUCCGA
AUUUACGCCCUCGGAGUGUUACAUCAAACGAAAGUAGCUUCUGUGAAAUCGG
ACACGUAGAACAGGGAAAUAAACAAAAUUUAUAGAUUUUUAUAAGUUUAUG
GCAACGG

No. 92:
> NC_017209_1_13469_14587_-WP_000604588.1(SEQ ID NO: 184) 
MILVKKVRLKPNREQEMQLWKSTGIARWAYNWALSRQEENYKQGGKFI
SDGTLRKELTQLKQTEEYAWLYDVSNNVTKQAVKDLCEAYKRFFKGVVDKPRFKS
RRHSKPSFYNDNVKLKVMKNMVLIGKVGWVKTSECIPVVNKYSNPRISFDGKYWY
LAVGIEQEIIHQELTNASIGIDVGIKELAFCSNGMKFKNINKTKVVKQVEKRLRRLQR
QVSRKYEINKEGSCFVKTSNIIKLEKKIQHLHRRLTNIRDNHIHQVTCAIVKTKPCRV
VMETLNIKGMMKNKHLSKAIAQQKLYDFKMKMKYKCEKYGIEFVEADKWFPSSK
QCSNCGEVNKHLTLSDRTYHCKCGHHIDRDLNASINLSRYQLVN
Associated RNA sequence (SEQ ID NO: 226)

CACUAAAACGUUAUUGCUAAUAUGUAGGAUUCGUUGUAUCCGAAU
UUACGCCCUUGGAGUGUUAUGUCAAACGAAAGUAGCUUCGGCAAAAUCGGAC
ACGUAGAGUAGGGAAGUAAACAGACUUUAUAAAGUUUUAUAAGGUUUUGGC
AACGG

No. 93:
> NZ_CP012098_1_2114035_2115228_+WP_077326813.1(SEQ ID NO: 185) 
MMVVKKMIKTIRVMLIPNNKQNTKLFRYANTARFAYNWALGREKENY
KNGGKFLSDSDLRKEFTQLKKTEEYSWLKEVSNNVTKQAIKDACHAYKRFFKGCS
KFPKFKSRKFSIPSFYQDNVKIQFSDTHVKIEGFAASKKKNKQKINWIRLAEKNRIPT
DCNYSNPPIRYDGINWWITVGIEYEDSVTVPSNDGIGIDLGIKDLAICSDGNKYKNIN
KTKKVKKLEKQKRRLQRSISRSYENNKQGKEYCKTKNVIKKEKLLLILNHRLTNIRH
DHLHHITSEIVKREPSFICIEDLNVKGMMKNRHLSKAVQQQGFYEFRRQMEYKSKW
NNIQAIIADQFFPSSKLCSCCGKIKKDLKLSERIYKCECGNVIDRDLQAALNLKKYGE
DVLKRSVA
Associated RNA sequence (SEQ ID NO: 227)

UAUAUUCAAGUGAUUACAGAUAUGUACUGAUACGUUAGUCAGGAA
UUAACGCCUAUGGAGAGUACAGGAACUUGUGAGUAGUACAUGAAUUGAUUCG
UUACAAAAGCAUACUCGUUGAAGUAGGAAUGAAACAUAAAGGUUUAUAACUU
UUUAUAAGUUUUCGGUAACGG

No. 94:
>IS607_orfB_GUT_GENOME170581_2_14431_15588_-(SEQ ID NO: 186) 
MYKSFKTEINPTPTQKAKIDKTIGTCRFVYNFYLAHNIELFNAGEDLMTG
KMFSVWLNNEYLPNNPEYNWIKDTYSKAIKKSIEDGFTSFKRFFKHESDFPRFKKKG
KSNVKMYFVKNNKTDCKCERHRIKIPTLGWIRIKEKGYIPTEKQGWNIKSGTVSYKA
GRYYVSVLAEIPDIEYEKTSNEGVGIDLGIKDFSVISNGNVHENINKSHKIRKLEKKL
RREQRCFSRKYENLKKEGEPTQKKNIEKQKLKVQKIYHKMDNIRTDYINKTIAEIVK
TKPSYITIEDLNVKGMMKNKHLSKAIASQKFCEFRRKLKIKCDNQNIELRIVDKFYPS
SKLCHHCDSIKKDLKLSDRVYRCKCGYIEDRDRNAALNLKDAQSYVVA*
Associated RNA sequence (SEQ ID NO: 228)

AAGAUAGAAAAGAAUUUUAAGCAUGUACCAAAGGCUAAUUUGGGA
AUUAACGACUGCGGAGUGUACAAGAACUUGUUAUUAAUGCCUUAUUAAAGAC
AUGAAAGCAUACACGUUGAAACAGUAAGAAGUAUCUGUGAAGACUUCAAUUU
CUCAAUAUGUUAAGUAUAUUAAUAUGAUUGAGUGGCAG

No. 95:
> NZ_CP011114_1_2448296_2449408_-WP_025697990.1(SEQ ID NO: 187) 
MILTKKVRIRPALEQEQQLWRSVGTARWVYNWTLSRQEENHKHGGKFL
SDGDLRKELTQLKHTEEYAWLCNVSNNVAKQAVKDACDAYKKMFKGLAKKPKFK
SRKKSRPSFYNDTAKLKVKDQVVLLEKVGWMKTVEQLPIGVSYSNPRVTFDGKYW
YLSVGINQEQVQQKLTDEVVGIDVGVKELAVCSNGMVFKNINKSAAVRKTEKRLR
RLQRKVSRKYEMNKEGSRFVKTSNIVKVEKSIRLLHRRLTNIRTNHIHQATSAVAKT
KPRAVVMETLNVSGMMKNRHLSQAIANQKLNEFIRQMKYKCEKRGATFMQADRW
FPSSKCCSGCGQVKTHLKLSDRIYECDCGLKIDRDLNAAINLAQLAV
Associated RNA sequence (SEQ ID NO: 229)

CAUCUUAAAGCUUACGCCAAUGUGUAGGAUUCGUUGCAUCCGAAU
UUACGCCUGUAGAGUGUUAUACCAAACGUAAGUAGCUUCGGCAAAAGCGGGC
ACGUUGAAGCAGGAAGCAAACAAGAGUUGUGUAGACUUGUACAAUCUCUUGG
CAACGG

No. 96:
> CP035280_1_2081523_2082695_-QAT40560.1(SEQ ID NO: 188) 
MKKLKKAYKTEIKPTKEQIIKIRRTIGVSRFIYNFYIAHNKEVYEKEKKFV
SGMDFSRWLNNEYIPNNQDKVWIKKVSSKAVKQAIMNGEKAFKKFFKGEAGFPKF
KKKKNQDIKAYFPKNNETDWTIERHRIKIPTLGWIGLKEYSYIPANGRITSGTVSQKA
DRYYVSVLVDEDIKINNKRCSEGIGVDLGLKDFAICSNGITKKNINKTKTVKKAEKK
LKREQRKLSRKYESLKLRNKKEKGEATRQNIQKQIVKVQKLHQRLTNIRTDYINKT
VSELIKQKPRFITIEDLNVRGMMKNRHLAKAVAQQKFYEFRIKLISKSKQDGIEVRIV
DRFYPSSKTCSCCGRIKKDLKLSDRVYKCDNCNTSIDRDLNASINLANAKEYKIA
[0263] Associated RNA sequence (SEQ ID NO: 230)

UUAAACAAGCGCUUAUAUGUGUACCGAAGGCUAUUUCGGGAAUUU
ACGCCUGUGGAGUGCUAUACAAACUGUAGUAGCUUAGGCAAGGCAGAGUACA
AUGAAACAGGAAUCUUCUCAGUAUGGAUACAUUUGACCAUAUUUUGAGUAGC
AG

No. 97:
> NC_017078_1_59860_61161_-WP_014431089.1(SEQ ID NO: 189) 
MIRSVRIRLLPNNKQRTKLFQFAGASRFAYNWALDKQMENFREGRKLQS
DYDLRKEFTVLRNSEEYLWLLGISNNVTKQAIKDLCIAYKNFFHKQRQKGYVKYSP
KKLAHFARIARTPTIYDMNGHPKFRSKKNGDFRFYQDNVKIQFTATHVKLENIAGSR
KKNRQRLNWIRLAEKGRIPTKGRYTQPRITFDGENWWLSVGIRTSYTLKPMRGLNF
SRKQPGRFCTEGVGIDLGIKDLAIISDSTVVKNINKSHTIRNIKKKRRRLQRQVSRKY
QMNKKGERYCKTSNLIKSEKRLLRINHRLADIRANHVHQATRIIINRKPRFICLEDLN
VQGMMKNKHLSEKVQEQNFYEFRRQIEYKAHWARIPVVIADRWFPSSKTCVECGYI
NKDLKLSDRTYVCPVCGNVIDRDFQASLNLKRYGETELSKSAS
Associated RNA sequence (SEQ ID NO: 231)

CACCGACAAGUCAAUGCAGAUAUGUACCGAUUCGUUAGUCGGGAA
UUUAAGCCUCUGGAGCGUUAUAUCAAACGUGAGUAGUCUGUUCUUCGGGACG
GCAAAAGCGGACGCAGUGAAUGAGGAAUGGAACGUAAAAGUUAACUUUCUAC
GGAAGGUUUAUGACUUUUUAUAAGUUUUCAGUAACGG

No. 98:
>IS607_orfB_GUT_GENOME088555_122_512_1693_+(SEQ ID NO: 190) 
MMIKTIRVMLVPNNKQKTKLFQYANTARFAYNWALGREQENYKNGGK
FISDGDLRKEFTQLKKKEEYAWLNYVSNNVTKQAIKDACEAYRDFFKGYTKFPRFK
SKKHSVPKFYQDNVKIQFTDTHVKVEGFATSRKKNKQKLNWIRLAEHGRIPTEKVK
YMNPRIKYDGLNWYITVGIEYEDSTTLSTNEGIGIDLGIKDLTICSDGNTYKNINKTH
KIKKLEKRKRRLQRSISRRYEKNKKGGSYCKTSNIIKSEKELLKLNHRLTNIRQNYLH
QTTTEIVKTRPSFIVLEDLNVKGMMKNKHLSKSVQQQCFGEFRRQIEYKSAWNNIQ
VIIADRFFPSSKLCSYCGNIKKDLKLSDRIYRCECGNVIDRDYQAALNLKKYGEMAL
EQSVA*
Associated RNA sequence (SEQ ID NO: 232)

CACAUUCAAGUUAAUACAGAUAUGUACGGAUUCGUUAGUUCGGAA
UUUAAGCCUAUGAAGUGUACAAGAACUUGUGAGUAGUAUCUGGGUUUAUUCA
GUACAAAAGCAUACACGACGAAGUAGGAAUGAAACAUAAAAGUUUAUAACUU
UUUAUAAGUUUUCAGUAACGG

No. 99:
> CP030117_1_439976_441088_+AWX54000.1(SEQ ID NO: 191) 
MILAKKVRLKPTPEQERQLWKSVGTARWAYNWTLAKQEENYRNGGKF
LLDGDLRKELTQLKQSEEYSWLYEVSNNITKQAVKDACDAYKKFFKKLAEKPTFKS
RRRSKPSFYNDNVKLKVKQNHVLIEKVGWVTTSEQLPTDVNYTMPRISFDGKYWY
LSVGVEQEFIQPELSGESLGIDVGVKELAVCSNGMTFKNINKTKVMKKAEKRLRRL
QRKVSRKYEMNKEGSRFVKTCNIVKVEKHIRLLHRRLTNIRTNHSHQATSAIVKTKP
STVVMETLNIKGMMRNRHLSKAIAQQKLYEFKRQLQYKCEKYGIRFMEADKWYPS
SKTCSDCGEVKKDLKISDRIFKCVCGLVLDRDVNASINLAKLAS
Associated RNA sequence (SEQ ID NO: 233)

CCCCAAUAAGUUACCGCUAAUCUGUAGGAUUCGUUGUAUCCGAAUU
UACGCCUGUGGAGUGUCCGAUCAAAUGCGAGUAGCUUCGGCCAAAGCAGACA
CAGAGAAGCAGGAAGCAAACAAAAGAUGUGUAGACUUUUACAAUCUUUUGGC
AACGG

### Examples

The present disclosure is further described by the following examples, which are not to be construed as limiting the scope of the present disclosure.

### Example 1: Discovery and acquisition of the gene sequences of RAD (IS607 TnpB) systems

Non-redundant, high-quality genome sequences of 1,520 gut strains isolated and cultured from healthy human fecal samples were downloaded from NCBI (project number PRJNA482748). 19,165 complete bacterial genome sequences were downloaded from the NCBI FTP site (ftp://ftp.ncbi.nih.gov/genomes/Bacteria/). 26,097 bacterial genome sequences were downloaded from the IMG database (https://img.jgi.doe.gov/). 12,715 microbial genome sequences assembled from marine metagenomes were collected from the literature (Maria G Pachiadaki et al., Cell. 2019 Dec 12;179(7):1623-1635.e11. doi: 10.1016/j.cell. 2019.11.017; Charting the Complexity of the Marine Microbiome through Single-Cell Genomics). 204,938 microbial genome sequences isolated from the human gastrointestinal tract were downloaded from the MGnify database. 141,115 unassembled metagenome samples covering multiple species (plants, insects, birds, etc.) were downloaded from MGnify database. 96,709 unassembled metagenome samples from multiple tissue sites of humans were downloaded from HumanMetagenomeDB. Further assembling was performed to obtain genome sequences.

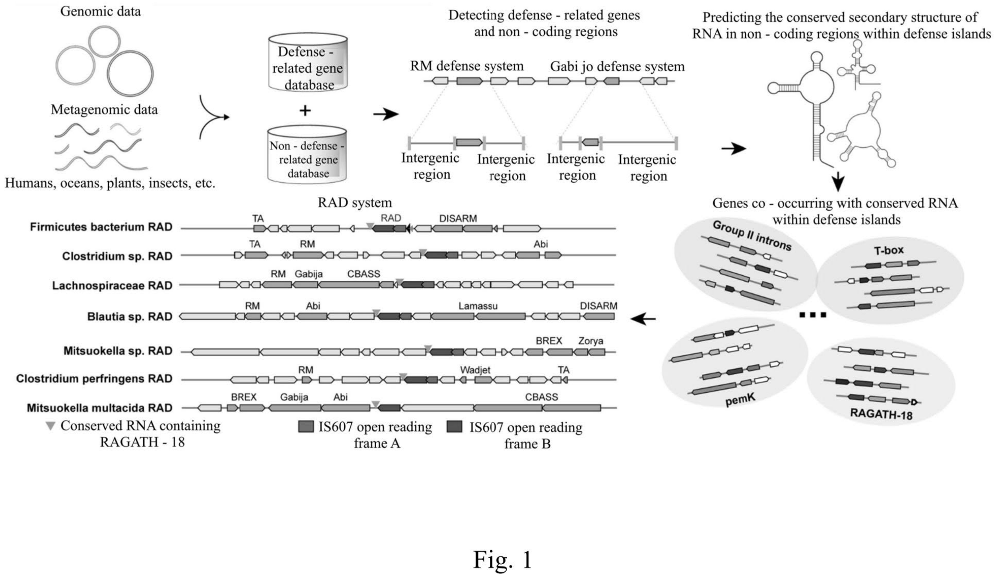

The inventors analyzed the intergenic region (IGR) proximal to defense-related genes one by one by enumeration, and predicted the conserved secondary structure by Rfam (Fig. 1). The prediction method refers to Rfam 14: expanded coverage of metagenomic, viral, and microRNA families. Hundreds of RNAs with conserved secondary structure were discovered, and gene family analysis was performed on 10 upstream or downstream proteins to explore other functional elements that cooperate with these defense-related RNA candidate proteins. This analysis identified a RAGATH-18-associated DNase system (RAD) comprising RAGATH-18-associated RNA and an IS607 element. The effector protein of RAD (IS607 TnpB) is the second coding sequence ORFB of IS607, encoding approximately 387 amino acids in size.

The inventors selected the source strains and effector proteins of a total of 99 (Table 1) RAD (IS607 TnpB) systems as representatives for subsequent analysis. Based on the effector proteins of the 99 RAD systems, the inventors had obtained the DNA sequence information of candidate proteins as well as the corresponding amino acid sequences and the sequence information of RAGATH-18-associated RNA, from a plurality of genomic data mentioned above. The candidate protein sequences and the sequences of the corresponding RAGATH-18-associated RNA are shown in Table 2, along with the sequences below Table 2. According to the strain ID, protein ID and amino acid information listed herein, the corresponding DNA information of the proteins can be obtained in the above database.

### Example 2: Expression and purification of RAD (IS607 TnpB) protein-nucleic acid complex

The DNA coding sequences of the obtained candidate proteins were sent to a gene synthesis company (Jinweizhi Biotechnology Co., Ltd.) for gene synthesis, and Pgex-6p1 universal vector was selected as the expression vector. The gene synthesis company synthesized 99 expression plasmids PGEX-RAD 1-99 (containing coding sequences of the candidate protein of each of SEQ ID NOs: 1-57 listed in Table 2 and SEQ ID NOs: 150-191 listed subsequently, respectively and the corresponding RNA coding sequences). The insertion sites for all expression cassettes were BamH1 and EcoR1 sites, and the structure of the expression cassettes was **Rad protein**-6HIS-**RNA**-N20-HDV (wherein Rad protein was each of SEQ ID NOs: 1-57 and SEQ ID NOs: 150-191, such as FbRAD1 protein of SEQ ID NO: 6).

The sequence of an exemplary plasmid PGEX-6P1-FbRAD1-N20-HDV is shown below, wherein the RAD protein (i.e., FbRAD1 protein) is underlined and in bold; 6HIS is underlined; the RNA coding sequence is italicized and underlined; N20 is in bold; and HDV is italicized.

ATGAGGAAATTGCTGAAGAGCTTCAAGACGGAAATAAATCCAACA
GAAGAGCAGAAAGTCAGGATTCGTAAAACAATAGGTACTTGCAGATTTATC
TATAACTTCTATCTTGCTCATAATAAGGAGCTTCATGAAAGTGGCAAAAAGT
TTATGAGCAGCAGCCAATTCAGAGTATGGCTCAACAATGAATATCTTCCGA
GCCATCCGGAATATTCCTGGATCAAGGAAGCTTATTCAAAATCGGTAACAC

AGGCAGTAAATGATGGACAGACCGCATTTACAAGATTCTTTAATCACAAGA
GCGCCTTTCCAAAGTTTAAAAAGAAAGGCAAGTCTGATGTGAAAATGTATT
TTGTAAGGAATAATCCAAAAGATTGCAGCTGCGAAAGACACAGAATCAAGA
TTCCTTCACTTGGCTGGGTCCGTATCAAAGAGAAAGGATATATTCCCACTAC
AAAAGATGGATATGTAATTAAGAGCGGTCATGTCTCAATAAAAGCTGATAG
ATACTATGTTTCAGTTCTTATAGAAATCCCAGACAAAACGTCAGCTAGTAAT
TCCAGTGAAGGAATTGGCATTGACCTTGGATTAAAGGACTTTGCGATTGCT
TCTAATGGAAAGACTTATAAGAACATCAACAAATCTGCTAAATTAAAGAAGC
TTGAAAAAAAGCTTATTCGAGAACAGAGAAGTCTTTCTCGAAAGTATGAAA
ATATAAAGAAAGGAGGGTCCACTCAAAAAAGAAATATACAAAAACAAAGGC
TTAAGATACAGAAACTTCATCATAGAATCGACAATATTCGTACTGATTACAT
CAATAAGATAATCGCAGAGATAGTGAAAACCAAGCCATCTTATATAACGAT
TGAAGATCTAAACATATCAGGGATGATGAAGAACAGACATCTTTCAAAGGC
AGTTGCTTCGCAGAAGTTTTATGAATTTAAGACAAAGCTTCAAGCTAAATGC
AGGGAGAACGGAATCGAACTTCGAGTTGTAGATAGATGGTTCCCATCATCT
AAGACTTGTCACTGCTGTAAAAGTATCAAGAAAGATTTAAAGCTTTCAGATA
GACTTTTCAGATGTGATTGTGGATATATCGAAGACAGAGATTTCAATGCTG
CACTAAATCTGAGAGATGCTACAACTTACGAAGTTGCAAGCAGCCATCACCA
TCATCACCACTAAATGAACGCAAACGTAAGTATGTACCGAAGGCTATTTCGGGAATTT
ACGACTATGGAGTGCACTAGAACTTGTGAGTAGATATGATTTCGGTCAATCCAAAGCAT
GCACAGTGACATAGTAAGTAATGTTCGTGAGAACTTACATTATCTCGATGTGAGTATAT
TTAATCACATTTTGAGTGGCAGCTGATGGTCCATGTCTGTTAGGCCGGCATGGTC
CCAGCCTCCTCGCTGGCGCCGGCTGGGCAACATGCTTCGGCATGGCGAATGGGACC
CG (SEQ ID NO: 115)

The expression plasmids of other RAD proteins were synthesized by the gene synthesis company in a similar manner to PGEX-6P1-FbRAD1-N20-HDV, except that the coding sequence of FbRAD1 was replaced with a coding sequence of other RAD proteins and the RNA sequence for FbRAD1 was replaced with a RNA sequence for other RAD systems, thereby obtaining the expression plasmids of all 99 candidate proteins, PGEX-RADs 1-99 (including PGEX-6P1-FbRAD1-N20-HDV). All plasmids were verified by the gene synthesis company to be correctly cloned.

Plasmid transformation experiment: the competent *E. coli* C43 (DE3) cells stored in a -80°C refrigerator were taken out and placed on ice. After thawing, 100 ng of each of the expression plasmids PGEX-RADs 1-99 was added to 50 µL of *E. coli* cells respectively, mixed and incubated on ice for 30 minutes. The cells were then heat-shocked in a 42°C water bath for 90 seconds and immediately placed on ice for 2 minutes, which were aseptically operated in an ultra-clean workbench. 500 µL of LB liquid culture medium without antibiotics was added to the cells. The cells were cultured with shaking in a shaker at the constant temperature of 37°C for 45-60 minutes, then evenly spread on LB plates containing 100 µg/mL antibiotic ampicillin. The plates were inverted and cultured overnight in an incubator at 37°C, and single colonies were seen on the second day.

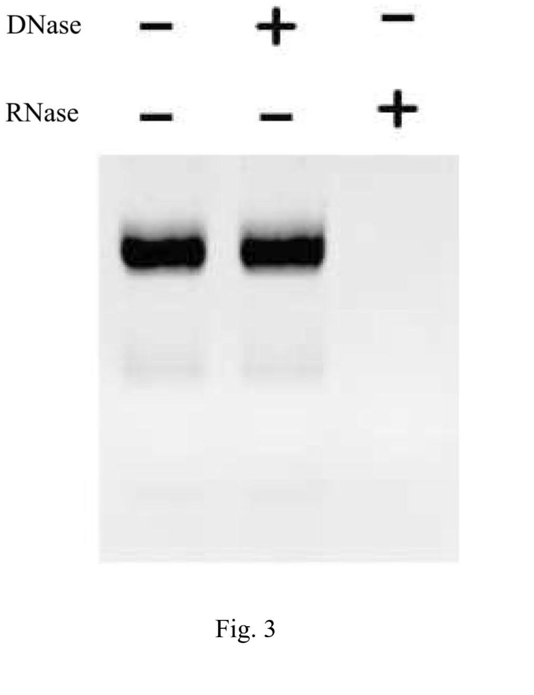

The expression and purification of RAD (IS607 TnpB) systems: positive clones were picked and inoculated into 5 mL of LB liquid medium containing antibiotic ampicillin. The cells were cultured while shaking in a shaker at 37°C for 12 hours. Then, cells were transferred to LB liquid medium containing antibiotic ampicillin, and cultured in a shaker at a constant temperature of 37°C to the OD600 of about 0.6. The culture was cooled to 18°C, and IPTG was added to a final concentration of 0.6 mmol/L in order to induce the expression for 16 hours. The bacterial cells were collected on the second day. Using a gravity column with column-material of GST-Sepharose, and utilizing the affinity effect between the GST tag and GST-Sepharose to extract protein from the cell lysate. The bacterial solution cultured overnight was centrifuged at 4 °C and 4000 rpm for 10 minutes. Then the resuspension solution was added at 20 mL GST resuspension solution per 1 L of bacterial solution and the bacterial cells were resuspended by shaking thoroughly with a vortex shaker. During resuspension, the protease inhibitor PMSF was added at a final concentration of 2 mmol/L, and the resuspension solution comprises 25 mmol/L Tris (pH 8.0), 1 mol/L NaCl, and 3 mmol/L DTT. Ultrasonication was used to fully lyse the resuspended bacterial cells. The whole ultrasonication process should be carried out on ice. In order to prevent the probe from overheating, it was necessary to pause for 3 seconds every 3 seconds of operation. The ultrasonication was performed for 1 minute, generally for 5-8 times, until the bacterial solution was no longer viscous and was homogenized. The lysed bacterial solution was dispensed into high-speed centrifuge tubes precooled at 4 °C, and centrifuged at 15,000 rpm and 4 °C for 40-50 minutes. Then the supernatant was removed. The supernatant was loaded into a gravity column filled with 2 mL of column-material GST Sepharose, and allowed to completely flow through the column material. Salt (NaCl) gradient concentration wash was used to remove non-specifically bound proteins and nucleic acids. 40 mL of GST Washing buffer I (25 mmol/L Tris (pH 8.0), 1.5 mol/L NaCl, 3 mmol/L DTT) was firstly used to wash the column, followed by washing with 15 mL of GST Washing buffer II (25 mmol/L Tris (pH 8.0), 0.5 mol/L NaCl, 3 mmol/L DTT), and finally 10 mL of GST Washing buffer III (25 mmol/L Tris (pH 8.0), 0.3 mol/L NaCl, 3 mmol/L DTT) was used to wash the column, with all Washing buffer being pre-cooled at 4°C. 5 mL of GST Washing buffer III was load into the column, followed by sealing the gravity column. 80 µL of Prescission protease was added for digestion overnight at 4°C. Then the column was resuspended 3 to 4 times, each with 30-minute interval. Another 5 mL of GST Washing buffer III was added on the second day and the effluent was collected. After concentration to 2 mL, the solution was centrifuged at 15000 rpm and 4°C for 5 minutes in preparation for the next step of purification. The gel filtration chromatography column (Superdex200 100/300) was connected to an FPLC Flash Protein Liquid Chromatography workstation as per the instruction of the device. The gel filtration chromatography column (Superdex200 100/300) was equilibrated with a pre-cooled gel filtration chromatography buffer (10 mmol/L Tris-HCl pH 8.0, 150 mmol/L NaCl, 3 mmol/L DTT, 2 mmol/L MgCl₂). The column was equilibrated for at least one or more column volumes, until the ultraviolet absorption at 280 nm was stable and no longer changed. 2 mL of sample was injected into the loading loop of the workstation and the preset program of the workstation was run. After completion of the program, the purity of the proteins was observed by 10% SDS-PAGE, and binding with nucleic acid was observed by 10% denaturing Urea-PAGE gel stained with EB. For the FbRAD1 complex, Fig. 3 shows the results of 10% denaturing Urea-PAGE gel stained with EB (far left of Fig. 3). It was confirmed by PAGE that the actual molecular weights of RAD protein and RNA of all 99 RAD nucleic acid complexes was consistent with the expected molecular weights, and the RAD nucleic acid complexes were correctly expressed. The following table shows the experimental data for several exemplary complexes.

| Protein name | ID | Expected Protein size | Experimentally observed size | Expected RNA size | Observed RNA size |
|---|---|---|---|---|---|
| FbRAD1/ISFba1 | RHS75625.1 | 45.0 kDa | about 45 kDa | 187 nt | about 180 nt |
| CbRAD1/ISCba1 | RHR46218.1 | 45.4 kDa | about 45 kDa | 186 nt | about 180 nt |
| RiRAD/ISRin | RGR69673.1 | 44.9 kDa | about 45 kDa | 187 nt | about 180 nt |
| ErRAD/ISErel | RHB06265.1 | 44.9 kDa | about 45 kDa | 187 nt | about 180 nt |
| WcRAD | RGR87203.1 | 43.5 kDa | about 44 kDa | 155 nt | about 160 nt |
| BsRAD/ISBsp4 | RHV03091.1 | 45.3 kDa | about 45 kDa | 184 nt | about 180 nt |
| CbRAD2/ISClsp2 | RHR42937.1 | 45.0 kDa | about 45 kDa | 187 nt | about 180 nt |
| CbRAD3/ISClsp3 | RGD88909.1 | 45.3 kDa | about 45 kDa | 186 nt | about 180 nt |
| CcRAD/ISCco | RGT87174.1 | 45.4 kDa | about 45 kDa | 168 nt | about 160 nt |
| FbRAD2/ISFba2 | RGH01875.1 | 45.5 kDa | about 45 kDa | 168 nt | about 160 nt |
| FbRAD3/ISFba3 | RHU26542.1 | 44.8 kDa | about 45 kDa | 187 nt | about 180 nt |
| FbRAD4/ISFba4 | RGH37793.1 | 45.2 kDa | about 45 kDa | 187 nt | about 180 nt |

Nucleic acid identification: The RAD-nucleic acid complex with a final concentration of 5 mg/ml was treated in a 10 µL reaction system using DNase or RNase with a final concentration of 0.1 mg/ml, respectively. The results are shown in Fig. 3. The nucleic acid on the complex was completely degraded only by RNase, indicating that the nucleic acid bound by RAD protein was RNA. All 99 RAD-nucleic acid complexes were validated, and the results demonstrated that the nucleic acids bound to RAD proteins in the 99 RAD nucleic acid complexes were RNAs.

RNA sequencing: The purified complex was sent to a gene sequencing company (Shanghai Personal Biotechnology Co.,Ltd) for processing to obtain sequence data information (Fig. 18).

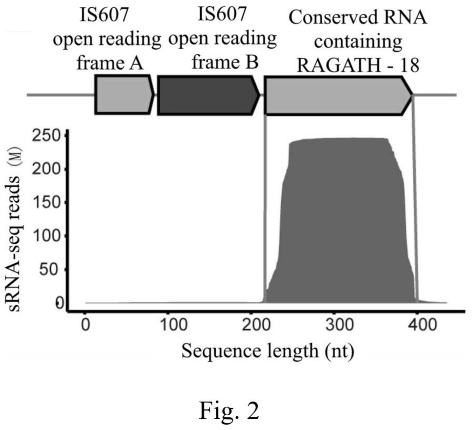

According to the sequencing results, the inventors found that the RNA bound to the RAD protein was a non-coding RNA containing RAGATH-18, which is downstream of the second coding frame of IS607 (as shown in Fig. 2), indicating that the RAD system was able to express and form a protein-RNA complex. This RNA is unique to this system, and there is no relevant study involving the function between this segment of non-coding RNA and the upstream protein.

### Example 3: Determination of PAM sequences and the enzymatic digestion activity

The construction of a substrate for enzymatic digestion: construction of a PAM substrate plasmid library: a plasmid library covering any combination of 7 bases. Primers PAM-I-F and PAM-I-R were used for PCR amplification (amplification time 3 minutes, primer annealing temperature of 58°C, number of amplification cycles being 30) of the recombinant fragments. Primers PAM-V-F and PAM-V-R were used for PCR amplification (amplification time 3 minutes, primer annealing temperature of 58°C, number of amplification cycles being 30) of the recombinant vector PUC19. Using the recombinase system (available from Vazyme Biotech Co., Ltd., Cat. No. Vazyme. C112-01), the fragment and vector PUC19 were recombined and transformed into *E. coli* DH5α competent cells. The cells were directly expanded and cultured, and the plasmid library was extracted using a gel extraction kit (Cat. No. CW2302; Cowin Biotech Co., Ltd.) according to the manufacturer's instructions.

The PAM plasmid library engineered based on the PUC19 plasmid is shown below, with N representing any of the four types of bases of A, T, C, or G, and the targeted sequence underlined.

gcgcccaatacgcaaaccgcctctccccgcgcgttggccgattcattaatgcagctggcacgacaggtttcccgac
tggaaagcgggcagtgagcgcaacgcaattaatgtgagttagctcactcattaggcaccccaggctttacactttatgcttccggctcg
tatgttgtgtggaattgtgagcggataacaatttcacacaggaaacagctatgaccatgattacgccaagcttgcatgcctgcaggcct
ggacatccgcgagcacagctaaggccanNNNNNNCTGATGGTCCATGTCTGTTActcacaccgaccggc
acagcatcaagaagcgggtaccgagctcgaattcactggccgtcgttttacaacgtcgtgactgggaaaaccctggcgttacccaact
taatcgccttgcagcacatccccctttcgccagctggcgtaatagcgaagaggcccgcaccgatcgcccttcccaacagttgcgcag
cctgaatggcgaatggcgcctgatgcggtattttctccttacgcatctgtgcggtatttcacaccgcatatggtgcactctcagtacaatc
tgctctgatgccgcatagttaagccagccccgacacccgccaacacccgctgacgcgccctgacgggcttgtctgctcccggcatcc
gcttacagacaagctgtgaccgtctccgggagctgcatgtgtcagaggttttcaccgtcatcaccgaaacgcgcgagacgaaaggg
cctcgtgatacgcctatttttataggttaatgtcatgataataatggtttcttagacgtcaggtggcacttttcggggaaatgtgcgcggaa
cccctatttgtttatttttctaaatacattcaaatatgtatccgctcatgagacaataaccctgataaatgcttcaataatattgaaaaaggaa
gagtatgagtattcaacatttccgtgtcgcccttattcccttttttgcggcattttgccttcctgtttttgctcacccagaaacgctggtgaaa

gtaaaagatgctgaagatcagttgggtgcacgagtgggttacatcgaactggatctcaacagcggtaagatccttgagagttttcgccc
cgaagaacgttttccaatgatgagcacttttaaagttctgctatgtggcgcggtattatcccgtattgacgccgggcaagagcaactcgg
tcgccgcatacactattctcagaatgacttggttgagtactcaccagtcacagaaaagcatcttacggatggcatgacagtaagagaat
tatgcagtgctgccataaccatgagtgataacactgcggccaacttacttctgacaacgatcggaggaccgaaggagctaaccgcttt
tttgcacaacatgggggatcatgtaactcgccttgatcgttgggaaccggagctgaatgaagccataccaaacgacgagcgtgacac
cacgatgcctgtagcaatggcaacaacgttgcgcaaactattaactggcgaactacttactctagcttcccggcaacaattaatagact
ggatggaggcggataaagttgcaggaccacttctgcgctcggcccttccggctggctggtttattgctgataaatctggagccggtga
gcgtgggtctcgcggtatcattgcagcactggggccagatggtaagccctcccgtatcgtagttatctacacgacggggagtcaggc
aactatggatgaacgaaatagacagatcgctgagataggtgcctcactgattaagcattggtaactgtcagaccaagtttactcatatat
actttagattgatttaaaacttcatttttaatttaaaaggatctaggtgaagatcctttttgataatctcatgaccaaaatcccttaacgtgagtt
ttcgttccactgagcgtcagaccccgtagaaaagatcaaaggatcttcttgagatcctttttttctgcgcgtaatctgctgcttgcaaacaa
aaaaaccaccgctaccagcggtggtttgtttgccggatcaagagctaccaactctttttccgaaggtaactggcttcagcagagcgca
gataccaaatactgttcttctagtgtagccgtagttaggccaccacttcaagaactctgtagcaccgcctacatacctcgctctgctaatc
ctgttaccagtggctgctgccagtggcgataagtcgtgtcttaccgggttggactcaagacgatagttaccggataaggcgcagcggt
cgggctgaacggggggttcgtgcacacagcccagcttggagcgaacgacctacaccgaactgagatacctacagcgtgagctatg
agaaagcgccacgcttcccgaagggagaaaggcggacaggtatccggtaagcggcagggtcggaacaggagagcgcacgagg
gagcttccagggggaaacgcctggtatctttatagtcctgtcgggtttcgccacctctgacttgagcgtcgatttttgtgatgctcgtcag
gggggcggagcctatggaaaaacgccagcaacgcggcctttttacggttcctggccttttgctggccttttgctcacatgttctttcctgc
gttatcccctgattctgtggataaccgtattaccgcctttgagtgagctgataccgctcgccgcagccgaacgaccgagcgcagcgag
tcagtgagcgaggaagcggaaga (SEQ ID NO: 118).

2700bp double-stranded DNA substrate: a 2700bp double-stranded DNA fragment was amplified by PCR using primers DS2700-F and DS2700-R, using the PAM substrate plasmid library as template. The amplification time was 3 minutes, the primer annealing temperature was 58°C, and the number of amplification cycles was 30. The PCR products were separated by 1% agarose gel electrophoresis, and the product was recovered. 2700bp mismatched double-stranded DNA substrate (the base complementarity of CTGATGGTCCATGTCTGTTA was changed one base by one base to form a mismatch), and the construction method was the same as described above. 5'FAM-labeled single-stranded DNA substrates: 5FAM-55TAM-F, 5FAM-55TAM-R, 5FAM-55NOTAM-F, 5FAM-55NOTAM-R, 5FAM-NTSSDNA was synthesized by a gene synthesis company (Comate Bioscience Co., Ltd.), in which the specific sequences of primers and the sequences of single-stranded DNA substrates are shown below.

### 2700bp double-stranded DNA substrate (AGGAG)

The 2700bp double-stranded DNA substrate employed in the *in vitro* enzymatic digestion experiment was derived from the pUC19 plasmid. This DNA substrate was amplified using primers DS2700-F and DS2700-R, after which it was obtained and recovered.

If the underlined sequence below is replaced with GAGGG, it becomes a GAGGG substrate.

5’-
tgagtgataacactgcggccaacttacttctgacaacgatcggaggaccgaaggagctaaccgcttttttgcacaacatgggggatca
tgtaactcgccttgatcgttgggaaccggagctgaatgaagccataccaaacgacgagcgtgacaccacgatgcctgtagcaatggc
aacaacgttgcgcaaactattaactggcgaactacttactctagcttcccggcaacaattaatagactggatggaggcggataaagttg
caggaccacttctgcgctcggcccttccggctggctggtttattgctgataaatctggagccggtgagcgtgggtctcgcggtatcatt
gcagcactggggccagatggtaagccctcccgtatcgtagttatctacacgacggggagtcaggcaactatggatgaacgaaatag
acagatcgctgagataggtgcctcactgattaagcattggtaactgtcagaccaagtttactcatatatactttagattgatttaaaacttca
tttttaatttaaaaggatctaggtgaagatcctttttgataatctcatgaccaaaatcccttaacgtgagttttcgttccactgagcgtcagac
cccgtagaaaagatcaaaggatcttcttgagatcctttttttctgcgcgtaatctgctgcttgcaaacaaaaaaaccaccgctaccagcg
gtggtttgtttgccggatcaagagctaccaactctttttccgaaggtaactggcttcagcagagcgcagataccaaatactgttcttctag
tgtagccgtagttaggccaccacttcaagaactctgtagcaccgcctacatacctcgctctgctaatcctgttaccagtggctgctgcca
gtggcgataagtcgtgtcttaccgggttggactcaagacgatagttaccggataaggcgcagcggtcgggctgaacggggggttcg
tgcacacagcccagcttggagcgaacgacctacaccgaactgagatacctacagcgtgagctatgagaaagcgccacgcttcccg
aagggagaaaggcggacaggtatccggtaagcggcagggtcggaacaggagagcgcacgagggagcttccagggggaaacg
cctggtatctttatagtcctgtcgggtttcgccacctctgacttgagcgtcgatttttgtgatgctcgtcaggggggcggagcctatggaa
aaacgccagcaacgcggcctttttacggttcctggccttttgctggccttttgctcacatgttctttcctgcgttatcccctgattctgtggat
aaccgtattaccgcctttgagtgagctgataccgctcgccgcagccgaacgaccgagcgcagcgagtcagtgagcgaggaagcgg
aagagcgcccaatacgcaaaccgcctctccccgcgcgttggccgattcattaatgcagctggcacgacaggtttcccgactggaaa
gcgggcagtgagcgcaacgcaattaatgtgagttagctcactcattaggcaccccaggctttacactttatgcttccggctcgtatgttg
tgtggaattgtgagcggataacaatttcacacaggaaacagctatgaccatgattacgccaagcttgcatgcctgcaggcctggacat

ccgcgagcacagctaaggccagggaggagCTGATGGTCCATGTCTGTTActcacaccgaccggcacagcatca
agaagcgggtaccgagctcgaattcactggccgtcgttttacaacgtcgtgactgggaaaaccctggcgttacccaacttaatcgcctt
gcagcacatccccctttcgccagctggcgtaatagcgaagaggcccgcaccgatcgcccttcccaacagttgcgcagcctgaatgg
cgaatggcgcctgatgcggtattttctccttacgcatctgtgcggtatttcacaccgcatatggtgcactctcagtacaatctgctctgatg
ccgcatagttaagccagccccgacacccgccaacacccgctgacgcgccctgacgggcttgtctgctcccggcatccgcttacaga
caagctgtgaccgtctccgggagctgcatgtgtcagaggttttcaccgtcatcaccgaaacgcgcgagacgaaagggcctcgtgata
cgcctatttttataggttaatgtcatgataataatggtttcttagacgtcaggtggcacttttcggggaaatgtgcgcggaacccctatttgt
ttatttttctaaatacattcaaatatgtatccgctcatgagacaataaccctgataaatgcttcaataatattgaaaaaggaagagtatgagt
attcaacatttccgtgtcgcccttattcccttttttgcggcattttgccttcctgtttttgctcacccagaaacgctggtgaaagtaaaagatg
ctgaagatcagttgggtgcacgagtgggttacatcgaactggatctcaacagcggtaagatccttgagagttttcgccccgaagaacg
ttttccaatgatgagcacttttaaagttctgctatgtggcgcggtattatcccgtattgacgccgggcaagagcaactcggtcgccgcat
acactattctcagaatgacttggttgagtactcaccagtcacagaa-3' (SEQ ID NO: 119)

**Table 3: The sequences of enzymatic digestion substrates and primers**

| **Name** | **Description** | **Sequence** | **SEQ ID NO** |
|---|---|---|---|
| **PAM-IF** | Forward primer for the construction of PAM plasmid library fragments | 5- cctggacatccgcgagcacagc taaggccaNNNNNNNCT GATGGTCCATGTCT GTTACTC-3 | 120 |
| **PAM-I-R** | Reverse primer for the construction of PAM plasmid library fragments | 5- cttcttgatgctgtgccggtcggt gtGAGTAACAGACAT GGACCATCAG-3 | 121 |
| **PAM-V-F** | Forward primers for the construction of PAM plasmid library vectors | 5- ggcacagcatcaagaagcggg taccgagctcgaattca-3 | 122 |
| **PAM-V-**R | Reverse primers for the construction of PAM plasmid library vectors | 5- agctgtgctcgcggatgtccag gcctgcaggcatgcaagcttg- 3 | 123 |
| **N20-TAR-F** | Forward primers for the construction of PGEX-6P1-FbRAD1-N20-HDV plasmid containing a 20bp targeted sequence | 5- CTCCTCGCTGGCGC CGGCTGGGCAACAT GCTTCGGCATGGCG AATGGGACCCGGAA TTCCCGGGTCGACT CGA-3 | 124 |
| **N20-TAR-R** | Forward primers for the construction of PGEX-6P1-FbRAD1-N20-HDV plasmid containing a 20bp targeted sequence | 5- CCAGCCGGCGCCAG CGAGGAGGCTGGG ACCATGCCGGCCTA ACAGACATGGACCA TCAGCTGCCACTCA AAATGTGATTAAAT -3 | 125 |
| **DS2700-F** | Forward primer for amplification of the 2700bp double-stranded DNA | 5- tgagtgataacactgcggccaa cttac-3 | 126 |
| **DS2700-R** | Reverse primers for amplification of the 2700bp double-stranded DNA | 5- ttctgtgactggtgagtactcaac caag-3 | 127 |
| **5FAM-55TAM-F** | 55 nt long, 5'-end FAM-modified forward single-stranded DNA containing AGGAG and the 20 nt targeted sequence | 5FAM- ggccaccattaggagCTGA TGGTCCATGTCTGT TACTCacaccgaccggcac agc-3 | 128 |
| **5FAM-55TAM-R** | 55 nt long, 5'-end FAM-modified reverse single-stranded DNA containing AGGAG and the 20 nt targeted sequence | 5FAM- gctgtgccggtcggtgtGAG TAACAGACATGGAC CATCAGctcctaatggtgg cc-3 | 129 |
| **5FAM-55NOT AM-F** | 55 nt long, 5'-end FAM-modified forward single-stranded DNA containing no AGGAG and containing the 20 nt targeted sequence | 5FAM- ggccaccattTTGATCTG ATGGTCCATGTCTG TTACTCacaccgaccggca cagc-3 | 130 |
| **5FAM-55NOT AM-R** | 55 nt long, 5'-end FAM-modified reverse single-stranded DNA containing no AGGAG and containing the 20 nt targeted sequence | 5FAM- gctgtgccggtcggtgtGAG TAACAGACATGGAC CATCAGATCAAaatgg tggcc-3 | 131 |
| **5FAM-NTSSD NA** | 50 nt long, 5'-end FAM-modified forward single-stranded DNA without AGGAG and the 20 nt targeted sequence | 5FAM- cggtatttcacaccgcatatggtg cactctcagtacaatctgctctga tg-3 | 132 |
| **20activa tor** | a single-stranded DNA of 20 nt complementary to 20 nt targeting sequence of the RAD system's RNA, for activation of trans-cleavage activity | 5- TAACAGACATGGAC CATCAG-3 | 133 |
| **Adapter -F** | The forward primer of the adapter sequence linked to the enzymatic digestion product in the TAM motif identification experiment, for subsequent high-throughput | 5- ATAAGGAGCTGGAG AACCTGGAGATCAA TCTGCGGAAGGAGA TCGCCAAGGCCTTC | 134 |
| | sequencing PCR amplification | AAGGGCAACGAGG GCTACAAGTCCT-3 | |
| **Adapter -R** | The reverse primer of the adapter sequence linked to the enzymatic digestion product in the TAM motif identification experiment, for subsequent high-throughput sequencing PCR amplification | 5- GGACTTGTAGCCCT CGTTGCCCTTGAAG GCCTTGGCGATCTC CTTCCGCAGATTGA TCTCCAGGTTCTCC AGCTCCTTAT-3 | 135 |
| **TAMSE Q-F** | The high-throughput sequencing forward primer for TAM motif identification | 5- GGAGTGAGTACGGT GTGCATAAGGAGCT GGAGAACCTG-3 | 136 |
| **TAMSE Q-R** | The high-throughput sequencing reverse primer for TAM motif identification | 5- GAGTTGGATGCTGG ATGGGAGCGGATAA CAATTTCACACAGG -3 | 137 |

*In vitro* enzymatic digestion experiment: 5 nM of each of the RAD protein-nucleic acid complexes was added into 20µL of reaction system (25mM Tris 8.0, 50mM NaCl, 2mM DTT, 5mM MgCl₂), and different substrates (200ng of PAM substrate plasmid library or 200ng of 2700bp double-stranded DNA substrate or 20ng 5' FAM-labeled single-stranded DNA substrate) were added for different reactions. The reaction time ranged from 30 seconds to 2 hours, and the reaction temperature was 37°C. The reaction was stopped by adding equal volumes of 2 x loading buffer (8 M urea, 0.05% bromophenol blue, 0.05% xylene blue, 1 x TBE).

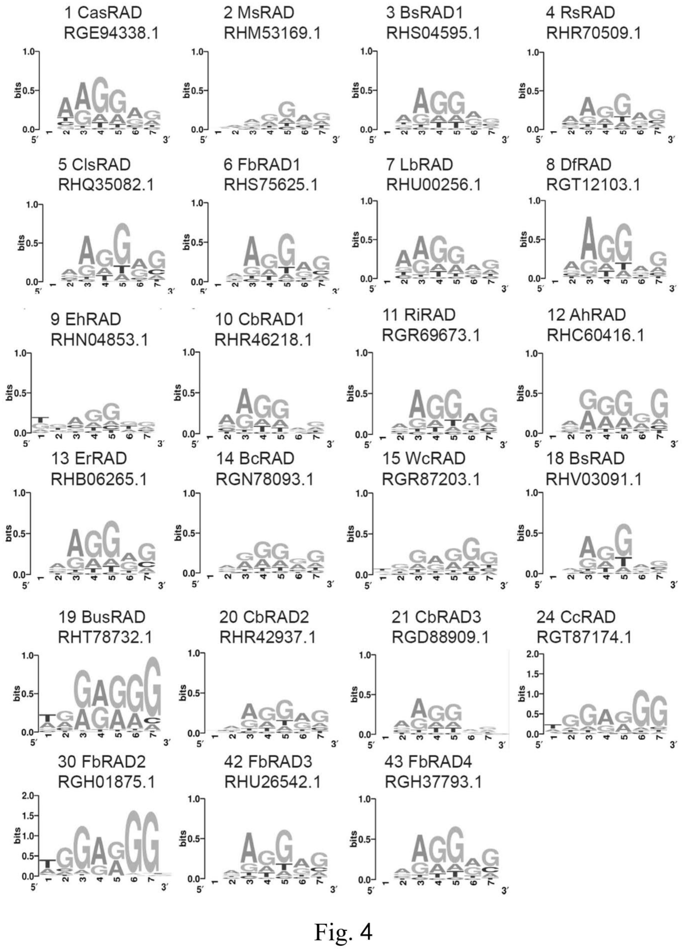

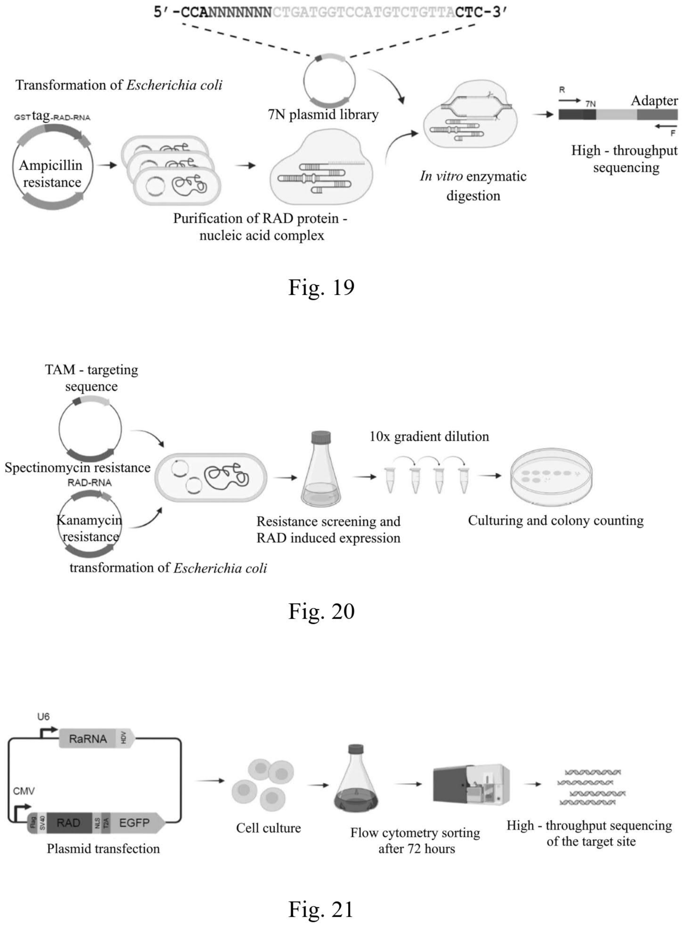

High-throughput sequencing of TAM sequences: the RAD system protein-nucleic acid complex and PAM substrate plasmid library were digested according to the above *in vitro* enzymatic digestion experiment, and then the reaction product was obtained and recovered. Then the recovered product was ligated into the adapter sequence (see in Table 3, Adapter-F/R) using TA cloning, and the ligated product sequence was amplified using primers TAMSEQ-F/R. The amplification time was 30 seconds, and the primer annealing temperature was 58°C, with 20 amplification cycles. The resulting samples were sent to a sequencing company for high-throughput sequencing analysis (Fig. 19). The obtained data were counted statistically to obtain the motif sequence (TAM) at the 5' end of the RAD systems (Fig. 4 and Table 4). The results showed that the TAM of different RAD systems numbered 1-57 showed a preference of AGGAG, and different RAD proteins also showed different preferences, such as GAGGG. The RNA proteins numbered 58-99 showed TAM preferences as shown in Table 4.

**Table 4: Motif sequences (TAM) at the 5' end of the RAD systems**

| Number | Protein ID | TAM |
|---|---|---|
| 58 | ERR3307045_63958_30277_4897_6000_- | NGGAG |
| 59 | GUT_GENOME151180_45_9178_10326 + | NGGNN |
| 60 | ERR2017626_1022590_12863_8034_9173_+ | AGGAG |
| 61 | GUT_GENOWE142162_18_64466_65659_- | NGGAG |
| 62 | NC 013216_1_421908_422903_+ | GAGGG |
| 63 | WP_035125047.1 | GGGGG |
| 64 | GUT_GENOME096560_11_244135_245259 - | GAGGG |
| 65 | GUT_GENOME083533_37_11359_12597_- | NNGGG |
| 66 | WP_023518930.1 | NNAGG |
| 67 | WP_012574395.1 | GGGGG |
| 68 | WP_099988772.1 | AGGNN |
| 69 | GUT_GENOME228548_94_18418_19641_+ | NTAAA |
| 70 | WP_012201916.1 | GGGGG |
| 71 | WP_081429131.1 | AGGAG |
| 72 | GUT_GENOME249453_33_14687_15901_- | NGGNN |
| 73 | GUT_GENOME046003_7_13445_14386 - | NGGNN |
| 74 | GUT_GENOME043660_1_131655_132716_- | GGGGG |
| 75 | WP_039636020.1 | GGGGG |
| 76 | WP_013360293.1 | NGAGG |
| 77 | GUT_GENOME243333_13_9207_10436 + | NNGGN |
| 78 | GUT_GENOME228909_131_318_1547 - | NNGGN |
| 79 | RGS74140.1 | NNGGN |
| 80 | GUT_GENOME029159_118_4969_5874 - | GAGGG |
| 81 | WP_012289953.1 | AGGAG |
| 82 | WP_044824965.1 | GGGGG |
| 83 | GUT GENOME100288 478 819 1994 - | NGGNN |
| 84 | GUT_GENOME089678_61_13091_14269_- | NNGGN |
| 85 | GUT_GENOME190643_17_8068_9183_- | GGGGN |
| 86 | ERR1539620_45264_57524_39821_41020 - | NNGGG |
| 87 | WP_072587064.1 | NNGGN |
| 88 | AYF55140.1 | GAGGG |
| 89 | AUM93701.1 | NNGGN |
| 90 | SRR5892202_233507_16022_11195_12361_+ | AGGNN |
| 91 | WP_000978855.1 | GGGGG |
| 92 | WP_000604588.1 | NNGGN |
| 93 | WP_077326813.1 | NGGAG |
| 94 | GUT_GENOME170581_2_14431_15588_- | NNGGG |
| 95 | WP_025697990.1 | AGGAG |
| 96 | QAT40560.1 | AGGAG |
| 97 | WP_014431089.1 | NNAGG |
| 98 | GUT_GENOME088555_122_512_1693_+ | GAGGG |
| 99 | AWX54000.1 | NNGGN |

### In vitro cleavage activity of the RAD systems

Construction of double-stranded DNA substrate: after obtaining the sequence of AGGAG, the inventors constructed a double-stranded DNA substrate that can be specifically recognized by RAD proteins (the sequence is shown above in the 2700 bp double-stranded DNA substrate). Specifically, the 2700bp double-stranded DNA substrate sequence used in the *in vitro* enzymatic digestion experiment was derived from PUC19 plasmid, and was obtained and recovered by an amplification with primers DS2700-F and DS2700-R.

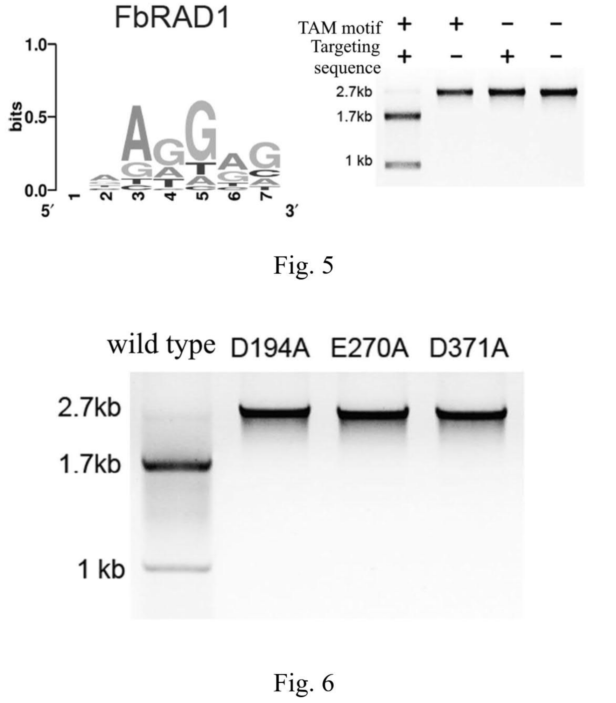

Determination of positions at which cleavage occurs: The FbRAD1 (RHS75625.1, SEQ ID NO: 6) system derived from the Firmicutes bacterium AM43-11BH was used as the experimental object. It was found that only when the TAM sequence and the targeted sequence existed at the same time, the RAD system (comprising the RAD protein SEQ ID NO: 6, its associated RNA sequence SEQ ID NO: 63 and the targeting sequence) could specifically cleave the double-stranded DNA substrate (comprising AGGAG and the targeted sequence), as shown in Fig. 5, where the left panel shows the analysis of the TAM sequence, and the right panel shows the results of cleavage. It shows that the RAD system has the same basic mechanism as conventional gene editing tools, and can function to specifically cleave double-stranded DNA *in vitro.*

The RAD system protein-nucleic acid complex and the 2700 bp double-stranded DNA substrate were enzymatically digested *in vitro* according to Example 4 above to obtain a reaction product. The PAM substrate plasmid library and 2700 bp double-stranded DNA substrate were separated into product bands using 1% agarose gel electrophoresis, and the 5'FAM labeled single-stranded DNA substrate was separated into product bands using 15% denaturing Urea-PAGE gel. The recovered products were sent to the sequencing company for Sanger sequencing and high-throughput sequencing, and the sequencing maps and quantitative statistical data of different cleavage positions were obtained, respectively.

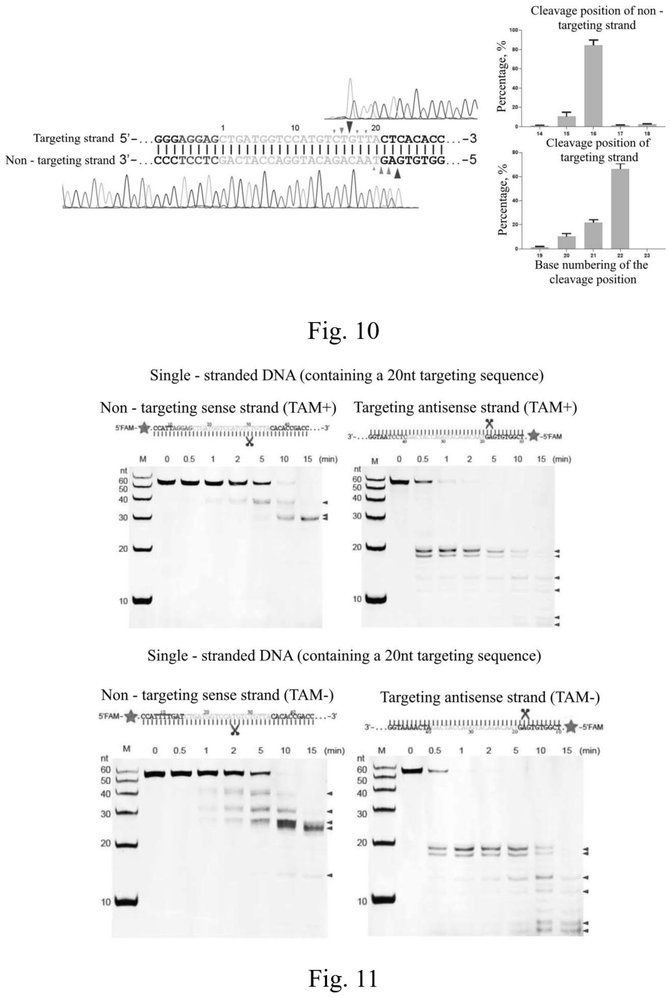

Using high-throughput sequencing and Sanger sequencing, it was found that RAD protein cleaved the double-stranded substrate DNA to produce a sticky end nick. The cleavage mainly occurred at the base position 22 of the targeted strand and the base position 16 of the non-targeted strand, respectively (Fig. 10) (for the experimental procedures, see *in vitro* enzymatic digestion experiment). A small amount of cleavage was produced at other positions. This experiment again proved that RAD protein had specific double-stranded DNA endonuclease activity.

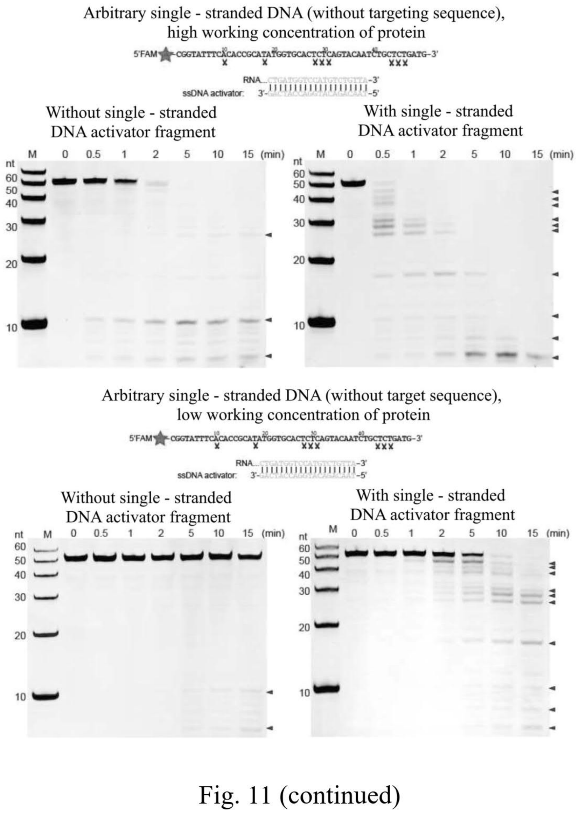

*In vitro* single-stranded enzymatic digestion experiment: the 5' end of single-stranded DNA was fluorescently labeled with FAM to perform an *in vitro* enzymatic digestion experiment, as described in the above *in vitro* enzymatic digestion experiment. As a result, as shown in Fig. 11, the RAD system can specifically cleave not only double-stranded DNA, but also single-stranded DNA. The cleavage of the single-stranded DNA did not require the TAM sequence. The activity of cleaving single-stranded DNA that matches the RNA targeting sequence was significantly higher than that for non-matching DNA. Furthermore, the single-stranded DNA was cleaved without matching the targeting sequence. The 20-base single-stranded DNA matching the end of the RNA (20activator in Table 3) was added for activation of the RAD protein. Subsequently, the activity of the RAD protein to cleave the non-specific single-stranded DNA was significantly increased, indicating that it has the trans-cleavage activity of single-stranded DNA. This single-stranded DNA cleavage activity can be developed as an identification tool for single-stranded DNA viruses as follows. A sequence from a target virus is constructed into the RNA of the RAD system. A single-stranded DNA substrate is used which produces chemical or fluorescent signals after cleavage. When the RAD system recognizes the target virus, the trans-cleavage activity is activated, and the substrate is cleaved followed by generation of a signal, thereby identifying the target virus.

### Determination of conditions of cleavage

A series of *in vitro* enzymatic digestion experiments were carried out under different conditions, and the experimental processes were the same as above. Suitable conditions for temperature, divalent cation type, divalent cation concentration, targeting sequence length, and salt concentration were obtained.

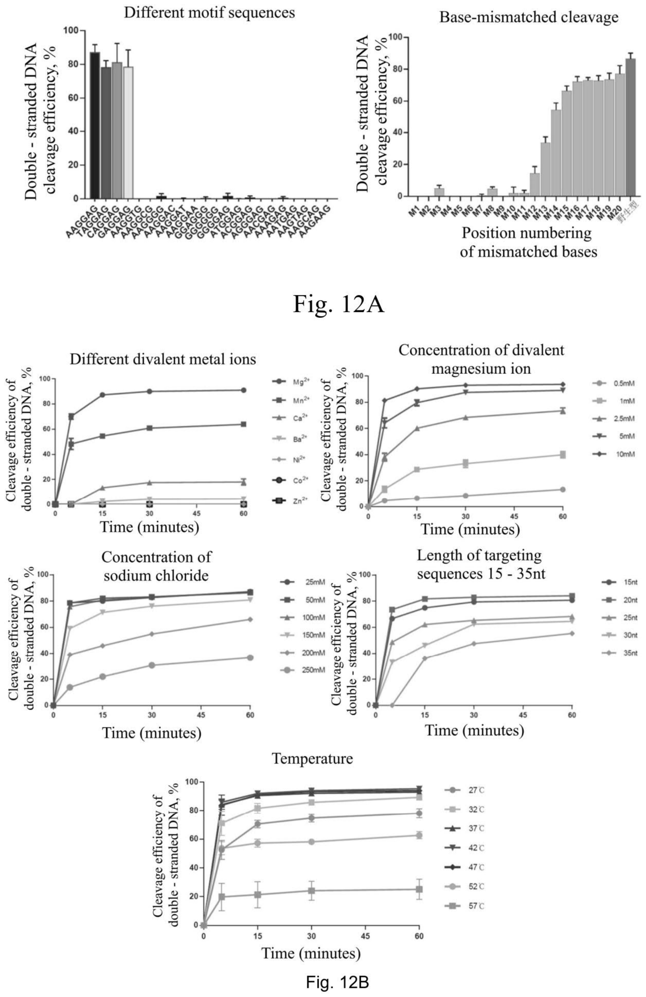

The results are shown in Fig. 12B. The results showed the suitable conditions of *in vitro* enzymatic digestion for the RAD system containing FbRAD1. The cleavage activity was considerably high at 42 °C in presence of 5 to 10 mM MgCl₂ and 25 to 100 mM NaCl, and the activity was highest with a targeting sequence length of 20 bases.

### Effects of motifs and mismatched sequences

Based on the constructed enzymatic digestion substrate and *in vitro* enzymatic digestion experiments as described above, 5 nM of FbRAD1 protein-nucleic acid complex and 200 ng of 2700bp double-stranded DNA substrate were added to 20 µL reaction system (25 mM Tris 8.0, 50 mM NaCl, 2 mM DTT, 5 mM MgCl₂) and were reacted at 37°C for 30 minutes. DNA cleavage with different TAM sequences was verified. It was found that AGGAG was the most suitable TAM for FbRAD1 protein which recognizes AGGAG sequence, and a change of any base would greatly reduce the cleavage activity or even result in loss of the cleavage activity (Fig. 12A).

Based on the constructed enzymatic digestion substrates and *in vitro* enzymatic digestion experiments as described above, the 2700 bp mismatched double-stranded DNA substrate was used. Mismatch cleavage experiments showed that a base mismatch in positions 14 to 20 at the 3' end of the targeting sequence reduced cleavage activity, but the effect was little. However, a mismatch at positions 1-13 greatly reduced the cleavage activity. The results of this experiment showed that the RAD system had a low tolerance to a base mismatch at positions 1-13, indicating the specificity of the RAD system was strong. As conventional CRISPR/Cas9 and Cas12a can still result in cleavage in the presence of the mismatch at positions 6 to 13, their specificities are poorer than that of the RAD system (Fig. 12B).

### Example 4: Analysis of RAD complex domain

Through a domain analysis, it was found that RAD protein contained a RuvC domain with three active centers, which in the FbRAD1 system were aspartic acid at amino acid position 194, glutamic acid at position 290, and aspartic acid at position 371. The three amino acids were highly conserved in all RAD proteins. In the domain analysis, firstly, the structurally resolved Cas proteins with RuvC were collected, and three RuvC domain sequences were picked up, respectively, to perform three iterative distal homology searches in the NCBI database, with evalue of 1e-4. Then, multiple-sequence alignment of the sequence of each domain was performed together with its homologous sequences. Then, hmm was constructed based on the results of the multiple-sequences alignment, using the commonly used tool hmmscan with the threshold set to evalue=1e-10, HMMER web server: interactive sequence similarity searching.

The wild-type sequence is FbRAD1. The mutants D194A, E290A, and D371A with amino acid inactive mutations to alanine of the three active centers in this example were prepared using the amino acid point mutation kit from Vazyme Biotech Co., Ltd. according to the instructions of the kit. Expression and purification of the RAD (IS607 TnpB) protein-nucleic acid complex were shown in Example 1. The double-stranded DNA cleavage of RAD (IS607 TnpB) protein-nucleic acid complexes of wild-type, mutants D194A, E290A, and D371A were determined as described in Example 3.

Functional verification of active centers: as shown in Fig. 6, the RAD protein lost the activity to cleave double-stranded DNA after the inactive mutations to alanine of the amino acids at the three active centers, indicating that the RuvC domain of the RAD protein itself exerted DNA endonuclease activity (Fig. 6).

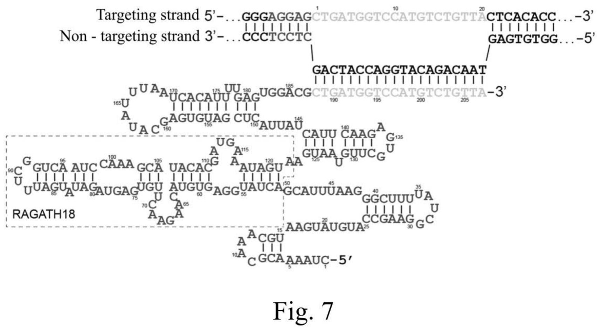

Structural analysis: using the RNA secondary structure prediction website RNA folder web server, it was found that the RNA of RAGATH-18 formed the longest stem-loop structure in the entire complex, as shown in Fig. 7.

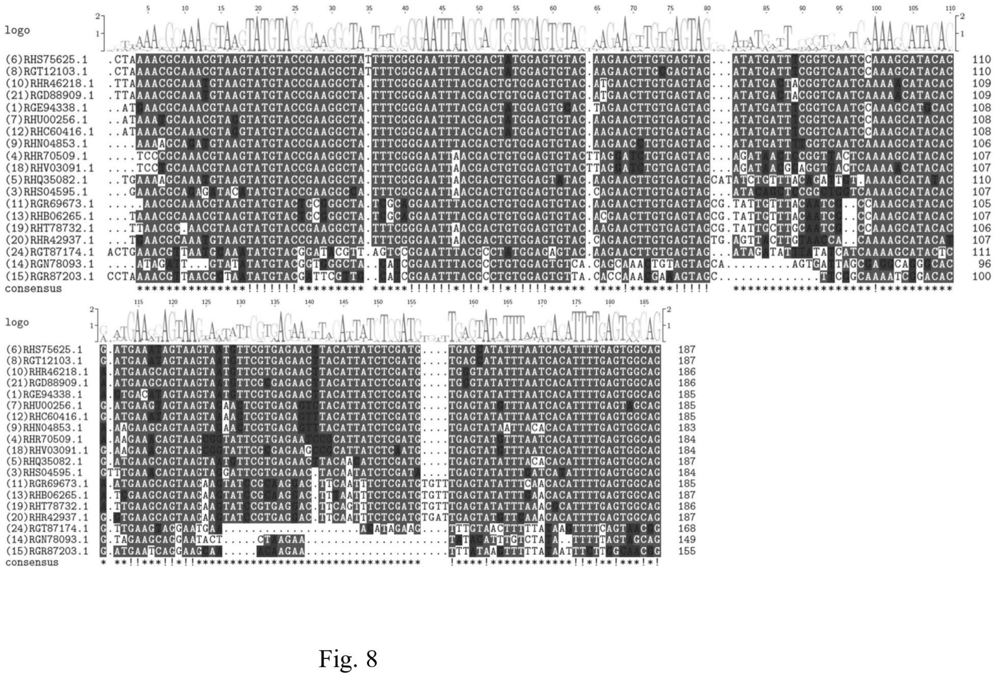

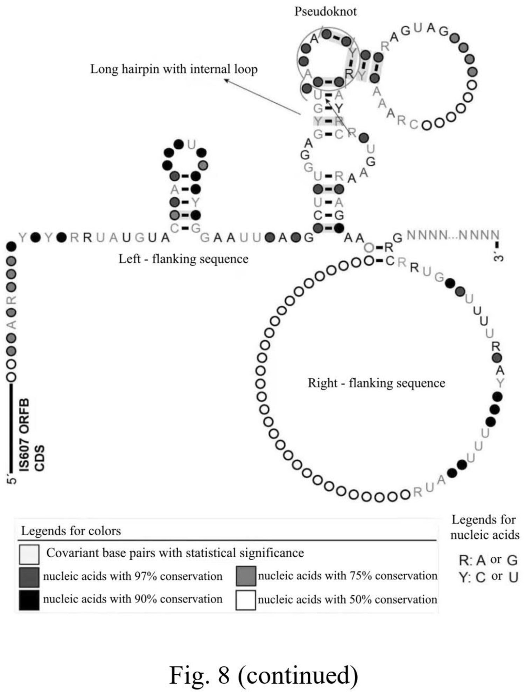

As shown in Fig. 8, RNA multiple-sequence alignment analysis on a variety of RAD systems with DNase activity was performed in the MAFFT version 7 website, using FbRAD1 (RHS75625.1) as the alignment reference. The inventors found that the average length of RAGATH18 RNA was 75 bp, containing a predicted long hairpin with an inner loop and a pseudo-knot, and the both left and right flanking sequences of RAGATH18 were also conserved. The left flanking sequence was about 50 bp in length containing a predicted stem loop, and the right flanking sequence was about 60 bp in length. The RNA sequences of RAGATH-18 with the predicted stem loop (Fig. 8) were highly conserved in RAD systems. The results indicated that RAGATH-18 RNA was necessary in the RAD system, and also indicated that the RAD system with RAGATH-18-associated RNA had DNase activity.

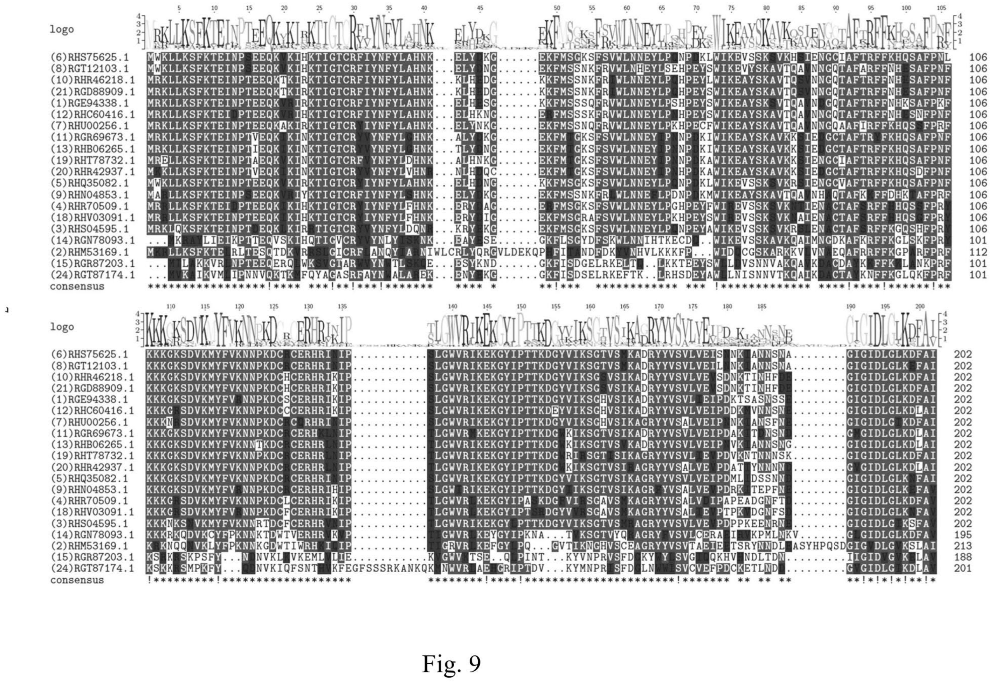

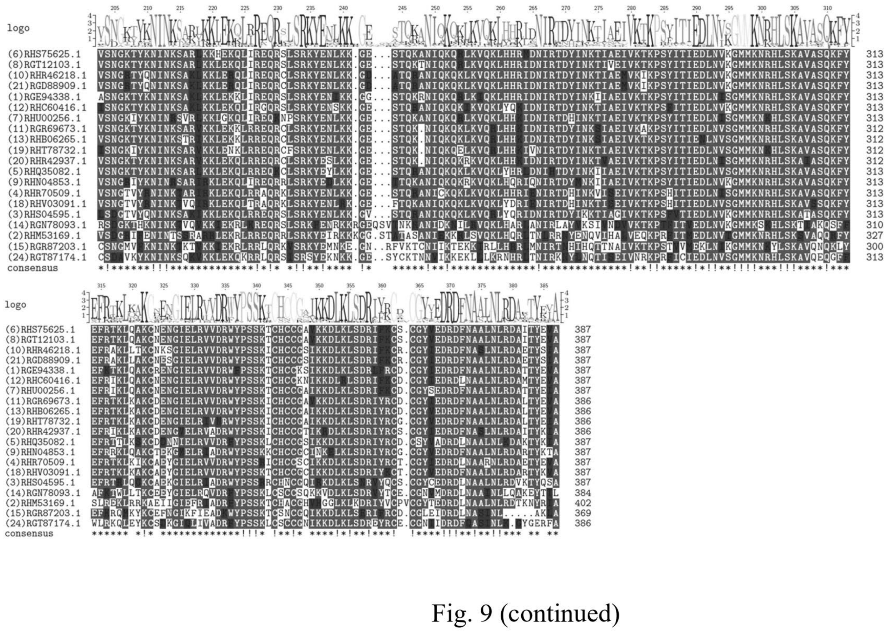

As shown in Fig. 9, multiple-sequence alignment analysis on 20 types of RAD systems with DNase activity was performed in the MAFFT version 7 website, using FbRAD1 (RHS75625.1) as the alignment reference. We found that in different strains of different genera, the following amino acids in proteins of the RAD system are highly conserved: Q18, G27, R30, N34, F50, W73, A92, F97, P104, K107, Y117, E145, P150, S173, G192, D194, G196, K198, A201, S204, N211, I212, N213, R227, Q229, S233, R234, E237, G242, N249, K252, N266, V280, K283, P284, E290, L292, N293, G296, M297, M298, K299, L303, S304, K305, Q310, K322, P338, S339, S340, C343, C346, L353, L355, R358, C362, C364, D369, R370, D371, A374, N377 and L378.

Based on the above results of sequence alignment analysis, we can infer that RAD proteins with these conserved amino acids also have DNase activity, because the RAD system is widely found in nature.

### Example 5: Intracellular cleavage activity in prokaryotes

Addition of targeting sequence: a 20-base sequence (CTGATGGTCCATGTCTGTTA, SEQ ID NO: 116) was directly added to the 3' end of the RNA sequence (SEQ ID NO: 63) bound by RAD protein FbRAD1 to serve as a targeting sequence. The 20-base sequence was complementary to the sequence of the PAM plasmid library and can target the PAM plasmid library. The addition was performed as described below. The expression plasmid was amplified by PCR using primers N20-TAR-F and N20-TAR-R (primer sequences are shown in Table 4), using each of PGEX-RAD 1-57 plasmids synthesized in Example 2 (for example, PGEX-6P1-FbRAD1-N20-HDV) as a template. The PCR amplification time was 6 minutes, the primer annealing temperature was 58°C, and the number of amplification cycles was 30. Then, the PCR product was digested with Dpn1 enzyme.

Constructing *E. coli* intracellular expression vectors: the digested product was ligated to the linear vector of the universal vector PET-28A by homologous recombination using the recombinase system from Vazyme Biotech Co., Ltd., and the recombinant product was transformed into *E. coli* DH5α competent cells. On the second day, single colonies were picked up and sequenced for identification, in order to obtain the RAD system expression plasmids pET-28a RAD 1-57 containing targeting sequences. An exemplary plasmid sequence, pET-28a-FbRAD1-N20-HDV, is shown below. This exemplary plasmid is a plasmid for the FbRAD1 system, which is a vector for the intracellular editing experiment in *E. coli.* The RAD system was inserted at the Ncol and Xho1 sites. The structure of FbRAD1 protein-6HIS-RNA-N20-HDV was shown below, where FbRAD1 protein is in bold and underlined, 6HIS is underlined, RNA is in bold, N20 is in italic, and HDV is in italic and in bold:

ATGAGGAAATTGCTGAAGAGCTTCAAGACGGAAATAAATCCAACA
GAAGAGCAGAAAGTCAGGATTCGTAAAACAATAGGTACTTGCAGATTTATC
TATAACTTCTATCTTGCTCATAATAAGGAGCTTCATGAAAGTGGCAAAAAGT
TTATGAGCAGCAGCCAATTCAGAGTATGGCTCAACAATGAATATCTTCCGA
GCCATCCGGAATATTCCTGGATCAAGGAAGCTTATTCAAAATCGGTAACAC
AGGCAGTAAATGATGGACAGACCGCATTTACAAGATTCTTTAATCACAAGA
GCGCCTTTCCAAAGTTTAAAAAGAAAGGCAAGTCTGATGTGAAAATGTATT
TTGTAAGGAATAATCCAAAAGATTGCAGCTGCGAAAGACACAGAATCAAGA
TTCCTTCACTTGGCTGGGTCCGTATCAAAGAGAAAGGATATATTCCCACTAC
AAAAGATGGATATGTAATTAAGAGCGGTCATGTCTCAATAAAAGCTGATAG
ATACTATGTTTCAGTTCTTATAGAAATCCCAGACAAAACGTCAGCTAGTAAT
TCCAGTGAAGGAATTGGCATTGACCTTGGATTAAAGGACTTTGCGATTGCT
TCTAATGGAAAGACTTATAAGAACATCAACAAATCTGCTAAATTAAAGAAGC
TTGAAAAAAAGCTTATTCGAGAACAGAGAAGTCTTTCTCGAAAGTATGAAA
ATATAAAGAAAGGAGGGTCCACTCAAAAAAGAAATATACAAAAACAAAGGC
TTAAGATACAGAAACTTCATCATAGAATCGACAATATTCGTACTGATTACAT
CAATAAGATAATCGCAGAGATAGTGAAAACCAAGCCATCTTATATAACGAT
TGAAGATCTAAACATATCAGGGATGATGAAGAACAGACATCTTTCAAAGGC
AGTTGCTTCGCAGAAGTTTTATGAATTTAAGACAAAGCTTCAAGCTAAATGC
AGGGAGAACGGAATCGAACTTCGAGTTGTAGATAGATGGTTCCCATCATCT

AAGACTTGTCACTGCTGTAAAAGTATCAAGAAAGATTTAAAGCTTTCAGATA
GACTTTTCAGATGTGATTGTGGATATATCGAAGACAGAGATTTCAATGCTG
CACTAAATCTGAGAGATGCTACAACTTACGAAGTTGCAAGCAGCCATCACCA
TCATCACCACTAAATGAACGCAAACGTAAGTATGTACCGAAGGCTATTTCGG
GAATTTACGACTATGGAGTGCACTAGAACTTGTGAGTAGATATGATTTCGG
TCAATCCAAAGCATGCACAGTGACATAGTAAGTAATGTTCGTGAGAACTTA
CATTATCTCGATGTGAGTATATTTAATCACATTTTGAGTGGCAGCTGATGGTC
CATGTCTGTTAGGCCGGCATGGTCCCAGCCTCCTCGCTGGCGCCGGCTGGGCAAC
ATGCTTCGGCATGGCGAATGGGACCCG (SEQ ID NO: 117)

Intracellular verification of DNA interference in E. coli: 57 RAD systems in Table 2 were cloned into PET28a (+) expression vectors at Ncol and Xhol cleavage sites by homologous recombination, and a spectinomycin-resistant plasmid PCS101ORI (pTargetF-aggag-n20-psc101ori) containing TAM sequence and a targeted sequence (CTGATGGTCCATGTCTGTTA) was constructed. The sequence of the plasmid is shown below.

pTargetF-aggag-n20-psc101ori (Targeted plasmid having spectinomycin resistance used in *E. coli* intracellular editing experiments). The motif sequence is underlined, and the targeted sequence is in bold)

catgttctttcctgcgttatcccctgattctgtggataaccgtattaccgcctttgagtgagctgataccgctcgccgcag
ccgaacgaccgagcgcagcgagtcagtgagcgaggaagcggaagagcgcctgatgcggtattttctccttacgcatctgtgcggta
tttcacaccgcatatgctggatccttgacagctagctcagtcctaggtataatactagtattgtcaccagactggcgtcgttttagagctag
aaatagcaagttaaaataaggctagtccgttatcaacttgaaaaagtggcaccgagtcggtgctttttttgaattctctagagtcgacctg
cagaagcttagatctattaccctgttatccctactcgagttcatgtgcagctccataagcaaaaggggatgataagtttatcaccaccgG
GGAGGAGCTGATGGTCCATGTCTGTTAactatttgcaacagtgccgttgatcgtgctatgatcgactgatg
tcatcagcggtggagtgcaatgtcatgagggaagcggtgatcgccgaagtatcgactcaactatcagaggtagttggcgtcatcgag
cgccatctcgaaccgacgttgctggccgtacatttgtacggctccgcagtggatggcggcctgaagccacacagtgatattgatttgct
ggttacggtgaccgtaaggcttgatgaaacaacgcggcgagctttgatcaacgaccttttggaaacttcggcttcccctggagagagc
gagattctccgcgctgtagaagtcaccattgttgtgcacgacgacatcattccgtggcgttatccagctaagcgcgaactgcaatttgg
agaatggcagcgcaatgacattcttgcaggtatcttcgagccagccacgatcgacattgatctggctatcttgctgacaaaagcaaga

gaacatagcgttgccttggtaggtccagcggcggaggaactctttgatccggttcctgaacaggatctatttgaggcgctaaatgaaac
cttaacgctatggaactcgccgcccgactgggctggcgatgagcgaaatgtagtgcttacgttgtcccgcatttggtacagcgcagta
accggcaaaatcgcgccgaaggatgtcgctgccgactgggcaatggagcgcctgccggcccagtatcagcccgtcatacttgaag
ctagacaggcttatcttggacaagaagaagatcgcttggcctcgcgcgcagatcagttggaagaatttgtccactacgtgaaaggcga
gatcaccaaggtagtcggcaaataagatgccgctcgccagtcgattggctgagctcataagttcctattccgaagttccgcgaacgcg
taaaggatctaggtgaagatcctttttgataatctcatgaccaaaatcccttaacgtgagttttcgttccactgagcgtcagaccccgtag
aaaagatcaaaggatcttctcagatccttccgtatttagccagtatgttctctagtgtggttcgttgtttttgcgtgagccatgagaacgaac
cattgagatcatacttactttgcatgtcactcaaaaattttgcctcaaaactggtgagctgaatttttgcagttaaagcatcgtgtagtgttttt
cttagtccgttatgtaggtaggaatctgatgtaatggttgttggtattttgtcaccattcatttttatctggttgttctcaagttcggttacgaga
tccatttgtctatctagttcaacttggaaaatcaacgtatcagtcgggcggcctcgcttatcaaccaccaatttcatattgctgtaagtgttta
aatctttacttattggtttcaaaacccattggttaagccttttaaactcatggtagttattttcaagcattaacatgaacttaaattcatcaaggc
taatctctatatttgccttgtgagttttcttttgtgttagttcttttaataaccactcataaatcctcatagagtatttgttttcaaaagacttaacat
gttccagattatattttatgaatttttttaactggaaaagataaggcaatatctcttcactaaaaactaattctaatttttcgcttgagaacttgg
catagtttgtccactggaaaatctcaaagcctttaaccaaaggattcctgatttccacagttctcgtcatcagctctctggttgctttagcta
atacaccataagcattttccctactgatgttcatcatctgaacgtattggttataagtgaacgataccgtccgttctttccttgtagggttttca
atcgtggggttgagtagtgccacacagcataaaattagcttggtttcatgctccgttaagtcatagcgactaatcgctagttcatttgcttt
gaaaacaactaattcagacatacatctcaattggtctaggtgattttaatcactataccaattgagatgggctagtcaatgataattactagt
ccttttcctttgagttgtgggtatctgtaaattctgctagacctttgctggaaaacttgtaaattctgctagaccctctgtaaattccgctaga
cctttgtgtgttttttttgtttatattcaagtggttataatttatagaataaagaaagaataaaaaaagataaaaagaatagatcccagccctg
tgtataactcactactttagtcagttccgcagtattacaaaaggatgtcgcaaacgctgtttgctcctctacaaaacagaccttaaaaccct
aaaggcttaagtagcaccctcgcaagctcggttgcggccgcaatcgggcaaatcgctgaatattccttttgtctccgaccatcaggca
cctgagtcgctgtctttttcgtgacattcagttcgctgcgctcacggctctggcagtgaatgggggtaaatggcactacaggcgcctttt
atggattcatgcaaggaaactacccataatacaagaaaagcccgtcacgggcttctcagggcgttttatggcgggtctgctatgtggtg
ctatctgactttttgctgttcagcagttcctgccctctgattttccagtctgaccacttcggattatcccgtgacaggtcattcagactggcta
atgcacccagtaaggcagcggtatcatcaacaacgccagcaacgcggcctttttacggttcctggccttttgctggccttttgctca(S
EQ ID NO: 138)

Two plasmids, spectinomycin-resistance plasmid pTargetF-aggag-n20-psc101ori and each of RAD system expression plasmids pET-28a RAD 1 to 57, were co-transformed into *E. coli* C43 (DE3), and the method for transformation was the same as that in Example 2. 100 ng of each of the two plasmids was added simultaneously to (*E. coli* C43 (DE3)) competent cells. The cells were cultured overnight on a LB plates containing both 100 µg/mL of spectinomycin and kanamycin at 30°C. The single colonies were picked up on the plates with the two antibiotics and cultured in 2 mL of LB medium containing spectinomycin and kanamycin and 0.6 mM IPTG at 30°C, 220 rpm for 12 hours overnight. On the second day, the bacterial solution was diluted serially to 10⁻⁶ in ten-fold dilutions. Then, 5µL of the bacterial solution was dropped on plates with the two antibiotics, and cultured at 30°C for 24 hours. The growth was observed and imaged for counting. Once DNA-specific cleavage occurs, significant growth differences can be observed on the screening plates with kanamycin and spectinomycin, and the cells in which cleavage takes place will die. Fig. 20 shows a flow chart showing the DNA cleavage by RAD protein in *E. coli* and the step of screening.

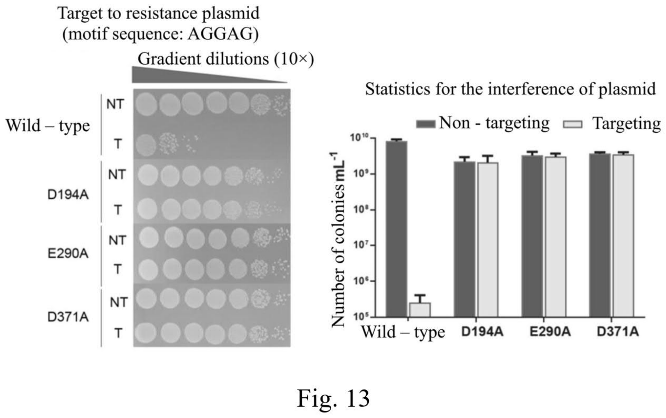

Results and Figs. 13, 14, and 15 show that the RAD systems successfully produce specific DNA cleavage in *E. coli.*

The DNA sequence to be interfered with was a spectinomycin resistance sequence, which was an exogenous sequence (PUC19-TARGET1-AGGAG, PUC19-TARGET1-GAGGG, see Table 4). Under the conditions where the TAM sequence was AGGAG or GAGGG, a total of 12 RAD systems were found to successfully achieve cleavage. The 12 RAD (IS607 TnpB) systems were from 7 different species, and were (6) FbRAD1/ISFba1 (RHS75625.1), (10) CbRAD1/ISCbal (RHR46218.1), (11) RiRAD/ISRin (RGR69673.1), (13) ErRAD/ISErel (RHB06265.1), (15) WcRAD (RGR87203.1), (18) BsRAD/ISBsp4 (RHV03091.1), (20) CbRAD2/ISClsp2 (RHR42937.1), (21) CbRAD3/ISClsp3 (RGD88909.1), (24) CcRAD/ISCco (RGT87174.1), (30) FbRAD2/ISFba2 (RGH01875.1), (42) FbRAD3/ISFba3(RHU26542.1), and (43) FbRAD4/ISFba4 (RGH37793.1), respectively.

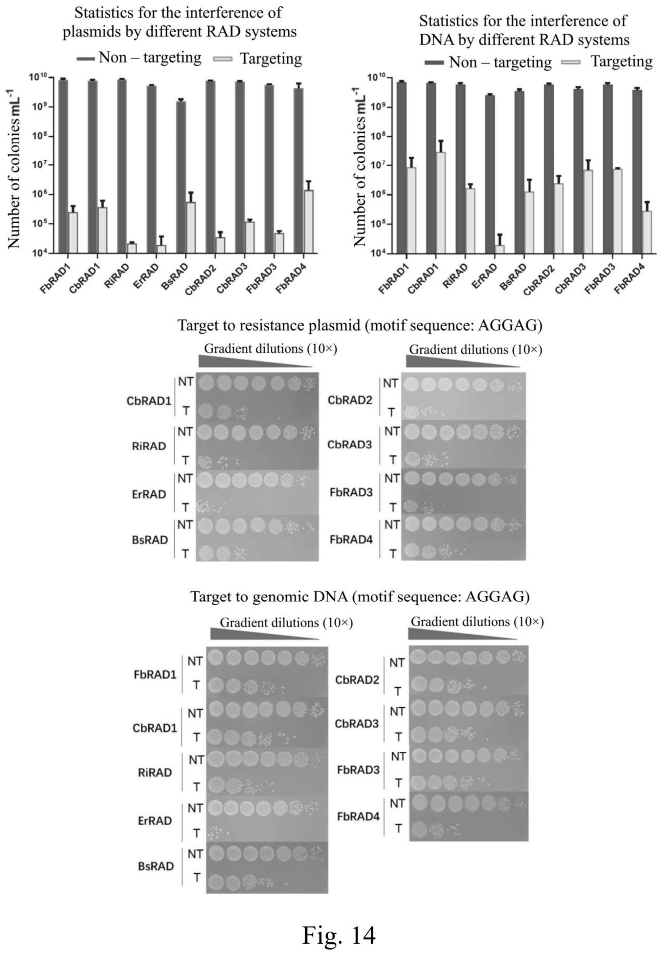

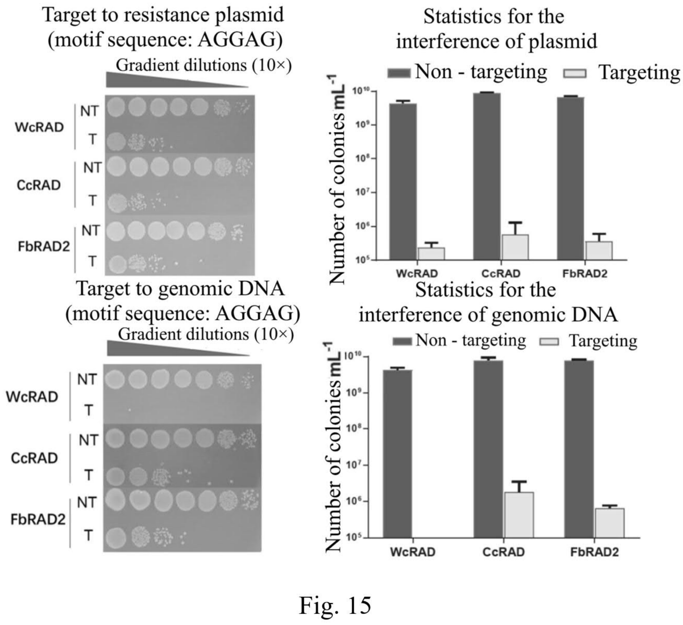

In order to cleave a DNA in the endogenous genome of *E. coli,* the targeting sequences were replaced against the genome of *E. coli.* The DNA sequence to be interfered with was a genome sequence of *E. coli*, which is an endogenous sequence. (ECOLI-TARGET-AGGAG and ECOLI-TARGET-GAGGG, see Table 4; the motifs and targeted sequences in pTargetF-aggag-n20-psc101ori described above were replaced with the sequences shown in Table 4). Plates with kanamycin were used to screen single colonies and were observed after 10-fold dilution titration (Figs. 14, 15). Figs. 14 and 15 show the DNA interference results in *E. coli* for different RAD systems when the TAM sequence is AGGAG or GAGGG, respectively. The colonies formed after titration show that the growths of the targeted group (T group) (i.e., FbRAD1, CbRAD1, RiRAD, ErRAD, and BsRAD) were significantly inhibited as compared to the non-targeted control group (NT group), showing a difference exceeding 10⁴ in more than one system.

Verification of the enzymatic digestion activity after a mutation in the active centers: DNA interference no longer occurred after the inactive mutations to alanine of the amino acids of three active centers in FbRAD1, aspartic acid at position 194, glutamic acid at position 290, and aspartic acid at position 371 (Fig. 13).

**Table 5: Sequences of targets in prokaryote experiments**

| **Name** | **Description** | **Sequence** | **SEQ ID NO** |
|---|---|---|---|
| **PUC19-TARGET1-AGGAG** | the targeted sequence of the spectinomycin resistance plasmid for *in vitro* enzymatic digestion experiments and *E. coli* intracellular experiments, the TAM motif was AGGAG, | aggagCTGATGGTCC ATGTCTGTTA | 139 |
| **PUC19-TARGET1-GAGGG** | the targeted sequence of the spectinomycin resistance plasmid for *E. coli* intracellular experiments, the TAM motif was GAGGG, | gagggCTGATGGTC CATGTCTGTTA | 140 |
| **ECOLI-TARGET-AGGAG** | For *E. coli* intracellular experiments, the genome sequence of *E. coli* was targeted, and the TAM motif was AGGAG. | aggagAGGTAGGGC AAACCGCCCCT | 141 |
| **ECOLI-TARGET-GAGGG** | For *E. coli* intracellular experiments, the genome sequence of *E. coli* was targeted, and the TAM motif was GAGGG. | gagggCAAAGGCCT CCCGGAAGTTC | 142 |

### Example 6: In vivo gene editing in humans

Individual proteins in each of the RAD systems in Table 2 were fused to a nuclear localization signal and green fluorescent protein, and the CMV promoter was used to regulate the expression. On the same plasmid, the U6 promoter was used to regulate the expression of RNA in each of the RAD systems, and the RNA contained the 20-base genome-targeting sequence at the end.

The exemplary plasmid sequence pcdna3.1-u6-FbRAD1-cmv-3flag-SV40-FbRAD1-NLS-2a-EGFP is shown below, which is a plasmid used in intracellular editing in human 293F cells. The U6 promoter sequence is in bold, the RNA sequence associated with RAD protein FbRAD1 is italic and in bold, the CMV sequence is in bold and underlined, the 3flag sequence is italic, the SV40 sequence is italic, in bold and underlined, the RAD protein FbRAD1 is italic, in bold and double underlined, the NLS sequence is double underlined, the EGFP sequence is italic and underlined, and the 2a sequence is in bold and double underlined.

gagggcctatttcccatgattccttcatatttgcatatacgatacaaggctgttagagagataattggaattaat
ttgactgtaaacacaaagatattagtacaaaatacgtgacgtagaaagtaataatttcttgggtagtttgcagttttaaaattat
gttttaaaatggactatcatatgcttaccgtaacttgaaagtatttcgatttcttggctttatatatcttgtggaaaggacGAA
ACACCGCTAAAACGCAAACGTAAGTATGTACCGAAGGCTATTTTCGGGAATTTAC
GACTATGGAGTGTACAAGAACTTGTGAGTAGATATGATTTCGGTCAATCCAAAGCA
TACACGATGAAATAGTAAGTAATGTTCGTGAGAACTTACATTATCTCGATGTGAGC
ATATTTAATCACATTTTGAGTGGCAGCTGATGGTCCATGTCTGTTAGGCCGGCATG
GTCCCAGCCTCCTCGCTGGCGCCGGCTGGGCAACATGCTTCGGCATGGCGAATGG
GACTTTTTTGTTTTAGAGCTAGAAATAGCAAGTTAAAATAAGGCTAGTCCGTTTT
TAGCGCGTGCGCCAATTCTGCAGACAAATGGCTCTAGAGGTACCCGATGTACGG
GCCAGATATACGCGTTGACATTGATTATTGACTAGTTATTAATAGTAATCAATTA
CGGGGTCATTAGTTCATAGCCCATATATGGAGTTCCGCGTTACATAACTTACGGT
AAATGGCCCGCCTGGCTGACCGCCCAACGACCCCCGCCCATTGACGTCAATAAT
GACGTATGTTCCCATAGTAACGCCAATAGGGACTTTCCATTGACGTCAATGGGT
GGAGTATTTACGGTAAACTGCCCACTTGGCAGTACATCAAGTGTATCATATGCC
AAGTACGCCCCCTATTGACGTCAATGACGGTAAATGGCCCGCCTGGCATTATGC
CCAGTACATGACCTTATGGGACTTTCCTACTTGGCAGTACATCTACGTATTAGTC
ATCGCTATTACCATGGTGATGCGGTTTTGGCAGTACATCAATGGGCGTGGAT
AGCGGTTTGACTCACGGGGATTTCCAAGTCTCCACCCCATTGACGTCAATG
GGAGTTTGTTTTGGCACCAAAATCAACGGGACTTTCCAAAATGTCGTAACA
ACTCCGCCCCATTGACGCAAATGGGCGGTAGGCGTGTACGGTGGGAGGTC

TATATAAGCAGAGCTCTCTGGCTAACTAGAGAACCCACTGCTTACTGGCTTATC
GAAATTAATACGACTCACTATAGGGAGACCCAAGCTGGCTAGCGTTTAAACTTA
AGCTTGGTACCGAGCTCGGATCCgccaccatggactataaggaccacgacggagactacaaggatcatga
tattgattacaaagacgatgacgataagatggccccaaagaagaagcggaaggtcggtatccacggagtcccagcagccAT
GTGGAAATTGCTAAAGAGCTTCAAGACGGAAATAAATCCGTCAGAGGAACAGAAA
GTCAAGATTCATAAGACGATAGGAACTTGTAGATTCATTTATAATTTCTATCTTGCT
CATAACAAAGAACTTTACGATAAAGGCGAAAAGTTTATGAGTGGTAAAAGTTTTAG
TGTTTGGCTGAACAATGAGTATCTTCCACAAAATCCAGACAAGTTATGGATTAAGG
AAGTCAGTTCAAAATCTGTAAAGCACTCAATTGAAAATGGTTGTATCGCATTTACC
AGATTTTTCAAACATCAAAGTGCTTTTCCGAATTTAAAAAAGAAAGGCAAATCTGA
TGTAAAAATGTATTTTGTAAAGAATAATCCAAAAGATTGTCGTTGCGAAAGACACA
GAATCAATATTCCGTCACTTGGTTGGGTTCGCATCAAAGAAAAAGGATATATTCCA
ACAACTAAAGACGGATATGTGATTAAAAGCGGCACGGTCTCAATGAAAGCTGACA
GATACTATGTTTCAGTTCTTGTAGAAATTTCAAATAACAAGATAGCCAATAATTCC
AATGCAGGAATTGGCATTGACCTTGGATTAAAGGATTTTGCCATTGTTTCTAATGG
TAAAACTTATAAGAACATTAACAAATCTGCAAGATTAAAGAAACATGAAAAACAGC
TTATTCGAGAACAGAGAAGTCTTTCTCGAAAGTATGAAAATTTAAAGAAAGGAGAG
TCCACTCAAAAAGCAAATATACAAAAACAAAGGCTTAAGGTACAGAAACTTCATCA
TAGAATGGATAATATTCGTACTGATTACATCAATAAAACAATCGCAGAGATAGTAA
AAACCAAACCATCTTATATTACGATTGAAGATTTGAATGTAAAAGGTATGATGAAA
AACAGACATCTTTCAAAAGCTGTTGCTTCACAGAAGTTTTATGAATTTAGAACAAA
GCTTCAAGCTAAATGCAATGAAAATGGAATCGAACTTCGAGTAGTAGACAGATGG
TATCCATCATCTAAAACCTGTCACTGCTGTGGCGCTGTCAAAAAAGATTTAAAGCT
TTCAGATAGAATATTTAAATGCAGTTGCGGTTATGTCGAAGACAGAGATTTCAATG
CTGCACTTAATTTGAGAGATGCGATAACTTACGAAGTTGCAaaaaggccggcggccacgaaa
aaggccggccaggcaaaaaagaaaaaggaattcggcagtggagagggcagaggaagtctgctaacatgcggtgacgtcgag
gagaatcctggcccagtgagcaagggcgaggagctgttcaccggggtggtgcccatcctggtcgagctggacggcgacgtaa
acggccacaagttcagcgtgtccggcgagggcgagggcgatgccacctacggcaagctgaccctgaagttcatctgcaccacc
ggcaagctgcccgtgccctggcccaccctcgtgaccaccctgacctacggcgtgcagtgcttcagccgctaccccgaccacatg

aagcagcacgacttcttcaagtccgccatgcccgaaggctacgtccaggagcgcaccatcttcttcaaggacgacggcaactac
aagacccgcgccgaggtgaagttcgagggcgacaccctggtgaaccgcatcgagctgaagggcatcgacttcaaggaggac
ggcaacatcctggggcacaagctggagtacaactacaacagccacaacgtctatatcatggccgacaagcagaagaacggc
atcaaggtgaacttcaagatccgccacaacatcgaggacggcagcgtgcagctcgccgaccactaccagcagaacaccccca
tcggcgacggccccgtgctgctgcccgacaaccactacctgagcacccagtccgccctgagcaaagaccccaacgagaagcg
cgatcacatggtcctgctggagttcgtgaccgccgccgggatcactctcggcatggacgagctgtacaaggaattctaaCTCG
AG

Other corresponding plasmid constructs can be obtained by replacing the nucleic acid sequences encoding FbRAD1 protein and associated RNA in the exemplary sequence pcdna3.1-u6-FbRAD1-cmv-3flag-sv40-FbRAD1-NLS-2a-EGFP with the nucleic acids encoding each of the other effector proteins and corresponding RNAs.

Cell transfection: human 293F cells were selected as the transfection objects. In 10mL cell transfection system, the cell density was 7 x 10⁵ cells/mL, and the culture medium was SMM 293TII produced by Sino Biological, Inc., Beijing. 10µg of the plasmid was added to 0.5 ml of OPTI-MEM at room temperature for 5 minutes, and 50µL of PEI was added to 0.5 mL of OPTI-MEM at room temperature for 5 minutes. The two solutions were mixed and incubated at room temperature for 25 minutes, and then the mixture was added to 10mL of cells. The cells were cultured at 5% CO₂, 120 rpm in a cell culture shaker for 72 hours.

Flow cytometry sorting and high-throughput sequencing: cells containing a green fluorescent signal were sorted by a FACSAria Fusion flow cytometer from BD Company, and cells which died or failed in transfection and expression were removed. A universal column-type genome extraction kit available from Cowin Biotech Co., Ltd. was used to extract the whole genome of cells, and the products were directly sent to a sequencing company for high-throughput sequencing. Then, the results were analyzed for gene editing efficiency.

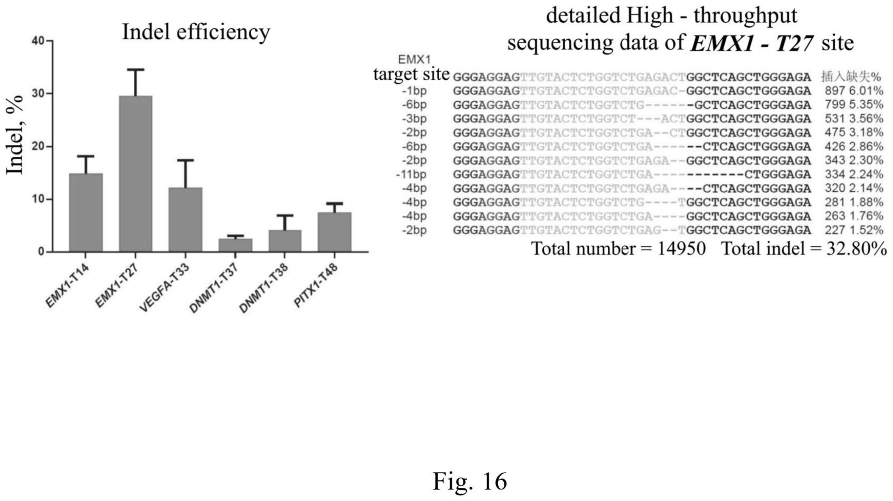

By constructing a eukaryotic expression vector (pcdna3.1-u6-FbRAD1-cmv-3flag-sv40-FbRAD1-NLS-2a-EGFP) and cell transfection experiments as well as high-throughput sequencing analysis, the inventors successfully verified that the FbRAD1 system can achieve specific DNA editing in human cells. Editing occurred at *EMX1*, *VEGFA*, *DNMT1*, and *PITX1* gene loci (see Table 5 for sequence information), and the editing efficiency at the *EMX1* locus reaches up to 32.8%. This efficiency is similar to the intracellular editing activities originally revealed for the widely used Cas9 and Cas12a. This study also provides a basis for the RAD system to be further optimized into an efficient gene editing tool (Fig. 16). Fig. 21 shows a flow chart showing the DNA editing by RAD protein in 293F cells and steps of detection.

**TABLE 6: gene locus information of EMX1, VEGFA, DNMT1, PITX1.**

| | | |
|---|---|---|
| **EMX1-T14** | Targeted site for intracellular editing of human 293F cells | aggagAAGGCCAAGTGGTCCCAGGC(SEQ ID NO: 144) |
| **EMX1-T27** | Targeted site for intracellular editing of human 293F cells | aggagTTGTACTCTGGTCTGAGACT(SEQ ID NO: 145) |
| **VEGFA-T33** | Targeted site for intracellular editing of human 293F cells | aggagGAGGGCAGAATCATCACGAA(SEQ ID NO: 146) |
| **DNMT1-T37** | Targeted site for intracellular editing of human 293F cells | aggagTCTGATGTCGGGTTATACAC(SEQ ID NO: 147) |
| **DNMT1-T38** | Targeted site for intracellular editing of human 293F cells | aggagACAAAGTTCCTGACATTCTC(SEQ ID NO: 148) |
| **PITX1-T48** | Targeted site for intracellular editing of human 293F cells | aggagAAGGCCAGGTGACAGAGGGT(SEQ ID NO: 149) |

Using the above experimental method, different RAD systems were expressed in 293F cells to verify whether they all have an intracellular editing function in mammalian cells. 9 RAD systems were found to be capable of intracellular editing, and these systems were derived from eight different species. They were: (6) FbRAD1/ISFba1 (RHS75625.1), (11) RiRAD/ISRin (RGR69673.1), (13) ErRAD/ISErel (RHB06265.1), (21) CbRAD3/ISClsp3 (RGD88909.1), (43) FbRAD4 (RGH37793.1), (51) DfRAD/ ISDfo4 (GUT_GENOME239804_2_85784_86938_+), (52) ArRAD/ ISAre (GUT_GENOME047820_36_478_1632_-), (54) HuRAD/ ISHun (GUT_GENOME022845_45_3955_5109_+) and (55) AfRAD/ ISAfa (GUT_GENOME112567_131_1855_3009_-) (Fig. 17).

The RAD systems in a total of 16 strains from 11 different species have an intracellular activity to cleave DNA and are representatives of RAD systems. This confirms that the DNA cleavage activity in RAD systems is ubiquitous, providing a theoretical basis for further research on RAD systems. More RAD systems can be developed and optimized as gene editing tools.

RAD protein has a smaller protein size (about 387 amino acids). Compared with effector proteins of gene editing tools that have been widely studied currently, such as Cas9 and Cas12a effectors (generally greater than 1,000 amino acids), the RAD protein has the smallest size (384-387 amino acids), which suggests the value of further development into new gene editing tools. The smaller size is more advantageous in delivering vector constructs than traditional large proteins. The inventors have characterized the fundamental features of the RAD systems and constructed a gene-editing platform founded on these systems. This work lays a foundation for further optimizing the RAD systems into a more precise and efficient gene-editing tool.

The present invention described and claimed herein is not limited to the scope of the specific aspects disclosed herein, as these aspects are intended to be illustrative of several aspects of the disclosure. Any equivalent aspect is intended to be within the scope of the disclosure. Indeed, various modifications of the disclosure in addition to those shown and described herein will become obvious to those skilled in the art from the above description. Such modifications are also intended to fall within the scope of the appended claims. In case of conflict, this disclosure, including definitions, prevails.

## Claims

1. An endonuclease complex comprising an RNA-guided endonuclease and a guide RNA,
wherein the RNA-guided endonuclease comprises an amino acid sequence selected from the group consisting of SEQ ID NOs: 6, 1-5, 7-57 and SEQ ID NOs: 150-191, or an amino acid sequence having at least 30%, 40%, 50%, 60%, 70%, 80%, 85%, 90%, 95%, 96%, 97%, 98%, 99% or 100% identity to any one of SEQ ID NOs: 6, 1-5, 7-57 and SEQ ID NOs: 150-191;
the guide RNA comprises an RNA-guided endonuclease-associated RNA, and preferably further comprises a targeting RNA;
the RNA-guided endonuclease-associated RNA comprises the sequence of any one of SEQ ID NOs: 63, 58-62, 64-114, and SEQ ID NOs: 192-233 or a sequence having at least 30%, 40%, 50%, 60%, 70%, 80%, 85%, 90%, 95%, 96%, 97%, 98%, 99% or 100% identity to the sequence of any one of SEQ ID NOs: 63, 58-62, 64-114, and SEQ ID NOs: 192-233;
the targeting RNA is at the 3' end of the RNA-guided endonuclease-associated RNA and is at least 70%, 80%, 85%, 90%, 95%, 96%, 97%, 98%, 99% or 100% identical or complementary to a targeted sequence in a gene of interest, preferably the targeting RNA has a length of 12-40 bp;
preferably, the endonuclease system comprises an endonuclease of SEQ ID NO: N or a sequence having at least 30%, 40%, 50%, 60%, 70%, 80%, 85%, 90%, 95%, 96%, 97%, 98%, 99% or 100% identity to SEQ ID NO: N paired with an RNA guide sequence comprising SEQ ID NO: N + 57 or a sequence having at least 30%, 40%, 50%, 60%, 70%, 80%, 85%, 95%, 96%, 97%, 98%, 99% or 100% identity to SEQ ID NO: N + 57, wherein N is an integer of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, or 57; or the endonuclease system comprises an endonuclease of SEQ ID NO: M or a sequence having at least 30%, 40%, 50%, 60%, 70%, 80%, 85%, 90%, 95%, 96%, 97%, 98%, 99% or 100% identity to SEQ ID NO: M paired with an RNA guide sequence comprising SEQ ID NO: M + 42 or a sequence having at least 30%, 40%, 50%, 60%, 70%, 80%, 85%, 95%, 96%, 97%, 98%, 99% or 100% identity to SEQ ID NO: M + 42, wherein the M is an integer of 150, 151, 152, 153, 154, 155, 156, 157, 158, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, or 191;
preferably, the endonuclease complex comprises an RNA-guided endonuclease of SEQ ID NO: 6 and a guide RNA of SEQ ID NO: 63; an RNA-guided endonuclease of SEQ ID NO: 11 and a guide RNA of SEQ ID NO: 68; an RNA-guided endonuclease of SEQ ID NO: 13 and a guide RNA of SEQ ID NO: 70; an RNA-guided endonuclease of SEQ ID NO: 21 and a guide RNA of SEQ ID NO: 78; an RNA-guided endonuclease of SEQ ID NO: 43 and a guide RNA of SEQ ID NO: 100; an RNA-guided endonuclease of SEQ ID NO: 51 and a guide RNA of SEQ ID NO: 108; an RNA-guided endonuclease of SEQ ID NO: 52 and a guide RNA of SEQ ID NO: 109; an RNA-guided endonuclease of SEQ ID NO: 54 and a guide RNA of SEQ ID NO: 111; or an RNA-guided endonuclease of SEQ ID NO: 55 and a guide RNA of SEQ ID NO: 112.

2. The endonuclease complex of claim 1, wherein the RNA-guided endonuclease comprises a RuvC domain, and the RNA-guided endonuclease comprises an amino acid selected from the group consisting of Q18, G27, R30, N34, F50, W73, A92, F97, P104, K107, Y117, E145, P150, S173, G192, D194, G196, K198, A201, S204, N211, I212, N213, R227, Q229, S233, R234, E237, G242, N249, K252, N266, V280, K283, P284, E290, L292, N293, G296, M297, M298, K299, L303, S304, K305, Q310, K322, P338, S339, S340, C343, C346, L353, L355, R358, C362, C364, D369, R370, D371, A374, N377 and L378 at an amino acid position corresponding to the amino acid sequence of SEQ ID NO: 6; preferably wherein the gene of interest is a single-stranded or double-stranded DNA, and preferably comprises a motif selected from the group consisting of NGGAG, NGGNN, AGGAG, GAGGG, GGGGG, NNGGG, NNAGG, AGGNN, NTAAA, NGAGG, NNGGN or GGGGN.

3. A polynucleotide comprising a polynucleotide encoding the RNA-guided endonuclease in any one of claims 1-2 and/or a polynucleotide encoding the RNA molecule.

4. A vector comprising the polynucleotide molecule of claim 3, and optionally comprising an expression control sequence; wherein the RNA-guided endonuclease and the guide RNA are under the control of the same or different regulatory elements, such as a promoter.

5. A viral particle, preferably an adeno-associated viral particle, comprising the endonuclease complex of claim 1 or 2, the polynucleotide of claim 3 or the vector of claim 4.

6. A method for specifically cleaving DNA comprising the step of contacting the endonuclease complex of any one of claims 1-2 with the DNA,
preferably, the DNA is DNA of a eukaryotic cell, a prokaryotic cell or a virus;
preferably, the DNA is a single-stranded or double-stranded DNA;
preferably, the method comprises the step of introducing the polynucleotide of claim 3, the vector of claim 4 or the viral particle of claim 5 into a cell;
preferably, the targeted sequence of the DNA comprises a motif selected from the group consisting of NGGAG, NGGNN, AGGAG, GAGGG, GGGGG, NNGGG, NNAGG, AGGNN, NTAAA, NGAGG, NNGGN or GGGGN, and the DNA at the 3' end of the motif is at least 70%, 80%, 85%, 90%, 95%, 96%, 97%, 98% or 99% identical or complementary to the targeting RNA.

7. The method of claim 6, wherein the method is performed *in vivo* or *in vitro.*

8. The method of claim 6 or 7, wherein the method is performed under conditions selected from the group consisting of 0.5 to 10 mM of a divalent metal ion selected from the group consisting of Mg²⁺, Mn²⁺ and Ca²⁺, 25 to 250 mM Na⁺, a reaction temperature between 27 and 57 °C and a reaction time between 3 and 60 minutes.

9. A method for gene editing comprising the step of contacting the endonuclease complex of any one of claims 1-2 with a genomic DNA of a cell for the gene editing;
preferably, the gene editing results in an insertion and/or a deletion in the genomic DNA;
preferably, the gene editing is performed in a mammalian cell to treat or prevent a disease, or to introduce a desired trait.

10. A kit comprising one or more of the following: the endonuclease complex of any one of claims 1-2, the polynucleotide of claim 3, the vector of claim 4, or the viral particle of claim 5.

11. A method for inactivating or identifying a virus, comprising contacting the virus with the endonuclease complex or the endonuclease in any one of claims 1-2, wherein the endonuclease comprises an amino acid sequence selected from the group consisting of SEQ ID NOs: 1-57 and SEQ ID NOs: 150-191, or an amino acid sequence having at least 30%, 40%, 50%, 60%, 70%, 80%, 85%, 90%, 95%, 96%, 97%, 98%, 99% or 100% identity to any one of SEQ ID NOs: 1-57 and SEQ ID NOs: 150-191.

12. A method for treating infection with a virus, comprising administering the endonuclease complex of any one of claims 1-2 to a patient infected with the virus.

13. A viral vector comprising the polynucleotide of claim 3 or the vector of claim 4, preferably wherein the viral vector is an adeno-associated viral vector.

14. A gene therapy method for treating a disease comprising using the viral vector of claim 13 or the viral particle of claim 5.

15. A cell comprising one or more of the following: the endonuclease complex of any one of claims 1-2, the polynucleotide of claim 3, the vector of claim 4, or the viral particle of claim 5; preferably, the cell is a eukaryotic cell or a prokaryotic cell, preferably, the eukaryotic cell is a fungal cell, a plant cell or an animal cell.
